(19) Europäisches Patentamt / European Patent Office / Office européen des brevets

(11) **EP 4 138 827 B1**

(12) # FASCICULE DE BREVET EUROPEEN

(45) Date de publication et mention de la délivrance du brevet:
**15.05.2024 Bulletin 2024/20**

(21) Numéro de dépôt: **21719923.1**

(22) Date de dépôt: **22.04.2021**

(51) Classification Internationale des Brevets (IPC):
***A61K 31/4375** (2006.01)* ***A61K 31/445** (2006.01)*
***A61K 38/43** (2006.01)* ***A61P 21/00** (2006.01)*
***A61P 25/00** (2006.01)* ***C07D 211/00** (2006.01)*

(52) Classification Coopérative des Brevets (CPC):
**A61K 31/445; A61K 31/4375; A61P 21/00; A61P 25/00; C07D 211/46; C07D 401/12; C07D 405/12; C07D 455/02; C07D 471/04; C12N 9/2405; C12Y 302/0102**

(86) Numéro de dépôt international:
**PCT/EP2021/060572**

(87) Numéro de publication internationale:
**WO 2021/214245 (28.10.2021 Gazette 2021/43)**

(54) **NOUVEAUX COMPOSÉS CHAPERONS PHARMACOLOGIQUES DE L'ALPHA-GLUCOSIDASE ACIDE HUMAINE ET LEUR UTILISATION THÉRAPEUTIQUE**

NEUE PHARMAKOLOGISCHE CHAPERONVERBINDUNGEN AUS MENSCHLICHER SAURER ALPHA-GLUCOSIDASE UND IHRE THERAPEUTISCHE VERWENDUNG

NOVEL PHARMACOLOGICAL CHAPERONE COMPOUNDS OF HUMAN ACID ALPHA-GLUCOSIDASE AND THE THERAPEUTIC USE THEREOF

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **23.04.2020 FR 2004065**

(43) Date de publication de la demande:
**01.03.2023 Bulletin 2023/09**

(73) Titulaires:
- **Université Grenoble Alpes**
  **38400 Saint Martin d'Hères (FR)**
- **Centre national de la recherche scientifique**
  **75016 Paris (FR)**

(72) Inventeurs:
- **PY, Sandrine**
  **38410 SAINT MARTIN D'URIAGE (FR)**
- **KANAZAWA, Alice**
  **38190 BERNIN (FR)**
- **VIEIRA DA CRUZ, Anaïs**
  **38430 MOIRANS (FR)**
- **TANGARA, Salia**
  **59800 LILLE (FR)**

(74) Mandataire: **Ipside**
**6, Impasse Michel Labrousse**
**31100 Toulouse (FR)**

(56) Documents cités:
- **JULIEN BOISSON ET AL: "Hydroxymethyl-Branched Polyhydroxylated Indolizidines: Novel Selective [alpha]-Glucosidase Inhibitors", ORGANIC LETTERS, vol. 17, no. 15, 16 juillet 2015 (2015-07-16), pages 3662-3665, XP055758292, US ISSN: 1523-7060, DOI: 10.1021/acs.orglett.5b01505**
- **SALIA TANGARA ET AL: "Aziridination of Cyclic Nitrones Targeting Constrained Iminosugars", ORGANIC LETTERS, vol. 19, no. 18, 1 septembre 2017 (2017-09-01), pages 4842-4845, XP055758299, US ISSN: 1523-7060, DOI: 10.1021/acs.orglett.7b02283**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 4 138 827 B1

- **ANAIS VIEIRA DA CRUZ ET AL: "Polyhydroxylated Quinolizidine Iminosugars as Nanomolar Selective Inhibitors of [alpha]-Glucosidases", JOURNAL OF ORGANIC CHEMISTRY, vol. 82, no. 18, 28 août 2017 (2017-08-28) , pages 9866-9872, XP055758301, Japan ISSN: 0022-3263, DOI: 10.1021/acs.joc.7b01494**
- **JOHN J. FLANAGAN ET AL: "The pharmacological chaperone 1-deoxynojirimycin increases the activity and lysosomal trafficking of multiple mutant forms of acid alpha-glucosidase", HUMAN MUTATION, vol. 30, no. 12, 1 décembre 2009 (2009-12-01), pages 1683-1692, XP055071682, ISSN: 1059-7794, DOI: 10.1002/humu.21121 cité dans la demande**

## Description

La présente invention s'inscrit dans le domaine du traitement de maladies lysosomales, et plus spécifiquement du traitement de la maladie de Pompe. Plus particulièrement, la présente invention concerne des composés, appartenant à la classe des iminosucres, capables d'interagir de manière sélective avec les α-glucosidases humaines et en particulier de stabiliser l'α-glucosidase acide humaine dans sa forme endogène (GAA) ou de nature recombinante (rhGAA), dans une conformation favorisant son transport vers le lysosome, pour leur utilisation en tant que médicament, notamment pour traiter la maladie de Pompe ; ainsi qu'une composition pharmaceutique contenant de tels composés. L'invention concerne également des composés appartenant à la classe des iminosucres capables d'interagir de manière sélective avec l'α-glucosidase acide humaine. L'invention concerne en outre un procédé de préparation de tels composés.

La maladie de Pompe, ou glycogénose de type II, est une maladie génétique rare caractérisée par une ou plusieurs mutations du gène *GAA* codant pour l'enzyme lysosomale α-glucosidase acide (GAA) humaine, ce qui entraine un déficit de l'activité de cette enzyme, et par voie de conséquence l'accumulation de glycogène, dont la biodégradation hydrolytique est assurée par cette enzyme, dans les lysosomes, ce qui entraîne des dysfonctionnements cellulaires dans les muscles et le coeur. Les symptômes sont divers, et leur progression plus ou moins rapide selon que la maladie se déclare à la naissance ou à l'âge adulte. Ils touchent tous les muscles, notamment les muscles respiratoires et le myocarde. L'espérance de vie des sujets atteints en est réduite, surtout chez le nourrisson qui décède au cours de sa première année de vie.

Il n'existe à l'heure actuelle qu'un seul traitement de la maladie de Pompe ayant reçu une autorisation de mise sur le marché. Ce traitement, dit de thérapie enzymatique substitutive (TES), consiste à administrer par voie intraveineuse la spécialité Myozyme®, dont le principe actif est une α-glucosidase acide humaine recombinante (rhGAA), qui permet de stabiliser les symptômes de la maladie.

Cependant, ce traitement présente de nombreux inconvénients : il est très onéreux, contraignant pour le patient, car il nécessite une perfusion toutes les deux semaines, en milieu hospitalier, et il est d'une efficacité limitée dans de nombreux cas. Il peut en effet entrainer une réaction immunitaire affectant son efficacité et sa tolérance par le sujet traité. En outre, l'enzyme recombinante est relativement instable dans le sang, et les doses devant être administrées aux malades sont bien supérieures à celles administrées pour le traitement d'autres maladies lysosomales.

Il subsiste ainsi à l'heure actuelle un besoin pour un traitement satisfaisant de la maladie de Pompe.

La présente invention vise à proposer un tel traitement. Plus particulièrement, l'invention vise à proposer des composés permettant un traitement efficace de la maladie de Pompe, ce traitement étant en outre peu contraignant à réaliser, et moins coûteux que le traitement par thérapie enzymatique substitutive proposé par l'art antérieur.

Un objectif supplémentaire de l'invention est que ces composés, administrés en même temps que le traitement par thérapie enzymatique substitutive de l'art antérieur, permettent d'augmenter l'efficacité de ce dernier de manière significative.

Cherchant à atteindre ces objectifs, les présents inventeurs se sont intéressés à une approche thérapeutique considérée à l'heure actuelle comme l'une des plus prometteuses pour le traitement des maladies lysosomales : l'utilisation de molécules dites chaperons pharmacologiques. Les molécules chaperons sont de petites molécules favorisant le repliement correct des enzymes mutantes et permettant leur transport vers les lysosomes (Boyd et al., 2013, J. Med. Chem. 56: 2705-2725) plutôt qu'une dégradation au niveau du réticulum endoplasmique. Cette approche thérapeutique a été mise en oeuvre avec succès pour une autre maladie lysosomale, la maladie de Fabry, contre laquelle l'iminosucre migalastat (Galafold®) est désormais prescrit comme chaperon pharmacologique de l'α-galactosidase acide (Markham, 2016, Drugs 76: 1147-1152).

Les présents inventeurs ont ainsi recherché des composés capables de stabiliser l'α-glucosidase acide humaine dans sa forme repliée active, ainsi que d'accroitre l'efficacité de l'α-glucosidase acide humaine recombinante mise en oeuvre dans les traitements par thérapie enzymatique substitutive, pour les utiliser dans le traitement de la maladie de Pompe.

Parmi les composés existants, la déoxynojirimycine (DNJ), un iminosucre de formule :

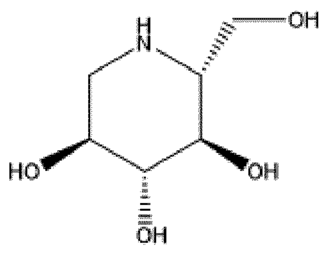

a été proposée par l'art antérieur en tant que potentielle molécule chaperon de l'α-glucosidase acide humaine (Flanagan

et al., 2009, Hum. Mutat. 30: 1683-1692).

Un dérivé de la DNJ, nommé NB-DNJ, de formule :

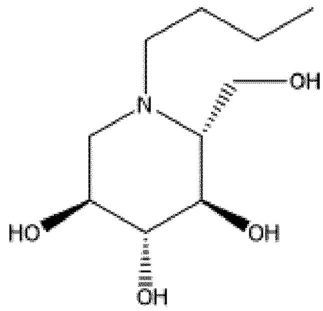

a également été proposé par l'art antérieur pour la même application (Parenti et al., 2007, Mol. Ther. 15(3): 508-14).

Cependant, ces composés présentent une faible sélectivité pour cette enzyme, et ils inhibent d'autres enzymes humaines, tant de type α- que de type β-glycosidases, et glycosyltransférases, ce qui cause de nombreux effets secondaires indésirables lorsqu'ils sont administrés à un sujet.

La publication de Boisson et al., Org. Lett., 2015, 17(15), 3662-5 décrit des dérivés polyhydroxylés d'indolizidines particuliers présentant une bonne capacité d'inhibition d'une α-glucosidase de *S. cerevisiae* et d'une α-glucosidase de riz.

La publication de Tangara et al., Org. Lett., 2017, 19(18), 4842-5 décrit des aziridinyl-iminosucres particuliers.

La publication de Vieira Da Cruz et al., J. Org. Chem., 2017, 82(18), 9866-72 décrit quant à elle des quinolizidines polyhydroxylées particulières et leur capacité d'inhibition d'α-glucosidases de levure et de riz.

Les présents inventeurs ont maintenant découvert que des composés spécifiques, de la classe des iminosucres, répondant à une structure particulière dérivée de celle de la DNJ, se lient à l'α-glucosidase acide humaine (GAA) et la stabilisent, ceci de manière hautement sélective, c'est-à-dire sans interagir, du moins de manière significative, avec d'autres glycosidases humaines, notamment avec les β-glucocérébrosidases humaines (GBA1 et GBA2), la β-glucosylcéramide transférase humaine (GCS) ou avec l'α-glucosidase II du réticulum endoplasmique humaine (GANAB). L'effet de stabilisation de la GAA par ces composés est en outre particulièrement important. Ainsi, administrés à des patients atteints de la maladie de Pompe, ces composés permettent de stabiliser leur α-glucosidase acide endogène dans une forme correctement repliée pour améliorer son transport jusqu'aux lysosomes et augmenter son activité d'hydrolyse du glycogène, et donc de traiter avec efficacité cette maladie, en réduisant les effets secondaires indésirables au cours du traitement. Ces composés permettent également, associés à une thérapie enzymatique substitutive (TES), de stabiliser l'enzyme recombinante (rhGAA), augmentant par là-même son efficacité.

Les molécules de structure proche, mais différente, telles que la DNJ ou la NB-DNJ, ne permettent pas d'obtenir un tel résultat particulièrement avantageux sur le plan thérapeutique.

Ainsi, selon un premier aspect, la présente invention concerne un composé de formule générale (I) ci-dessous, ou l'un de ses sels pharmaceutiquement acceptables, pour son utilisation en tant que médicament, en particulier comme chaperon pharmacologique, notamment pour le traitement de la maladie de Pompe, en particulier pour la stabilisation de l'α-glucosidase acide humaine :

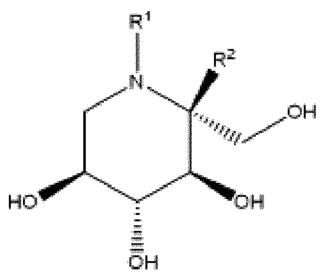

(I)

dans laquelle:

- $R^1$ représente un atome d'hydrogène ou un radical hydrocarboné linéaire, ramifié et/ou cyclique, saturé ou insaturé, aromatique ou non, éventuellement substitué, pouvant comporter un ou plusieurs hétéroatomes et/ou un ou plusieurs groupements comportant au moins un hétéroatome et pouvant comporter un seul cycle ou plusieurs cycles le cas échéant fusionnés,

et $R^2$ représente un radical hydrocarboné linéaire, ramifié et/ou cyclique, saturé ou insaturé, aromatique ou non, éventuellement substitué, pouvant comporter un ou plusieurs hétéroatomes et/ou un ou plusieurs groupements comportant au moins un hétéroatome et pouvant comporter un seul cycle ou plusieurs cycles, le cas échéant fusionnés, ledit radical hydrocarboné comportant au moins 2 atomes de carbone lorsque $R^1$ représente un atome d'hydrogène,

- ou $R^1$ et $R^2$ forment ensemble, avec les atomes du cycle pipéridine auquel chacun est rattaché, un hétérocycle de 3 à 6 chaînons fusionné avec le cycle pipéridine, éventuellement substitué par un ou plusieurs radicaux, pouvant être identiques ou différents, choisis chacun parmi un groupement hydroxyle, un groupement amino ou un radical carboné, comportant de préférence de 1 à 18, préférentiellement de 1 à 12 et notamment de 1 à 6, atomes de carbone, linéaire, ramifié et/ou cyclique, saturé ou insaturé, aromatique ou non, éventuellement substitué, pouvant comporter un ou plusieurs hétéroatomes et/ou un ou plusieurs groupements comportant au moins un hétéroatome et pouvant comporter un seul cycle ou plusieurs cycles le cas échéant fusionnés. Une telle configuration englobe les cas dans lesquels le radical carboné est branché à l'hétérocycle à 3 à 6 chaînons par un atome de carbone, ainsi que les cas dans lesquels le radical carboné y est branché par l'intermédiaire d'un hétéroatome qu'il comporte.

Le terme hétéroatome désigne dans la présente description, de manière classique en elle-même, tout atome appartenant à un autre élément que le carbone et l'hydrogène, tel qu'un atome d'azote, d'oxygène, de soufre, de phosphore, de silicium, d'halogène, etc.

Préférentiellement, lorsque $R^1$ représente un atome d'hydrogène, $R^2$ ne représente pas un radical méthyle ou un groupement benzyle.

On entend dans la présente description, par « sel pharmaceutiquement acceptable », de manière classique en elle-même, tout sel du composé de formule générale (I) comprenant, en tant que contre-ion, une substance qui ne produit aucune réaction adverse, allergique ou autrement indésirable lorsqu'elle est administrée à un sujet, en particulier à un mammifère.

Tout sel conventionnel pharmaceutiquement acceptable du composé de formule générale (I), peut être mis en oeuvre selon l'invention. A titre d'exemples, on peut citer les chlorures, bromures, formiates, acétates, etc.

On entend dans la présente description, par le terme « traitement », l'obtention d'un effet pharmacologique et physiologique souhaité. Le terme « traitement », tel qu'il est utilisé dans la présente description, comprend la prévention ou la prévention partielle d'un ou plusieurs des symptômes de la maladie et/ou la guérison partielle ou totale de la maladie et/ou la disparition totale ou partielle d'un ou plusieurs de ses symptômes.

L'$\alpha$-glucosidase acide humaine est une protéine de 952 acides aminés, de n° d'accession GenBank ABI53718.1.

Le composé utilisé selon l'invention, répondant à la formule générale (I), en est un ligand. Il présente en outre une sélectivité particulièrement forte vis-à-vis de cette enzyme. Cette sélectivité est notamment bien plus importante que celle de la DNJ ou de la NB-DNJ, ou d'autres iminosucres interagissant avec des glycosidases.

On ne préjugera pas ici des caractéristiques du composé utilisé selon l'invention, relevant notamment de sa structure tridimensionnelle, qui permettent l'obtention de telles propriétés avantageuses. On peut cependant supposer qu'y participent : la présence d'un centre quaternaire en position $\alpha$ de l'azote du noyau pipéridine, la configuration D-*gluco* du composé, et les substituants particuliers présents sur le carbone quaternaire en position $\alpha$ de l'azote du noyau pipéridine et sur cet azote. Grâce, au moins en partie, à ces caractéristiques, le composé utilisé selon l'invention se lierait de façon non covalente avec l'enzyme et en stabiliserait le repliement, lui conférant un rôle de chaperon permettant de restaurer le transport et l'activité de l'enzyme déficiente dans les lysosomes. Le composé utilisé selon l'invention stabilise ainsi l'$\alpha$-glucosidase acide humaine à de faibles concentrations, et il possède les propriétés essentielles de chaperons pharmacologiques appropriés pour le traitement de la maladie de Pompe. En particulier, à une concentration de 100 $\mu$M, il augmente, *in vitro,* de 8 à 12 °C la température de dénaturation thermique de l'$\alpha$-glucosidase acide humaine à pH 4,0 et de 10 à 13 °C la température de dénaturation thermique de l'$\alpha$-glucosidase acide humaine à pH 7,4. L'effet chaperon du composé utilisé selon l'invention sur l'$\alpha$-glucosidase acide humaine est confirmé par des expériences sur des cellules humaines en culture, plus précisément des fibroblastes de patients atteints par la maladie, ainsi que, *in vivo,* chez la souris. Le composé utilisé selon l'invention est ainsi tout à fait adapté et avantageux pour être mis en oeuvre en tant que chaperon pharmacologique pour le traitement de la maladie de Pompe, par liaison sélective à l'$\alpha$-glucosidase acide humaine et stabilisation de cette dernière, restaurant ainsi l'activité de l'enzyme déficiente du patient, et/ou améliorant la biodisponibilité de l'enzyme recombinante rhGAA administrée lors d'une thérapie par enzyme substitutive rhGAA lorsque cette thérapie est associée à la mise en oeuvre du composé utilisé selon l'invention.

Ainsi, le composé de formule générale (I) peut être utilisé pour stabiliser l'$\alpha$-glucosidase acide humaine dans sa forme repliée active, ainsi qu'accroitre l'efficacité de l'$\alpha$-glucosidase acide humaine recombinante mise en oeuvre dans les traitements par thérapie enzymatique substitutive, le composé de formule générale (I) et l'$\alpha$-glucosidase acide humaine recombinante étant coadministrées au patient.

Le composé utilisé selon l'invention peut avantageusement être administré au patient facilement, notamment par voie orale, et il est peu onéreux à préparer.

Il présente un faible risque de toxicité, et une bonne biodisponibilité.

En outre, en association avec la thérapie enzymatique substitutive, le composé utilisé selon l'invention améliore avantageusement la biodisponibilité de l'enzyme recombinante injectée, ce qui permet d'en diminuer les doses pour obtenir une même efficacité de traitement, et même une efficacité supérieure. Le composé utilisé selon l'invention, de formule générale (I), peut être administré à tout sujet en ayant besoin, c'est-à-dire atteint ou susceptible d'être atteint par la maladie. Ce sujet peut notamment être un mammifère, et en particulier un humain.

Le composé utilisé selon l'invention est de préférence administré au sujet dans une quantité thérapeutiquement efficace.

Par "quantité thérapeutiquement efficace", on entend la quantité du composé qui, lorsqu'il est administré à un sujet pour traiter la maladie, est suffisante pour assurer un tel traitement de la maladie.

La quantité thérapeutiquement efficace du composé utilisé selon l'invention dépend de plusieurs facteurs, tels que la maladie et sa gravité, l'âge, le poids, etc., du sujet à traiter, le composé particulier utilisé, la voie et la forme d'administration, etc. La quantité thérapeutiquement efficace du composé utilisé selon l'invention sera déterminée par le médecin pour chaque cas individuel.

L'administration du composé utilisé selon l'invention au sujet à traiter peut être réalisée par toute voie classique en elle-même, notamment par voie parentérale, par exemple par voie sous-cutanée, sous-durale, intraveineuse, intramusculaire, intrathécale, intrapéritonéale, intracérébrale, intra-artérielle ou intra-lésionnelle ; par voie intranasale ; par voie rectale ; par voie pulmonaire, par exemple par aérosol ou inhalation ; ou même par voie topique. Elle est préférentiellement réalisée par voie orale.

La détermination de la posologie d'administration du composé utilisé selon l'invention relève des compétences du médecin. Le composé peut par exemple être administré au sujet en ayant besoin une ou deux fois par jour, sur une longue période, à intervalles réguliers, ou de manière ciblée lors d'un traitement par thérapie enzymatique substitutive associé.

Comme exposé ci-avant, le composé utilisé selon l'invention peut avantageusement être administré au sujet conjointement à une enzyme mise en oeuvre pour la thérapie enzymatique substitutive, notamment conjointement à une $\alpha$-glucosidase acide humaine recombinante telle que l'enzyme recombinante commercialisée sous le nom Myozyme®. Il en augmente alors avantageusement l'efficacité.

Dans des modes de mise en oeuvre particuliers de l'invention, dans la formule générale (I) $R^1$ et $R^2$ sont indépendants l'un de l'autre, en ce sens qu'ils ne sont pas liés l'un à l'autre, notamment qu'ils ne forment pas ensemble un cycle fusionné avec le noyau pipéridine du composé.

En particulier, dans la formule générale (I) :

$R^1$ représente alors un atome d'hydrogène ou un radical hydrocarboné linéaire, ramifié et/ou cyclique, saturé ou insaturé, aromatique ou non, éventuellement substitué, pouvant comporter un ou plusieurs hétéroatomes et/ou un ou plusieurs groupements comportant au moins un hétéroatome et pouvant comporter un seul cycle ou plusieurs cycles le cas échéant fusionnés,

et $R^2$ peut représenter un groupement $-CH(R^3)-R^4$, où $R^3$ et $R^4$, pouvant être identiques ou différents, représentent chacun un atome d'hydrogène ou un radical hydrocarboné linéaire, ramifié et/ou cyclique, saturé ou insaturé, aromatique ou non, éventuellement substitué, pouvant comporter un ou plusieurs hétéroatomes et/ou un ou plusieurs groupements comportant au moins un hétéroatome et pouvant comporter un seul cycle ou plusieurs cycles, le cas échéant fusionnés, $R^3$ et $R^4$ ne représentant pas simultanément un atome d'hydrogène lorsque $R^1$ représente un atome d'hydrogène.

Dans des modes de mise en oeuvre particuliers de l'invention, $R^3$ et $R^4$ ne représentent pas simultanément un atome d'hydrogène.

Dans des modes de mise en oeuvre particuliers de l'invention, $R^1$ et $R^2$ sont tels qu'ils ne représentent pas simultanément, respectivement, un radical propyle et un radical éthyle.

Préférentiellement, $R^3$ et $R^4$ sont tels que, lorsque $R^1$ et $R^3$ représentent chacun un atome d'hydrogène, $R^4$ ne représente pas un radical phényle.

Dans des modes de mise en oeuvre particuliers de l'invention, $R^2$ représente un groupement $-CH(R^3)-R^4$, où $R^3$ est tel que défini ci-dessus et $R^4$ représente un radical hydrocarboné, comportant de préférence de 1 à 18, préférentiellement de 1 à 12 et notamment de 1 à 6, atomes de carbone, linéaire, ramifié et/ou cyclique, saturé ou insaturé, aromatique ou non, éventuellement substitué, pouvant comporter un seul cycle ou plusieurs cycles, le cas échéant fusionnés, et comportant un ou plusieurs hétéroatomes choisis chacun parmi l'oxygène, l'azote, le soufre et le silicium et/ou un ou plusieurs groupements comportant au moins un hétéroatome choisis chacun parmi les groupements carbonyle, sulfoxyde, sulfonyle et silane.

Autrement, $R^1$ représentant un atome d'hydrogène ou un radical hydrocarboné linéaire, ramifié et/ou cyclique, saturé ou insaturé, aromatique ou non, éventuellement substitué, pouvant comporter un ou plusieurs hétéroatomes

et/ou un ou plusieurs groupements comportant au moins un hétéroatome et pouvant comporter un seul cycle ou plusieurs cycles le cas échéant fusionnés, $R^2$ peut représenter un groupement $-(CHR^7)-SO_2-Ar^1$ où $Ar^1$ représente un radical aryle ou hétéroaryle, le cas échéant substitué, comportant notamment de 5 à 18 atomes, et $R^7$ représente un atome d'hydrogène ou un radical hydrocarboné, comportant de préférence de 1 à 18, préférentiellement de 1 à 12 et notamment de 1 à 6, atomes de carbone, linéaire, ramifié et/ou cyclique, saturé ou insaturé, aromatique ou non, éventuellement substitué, pouvant comporter un ou plusieurs hétéroatomes et/ou un ou plusieurs groupements comportant au moins un hétéroatome et pouvant comporter un seul cycle ou plusieurs cycles, le cas échéant fusionnés. Dans des modes de mise en oeuvre particuliers de l'invention, $Ar^1$ représente un hétérocycle aromatique, notamment un cycle pyridine, le cas échéant substitué, et/ou $R^7$ représente un atome d'hydrogène.

Dans des modes de mise en oeuvre particuliers de l'invention, $R^1$ représentant un atome d'hydrogène ou un radical hydrocarboné linéaire, ramifié et/ou cyclique, saturé ou insaturé, aromatique ou non, éventuellement substitué, pouvant comporter un ou plusieurs hétéroatomes et/ou un ou plusieurs groupements comportant au moins un hétéroatome et pouvant comporter un seul cycle ou plusieurs cycles le cas échéant fusionnés, $R^2$ peut représenter un groupement triazole, le cas échéant substitué.

Dans d'autres variantes de l'invention, $R^1$ représentant un atome d'hydrogène ou un radical hydrocarboné linéaire, ramifié et/ou cyclique, saturé ou insaturé, aromatique ou non, éventuellement substitué, pouvant comporter un ou plusieurs hétéroatomes et/ou un ou plusieurs groupements comportant au moins un hétéroatome et pouvant comporter un seul cycle ou plusieurs cycles le cas échéant fusionnés, $R^2$ représente un groupement $-(CH_2)_2-R^8$, où $R^8$ représente :

- un atome d'hydrogène ;
- un groupement alkyle ou cycloalkyle en C1-C12, pouvant comporter un seul cycle ou plusieurs cycles fusionnés, tel qu'un groupement adamantyle par exemple ;
- un groupement $-(CH_2)_a-OH$ où a est en nombre entier compris entre 0 et 18, de préférence compris entre 0 et 12 et notamment compris entre 0 et 6 ;
- un groupement $-(CH_2)_b-Ar^2$ où $Ar^2$ représente un radical aryle ou hétéroaryle éventuellement substitué, pouvant comporter un ou plusieurs hétéroatomes et/ou un ou plusieurs groupements comportant au moins un hétéroatome et pouvant comporter un seul cycle ou plusieurs cycles, le cas échéant fusionnés et b est en nombre entier compris entre 0 et 18, de préférence compris entre 0 et 12 et notamment compris entre 0 et 6 ;
- un groupement $-(CH_2)_c-Si(R^9)_3$ où $R^9$ représente un radical hydroxyle, un radical alkyle en C1-C4, un radical alcoxyle en C1-C4 ou un radical phényle et c est un nombre entier compris entre 0 et 18, de préférence compris entre 0 et 12 et notamment compris entre 0 et 6 ;
- ou un groupement $-(CH_2)_d-Z-R^{10}$ où Z est un hétéroatome choisi parmi l'oxygène, l'azote et le soufre, $R^{10}$ représente un atome d'hydrogène ou un radical alkyle, cycloalkyle, alkylaryle, aryle ou acyle en C1-C18, de préférence en C1-C12 et notamment en C1-C6, ledit radical étant éventuellement interrompu et/ou substitué par un ou plusieurs hétéroatomes et/ou un ou plusieurs groupements comportant au moins un hétéroatome, notamment un radical sulfonyle, et d est en nombre entier compris entre 0 et 18, de préférence compris entre 0 et 12 et notamment compris entre 0 et 6.

Dans des modes de réalisation particulièrement préférés de l'invention, $R^1$ représente un atome d'hydrogène ou un groupe alkyle linéaire, ramifié et/ou cyclique en C1-C18, de préférence en C1-C6, par exemple en C1-C3 et plus particulièrement en C1-C2, ce groupe alkyle étant éventuellement interrompu et/ou substitué par un ou plusieurs hétéroatomes et/ou un ou plusieurs groupements comportant au moins un hétéroatome.

Le composé utilisé selon l'invention peut notamment répondre à la formule générale (I'a) :

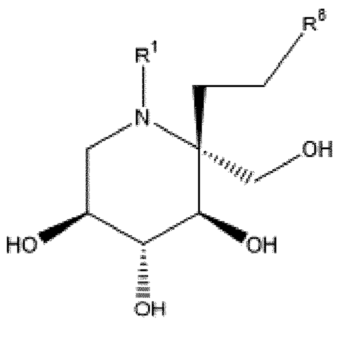

(I'a)

dans laquelle :

$R^1$ représente un atome d'hydrogène ou groupe alkyle linéaire, ramifié et/ou cyclique en C1-C18, de préférence en C1-C6, éventuellement interrompu et/ou substitué par un ou plusieurs hétéroatomes et/ou un ou plusieurs groupements comportant au moins un hétéroatome,
et $R^8$ représente :

- un atome d'hydrogène ;
- un radical méthyle, éthyle, propyle, butyle, pentyle, hexyle, cycloalkyle, adamantyle, alkylcycloalkyle, alkylaryle ou aryle, notamment phényle, ledit radical étant éventuellement interrompu et/ou substitué par un ou plusieurs hétéroatomes et/ou un ou plusieurs groupements comportant au moins un hétéroatome ;
- un groupement hydroxyle ;
- un groupement $-Si(R^{12})_3$ où $R^{12}$ représente un radical hydroxyle, un radical alkyle en C1-C4, un radical alcoxyle en C1-C4 ou un radical phényle ;
- un groupement $-(CH_2)_f-Y-R^{13}$ où f est un nombre entier compris entre 0 et 6, Y est un hétéroatome choisi parmi l'oxygène, l'azote et le soufre et $R^{13}$ représente un radical alkyle en C1-C18, de préférence en C1-C12 et notamment en C1-C6, ou un radical aryle ou hétéroaryle en C1-C18, de préférence en C1-C12 et notamment en C1-C6 ;
- un groupement $-(CH_2)_g-CO-R^{13}$ où g est un nombre entier compris entre 0 et 6 et $R^{13}$ est tel que défini ci-dessus ;
- ou un groupement $-(CH_2)_h-SO_e-R^{13}$ où h est un nombre entier compris entre 0 et 6, e est égal à 1 ou 2 et $R^{13}$ est tel que défini ci-dessus,

$R^8$ ne représentant pas un atome d'hydrogène lorsque $R^1$ représente un radical propyle.

Dans des modes de mise en oeuvre particuliers de l'invention, le composé utilisé répond à la formule générale (I"a) :

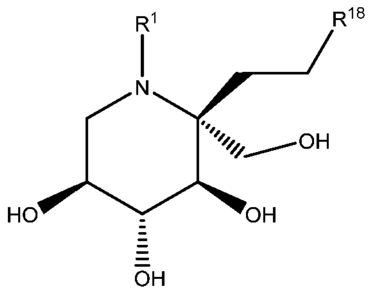

(I"a)

dans laquelle:

$R^1$ représente un atome d'hydrogène ou un groupe alkyle linéaire, ramifié et/ou cyclique en C1-C18, éventuellement interrompu et/ou substitué par un ou plusieurs hétéroatomes et/ou un ou plusieurs groupements comportant au moins un hétéroatome,
$R^{18}$ représente un radical hydrocarboné linéaire, ramifié et/ou cyclique, saturé ou insaturé, aromatique ou non, éventuellement substitué, comportant de 4 à 18 atomes de carbone, de préférence de 4 à 12 atomes de carbone, et notamment de 5 à 12 atomes de carbone, pouvant comporter un ou plusieurs hétéroatomes et/ou un ou plusieurs groupements comportant au moins un hétéroatome et pouvant comporter un seul cycle ou plusieurs cycles le cas échéant fusionnés. $R^{18}$ peut notamment représenter un groupement butyle, pentyle, hexyle, cyclohexyle, phényle, benzyle, adamantyle.

Préférentiellement, dans la formule (I"a), $R^1$ représente un atome d'hydrogène. Des composés particuliers utilisables selon l'invention répondent aux formules (IIa), (IIb), (IIb1), (IIb2), (IIb3), (IIb4), (IIb5), (IIb6), (IIb7), (IIc), (IIc1), (IId), (IIe), (IIo), (IIp), (IIp1), (IIq), (IIr), (IIs), (IIt), (IIu), (IIv), (IIw), (IIw1), (IIx), (IIx1) et (IIy) suivantes :

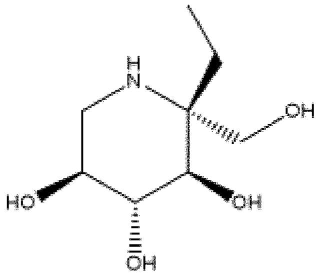

(IIa)

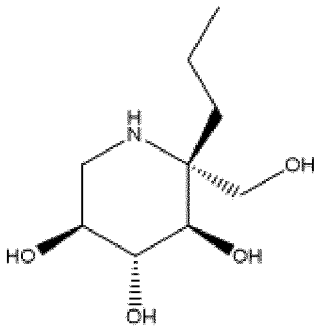

(IIb)

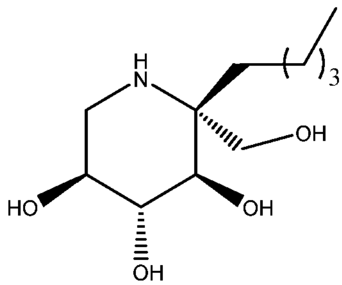

(IIb1)

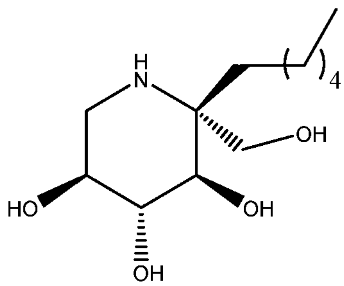

(IIb2)

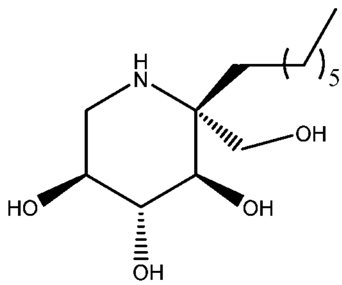

(IIb3)

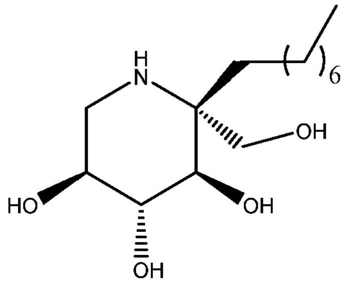

(IIb4)

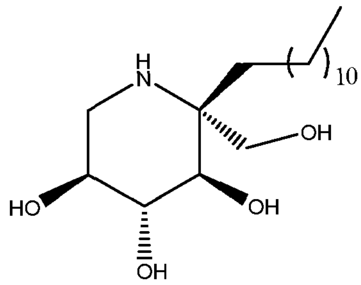

(IIb5)

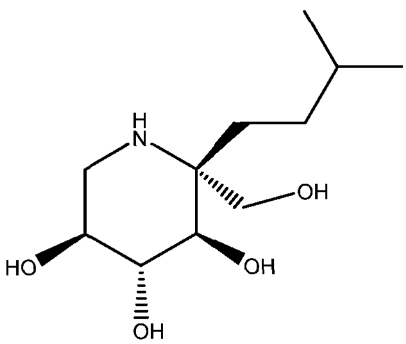

(IIb6)

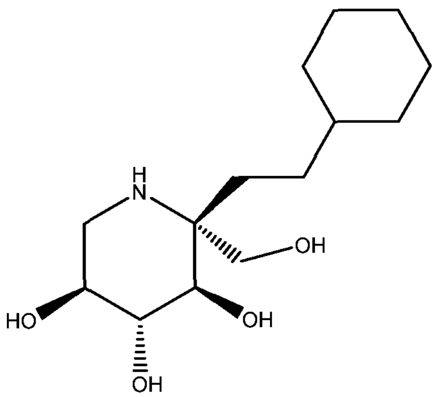

(IIb7)

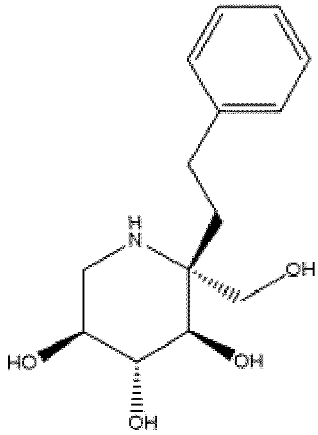

(IIc)

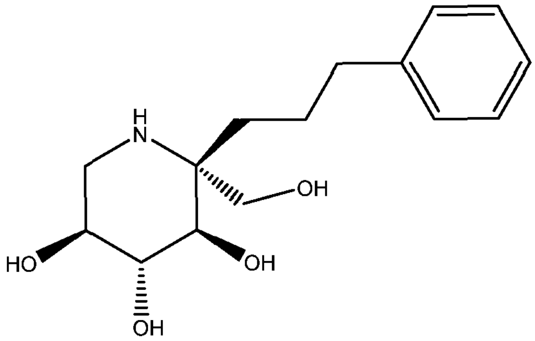

(IIc1)

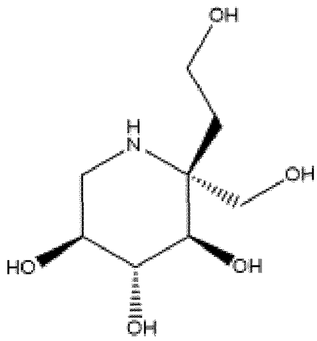

(IId)

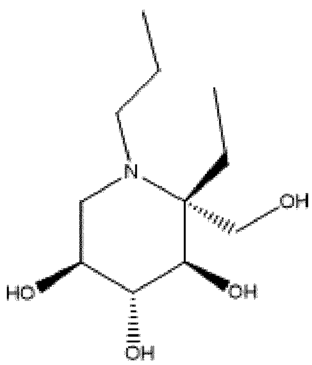

(IIe)

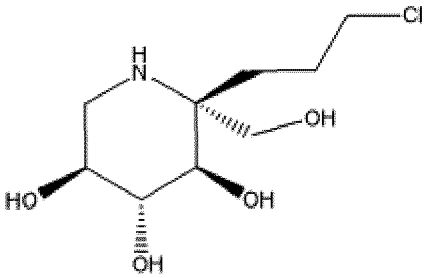

(IIo)

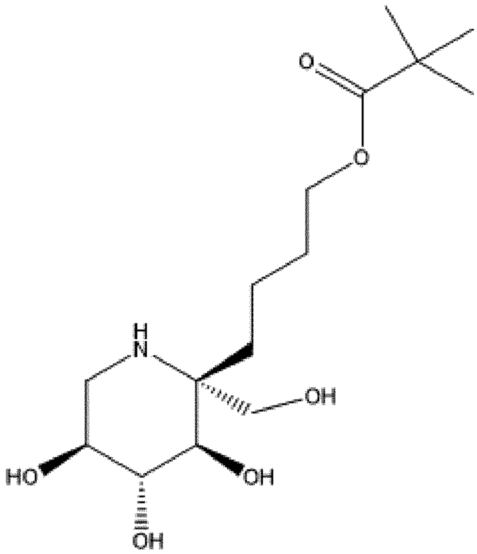

(IIp)

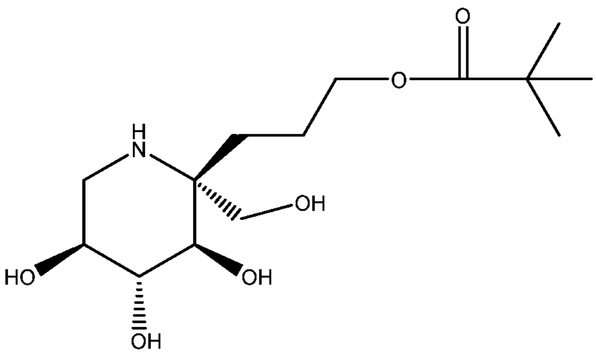

(IIp1)

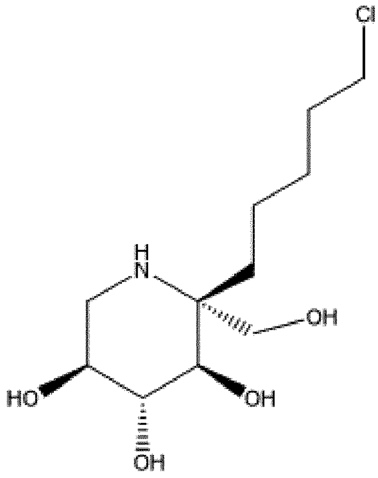

(IIq)

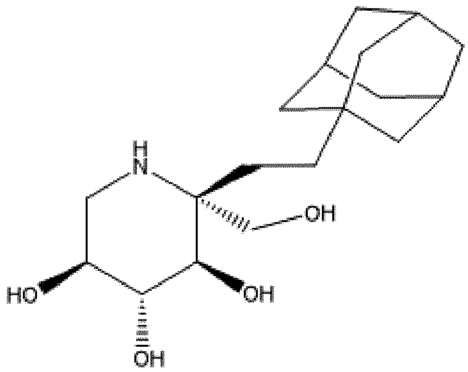

(IIr)

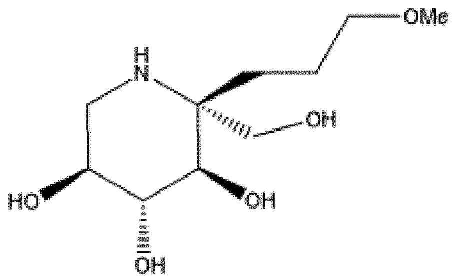

(IIs)

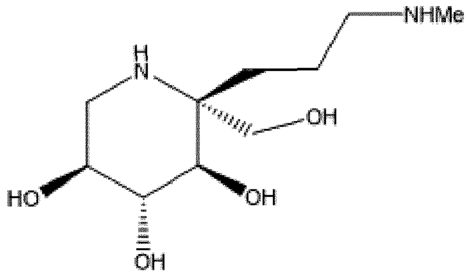

(IIt)

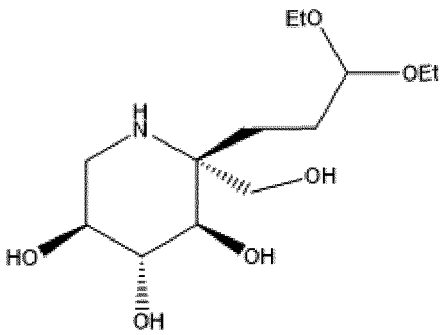

(IIu)

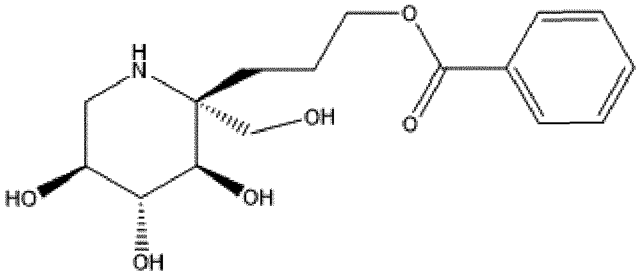

(IIv)

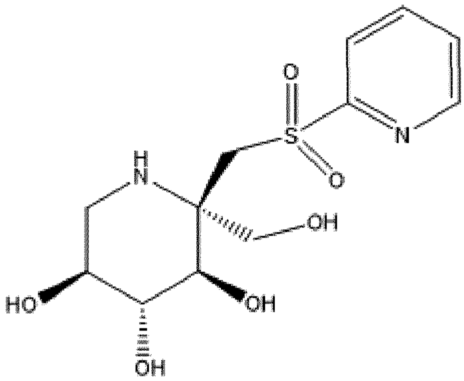

(IIw)

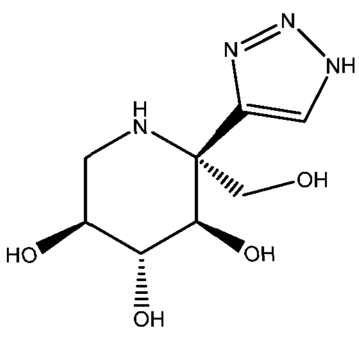

(IIw1)

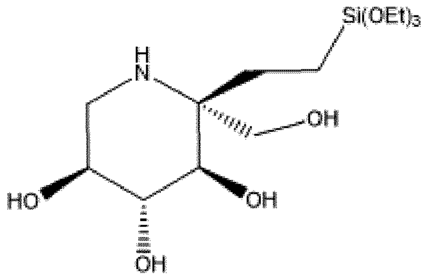

(IIx)

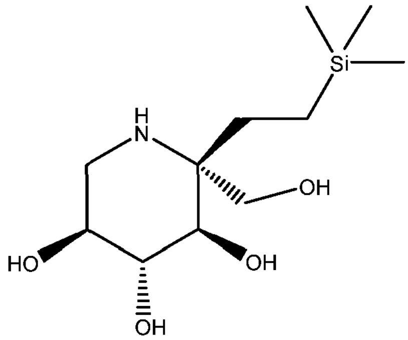

(IIx1)

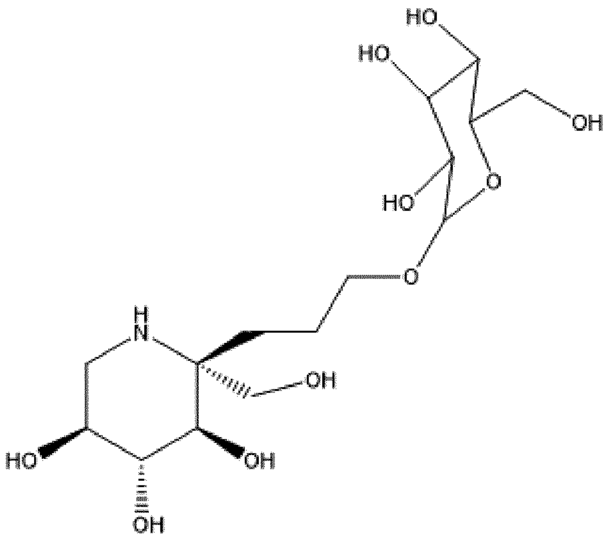

(IIy)

Dans des modes de mise en oeuvre alternatifs de l'invention, le composé utilisé est tel que, dans la formule générale (I), $R^1$ et $R^2$ forment ensemble, avec les atomes du cycle pipéridine auquel chacun est rattaché, un hétérocycle à 6 chainons fusionné avec le cycle pipéridine, éventuellement substitué par un ou plusieurs radicaux, pouvant être identiques ou différents, choisis chacun parmi un groupement hydroxyle, un groupement amino, un groupement carbonyle ou un radical carboné, comportant de préférence de 1 à 18, préférentiellement de 1 à 12 et notamment de 1 à 6, atomes de carbone, linéaire, ramifié et/ou cyclique, saturé ou insaturé, aromatique ou non, éventuellement substitué, pouvant comporter un ou plusieurs hétéroatomes et/ou un ou plusieurs groupements comportant au moins un hétéroatome et pouvant comporter un seul cycle ou plusieurs cycles, le cas échéant fusionnés.

Un composé particulier utilisable selon l'invention répond à la formule (IIf) :

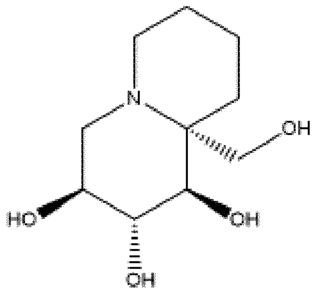

(IIf)

Dans d'autres modes de mise en oeuvre de l'invention, le composé utilisé est tel que, dans la formule générale (I), $R^1$ et $R^2$ forment ensemble, avec les atomes du cycle pipéridine auquel chacun est rattaché, un hétérocycle à 5 chainons fusionné avec le cycle pipéridine, éventuellement substitué par un ou plusieurs radicaux, pouvant être identiques ou différents, choisis chacun parmi un groupement hydroxyle, un groupement amino, un groupement carbonyle ou un radical carboné, comportant de préférence de 1 à 18, préférentiellement de 1 à 12 et notamment de 1 à 6, atomes de carbone, linéaire, ramifié et/ou cyclique, saturé ou insaturé, aromatique ou non, éventuellement substitué, pouvant comporter un ou plusieurs hétéroatomes et/ou un ou plusieurs groupements comportant au moins un hétéroatome et pouvant comporter un seul cycle ou plusieurs cycles, le cas échéant fusionnés.

Un composé particulier utilisable selon l'invention répond à la formule (IIg) :

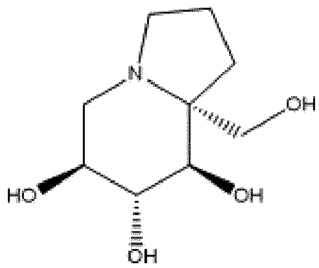

(IIg)

Dans des modes de mise en oeuvre encore alternatifs de l'invention, le composé utilisé est tel que, dans la formule générale (I), $R^1$ et $R^2$ forment ensemble, avec les atomes du cycle pipéridine auquel chacun est rattaché, un hétérocycle à 4 chainons fusionné avec le cycle pipéridine, éventuellement substitué par un ou plusieurs radicaux, pouvant être identiques ou différents, choisis chacun parmi un groupement hydroxyle, un groupement amino ou un radical carboné, comportant de préférence de 1 à 18, préférentiellement de 1 à 12 et notamment de 1 à 6, atomes de carbone, linéaire, ramifié et/ou cyclique, saturé ou insaturé, aromatique ou non, éventuellement substitué, pouvant comporter un ou plusieurs hétéroatomes et/ou un ou plusieurs groupements comportant au moins un hétéroatome et pouvant comporter un seul cycle ou plusieurs cycles, le cas échéant fusionnés.

Le composé peut notamment répondre à la formule générale (I'b) :

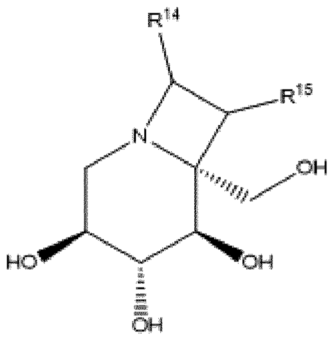

(I'b)

dans laquelle

$R^{14}$ représente un atome d'hydrogène, un radical carbonyle ou un radical alkyle, alcényle, alcynyle, alkylaryle ou aryle en C1-C18, de préférence en C1-C12 et notamment en C1-C6, ledit radical étant éventuellement interrompu et/ou substitué par un ou plusieurs hétéroatomes et/ou un ou plusieurs groupements comportant au moins un hétéroatome,

et $R^{15}$ représente un atome d'hydrogène, un radical hydroxyle, un radical amino, ou un radical alkyle, alcényle ou aryle en C1-C18, de préférence en C1-C12 et notamment en C1-C6, ledit radical étant éventuellement interrompu et/ou substitué par un ou plusieurs hétéroatomes et/ou un ou plusieurs groupements comportant au moins un hétéroatome.

Des composés particuliers utilisables selon l'invention répondent notamment aux formule (IIh), (IIz1), (IIz2), (IIz3), (IIz4) et (IIz5) :

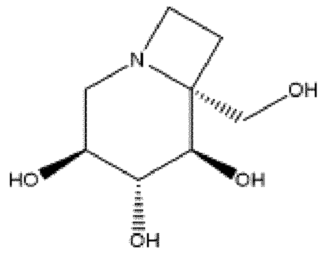

(IIh)

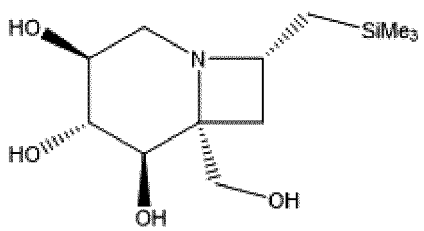

(IIz1)

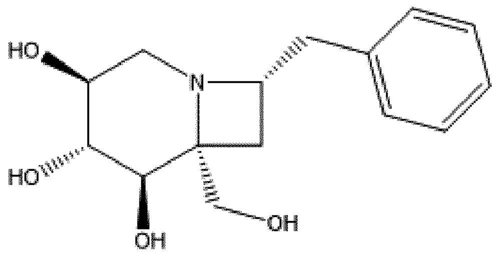

(IIz2)

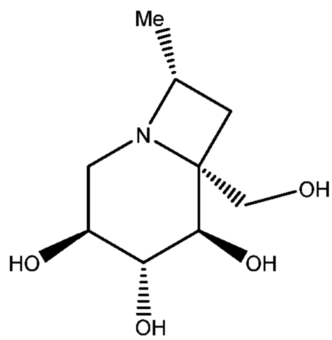

(IIz3)

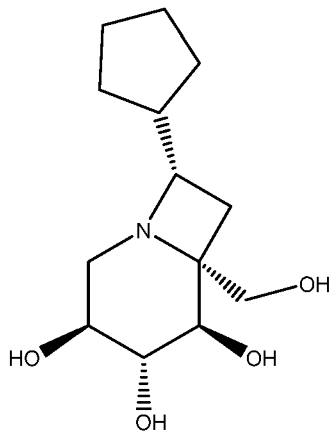

(IIz4)

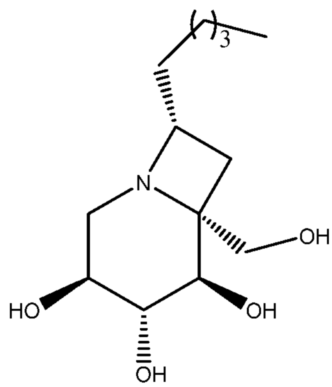

(IIz5)

Dans des modes de réalisation encore alternatifs de l'invention, le composé utilisé est tel que, dans la formule générale (I), $R^1$ et $R^2$ forment ensemble, avec les atomes du cycle pipéridine auquel chacun est rattaché, un hétérocycle à 3 chainons fusionné avec le cycle pipéridine, éventuellement substitué par un groupement -X-$R^5$, dans lequel :

- X représente un radical -C(=O)- ou -CH(O$R^6$)- où $R^6$ représente un radical hydrocarboné linéaire, ramifié et/ou cyclique, saturé ou insaturé, aromatique ou non, éventuellement substitué, pouvant comporter un ou plusieurs hétéroatomes et/ou un ou plusieurs groupements comportant au moins un hétéroatome et pouvant comporter un seul cycle ou plusieurs cycles, le cas échéant fusionnés, et $R^5$ représente un atome d'hydrogène, un groupement amino, ou un radical hydrocarboné linéaire, ramifié et/ou cyclique, saturé ou insaturé, aromatique ou non, éventuellement substitué, pouvant comporter un ou plusieurs hétéroatomes et/ou un ou plusieurs groupements comportant au moins un hétéroatome et pouvant comporter un seul cycle ou plusieurs cycles, le cas échéant fusionnés,
- ou X représente un radical -CH(OH)- et $R^5$ représente un atome d'hydrogène, ou un radical hydrocarboné linéaire, ramifié et/ou cyclique, saturé ou insaturé, aromatique ou non, éventuellement substitué, pouvant comporter un ou plusieurs hétéroatomes et/ou un ou plusieurs groupements comportant au moins un hétéroatome et pouvant comporter un seul cycle ou plusieurs cycles, le cas échéant fusionnés. Dans cette dernière configuration, en particulier, $R^5$ ne représente préférentiellement pas un radical n-propyle non substitué, un radical c-hexyle non substitué ou un radical phényle non substitué.

Le composé utilisé selon l'invention peut notamment répondre à la formule générale (I'c) :

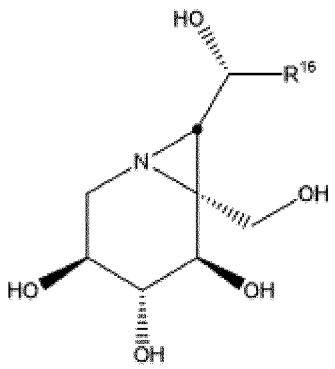

(I'c)

dans laquelle $R^{16}$ représente un atome d'hydrogène ou un radical alkyle en C1-C18, de préférence en C1-C12 et préférentiellement en C1-C6, ledit radical étant éventuellement interrompu et/ou substitué par un ou plusieurs hétéroatomes et/ou un ou plusieurs groupements comportant au moins un hétéroatome.

En particulier, $R^{16}$ peut représenter un groupement $-CH_2-OH$, un radical méthyle ou un radical éthyle.

Des composés particuliers utilisables selon l'invention répondent aux formules (IIj), (IIk), (IIm) et (IIn) :

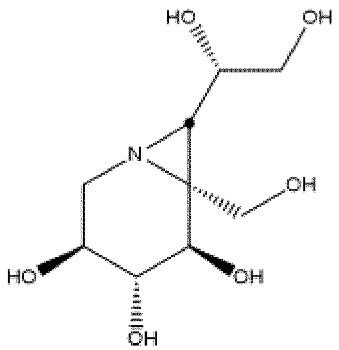

(IIj)

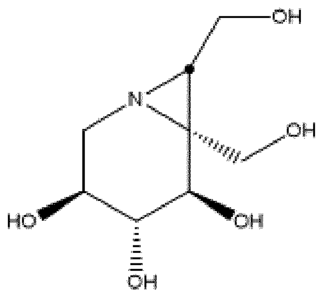

(IIk)

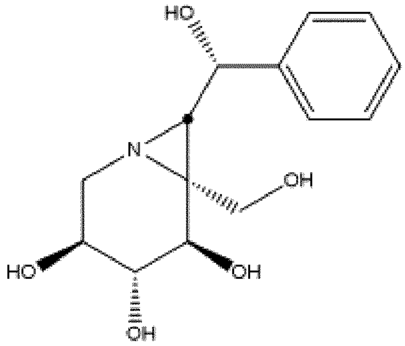

(IIm)

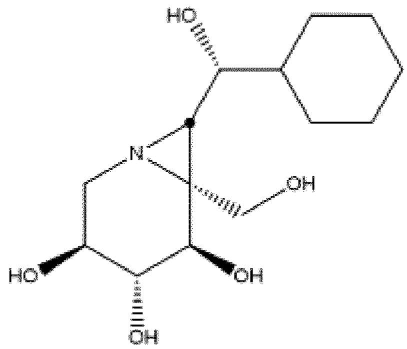

(IIn)

L'invention s'exprime également dans les termes d'un procédé de traitement thérapeutique d'un sujet souffrant ou susceptible de souffrir d'une maladie, en particulier de la maladie de Pompe, ce procédé comprenant une étape d'administration audit sujet en ayant besoin d'une quantité thérapeutiquement efficace d'un composé répondant à la formule générale (I) ou un de ses sels pharmaceutiquement acceptables. Ce procédé peut répondre à l'une ou plusieurs des caractéristiques décrites ci-avant en référence à l'utilisation thérapeutique du composé de formule générale (I), ou un de ses sels pharmaceutiquement acceptables, en tant que médicament.

L'invention concerne également l'utilisation d'un composé de formule générale (I), ou un de ses sels pharmaceutiquement acceptables, tel que défini ci-avant, pour la fabrication d'un médicament, notamment d'un médicament pour traiter la maladie de Pompe.

Selon un autre aspect, la présente invention concerne une composition pharmaceutique contenant, en tant que principe actif, un composé répondant à la formule générale (I) ou l'un de ses sels pharmaceutiquement acceptables, dans un véhicule pharmaceutiquement acceptable.

On entend dans la présente description, par « véhicule pharmaceutiquement acceptable », tout véhicule utile pour la préparation d'une composition pharmaceutique et qui est généralement sûr, non toxique et ni biologiquement ni autrement indésirable pour le sujet à traiter, en particulier pour les mammifères et notamment les humains.

Le véhicule de la composition pharmaceutique selon l'invention peut aussi bien être solide que semi-solide ou liquide. Il peut s'agir d'un diluant, d'un adjuvant ou de tout autre véhicule classique en lui-même pour la constitution des compositions pharmaceutiques.

La composition pharmaceutique selon l'invention peut se présenter sous toute forme galénique, notamment sous forme adaptée à une administration par voie parentérale, intranasale, rectale, pulmonaire ou topique. Préférentiellement, elle se présente sous une forme convenant à une administration par voie orale. A titre d'exemples de telles formes galéniques, non limitatives de l'invention, on peut citer les formes de granulés, poudre, comprimés, gélules, pilules, sirop, solution ou suspension buvable, etc.

La composition pharmaceutique selon l'invention peut contenir un ou plusieurs excipients / additifs classiques en eux-mêmes pour la constitution des compositions pharmaceutiques, par exemple choisis parmi les conservateurs, les agents édulcorants, aromatisants, de charge, désintégrants, mouillants, émulsifiants, tensioactifs, dispersants, lubrifiants, stabilisants, tampons, antibactériens, antifongiques, etc., ou l'un quelconque de leurs mélanges; et/ou tout composé permettant une libération rapide, prolongée ou retardée, et/ou ciblée, du principe actif après son administration au sujet.

La composition pharmaceutique selon l'invention peut en outre contenir un ou plusieurs principes actifs autres que le composé de formule générale (I) ou un de ses sels pharmaceutiquement acceptables, ces principes actifs pouvant ou non agir de manière synergique avec ledit composé.

La composition pharmaceutique selon l'invention est de préférence formulée sous forme de doses unitaires.

L'invention concerne également l'utilisation thérapeutique d'une composition pharmaceutique selon l'invention, telle que définie ci-avant, pour le traitement d'une maladie, en particulier de la maladie de Pompe. Cette utilisation peut répondre à l'une ou plusieurs des caractéristiques décrites ci-avant en référence à l'utilisation thérapeutique du composé de formule générale (I) ou d'un de ses sels pharmaceutiquement acceptables.

Selon un autre aspect, la présente invention concerne un composé répondant à la formule générale (I') ci-après, cette formule générale (I') définissant une sous-famille des composés de formule générale (I) décrits ci-dessus, ou l'un de ses sels pharmaceutiquement acceptables :

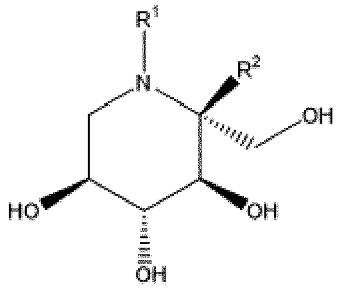

(I')

dans laquelle :

- $R^1$ représente un atome d'hydrogène ou un radical hydrocarboné linéaire, ramifié et/ou cyclique, saturé ou insaturé, aromatique ou non, éventuellement substitué, pouvant comporter un ou plusieurs hétéroatomes et/ou un ou plusieurs groupements comportant au moins un hétéroatome et pouvant comporter un seul cycle ou plusieurs cycles le cas échéant fusionnés,

  et $R^2$ représente un groupement $-CH(R^3)-R^4$, où $R^3$ et $R^4$, pouvant être identiques ou différents, représentent chacun un atome d'hydrogène ou un radical hydrocarboné linéaire, ramifié et/ou cyclique, saturé ou insaturé, aromatique ou non, éventuellement substitué, pouvant comporter un ou plusieurs hétéroatomes et/ou un ou plusieurs groupements comportant au moins un hétéroatome et pouvant comporter un seul cycle ou plusieurs cycles le cas échéant fusionnés, $R^3$ et $R^4$ étant tels que, lorsque $R^1$ et $R^3$ représentent chacun un atome d'hydrogène, $R^4$ ne représente pas un atome d'hydrogène ou un radical phényle,
  et $R^1$ et $R^2$ étant tels qu'ils ne représentent pas simultanément, respectivement, un radical propyle et un radical éthyle,

- ou $R^1$ et $R^2$ forment ensemble, avec les atomes du cycle pipéridine auquel chacun est rattaché, un hétérocycle à 4 chainons fusionné avec le cycle pipéridine, éventuellement substitué par un ou plusieurs radicaux, pouvant être identiques ou différents, choisis chacun parmi un groupement hydroxyle, un groupement amino ou un radical carboné, comportant de préférence de 1 à 18, préférentiellement de 1 à 12 et notamment de 1 à 6, atomes de carbone, linéaire, ramifié et/ou cyclique, saturé ou insaturé, aromatique ou non, éventuellement substitué, pouvant comporter un ou plusieurs hétéroatomes et/ou un ou plusieurs groupements comportant au moins un hétéroatome et pouvant comporter un seul cycle ou plusieurs cycles, le cas échéant fusionnés,
- ou $R^1$ et $R^2$ forment ensemble, avec les atomes du cycle pipéridine auquel chacun est rattaché, un hétérocycle à 3 chainons fusionné avec le cycle pipéridine, éventuellement substitué par un groupement $-X-R^5$, dans lequel :

  - X représente un radical -C(=O)- ou $-CH(OR^6)-$ où $R^6$ représente un radical hydrocarboné linéaire, ramifié et/ou cyclique, saturé ou insaturé, aromatique ou non, éventuellement substitué, pouvant comporter un ou plusieurs hétéroatomes et/ou un ou plusieurs groupements comportant au moins un hétéroatome et pouvant comporter un seul cycle ou plusieurs cycles le cas échéant fusionnés, et $R^5$ représente un atome d'hydrogène, un groupement amino ou un radical hydrocarboné linéaire, ramifié et/ou cyclique, saturé ou insaturé, aromatique ou non, éventuellement substitué, pouvant comporter un ou plusieurs hétéroatomes et/ou un ou plusieurs groupements comportant au moins un hétéroatome et pouvant comporter un seul cycle ou plusieurs cycles le cas échéant fusionnés ;

  - ou X représente un radical -CH(OH)- et $R^5$ représente un atome d'hydrogène ou un radical hydrocarboné, pouvant par exemple comporter de 1 à 6 atomes de carbone, linéaire, ramifié et/ou cyclique, saturé ou insaturé, aromatique ou non, éventuellement substitué, pouvant comporter un ou plusieurs hétéroatomes et/ou un ou plusieurs groupements comportant au moins un hétéroatome et pouvant comporter un seul cycle ou plusieurs cycles, le cas échéant fusionnés, $R^5$ ne représentant pas un radical n-propyle, un radical c-hexyle ou un radical phényle. On entend ici, par radical n-propyle, radical c-hexyle et radical phényle, ces radicaux sous forme non substituée. Ainsi, $R^5$ peut représenter un radical n-propyle substitué, un radical c-hexyle substitué ou un radical phényle substitué. Dans des modes de réalisation particuliers de l'invention, le composé est tel que, dans la formule (I') :

    $R^1$ représente un atome d'hydrogène ou un radical hydrocarboné linéaire, ramifié et/ou cyclique, saturé ou

insaturé, aromatique ou non, éventuellement substitué, pouvant comporter un ou plusieurs hétéroatomes et/ou un ou plusieurs groupements comportant au moins un hétéroatome et pouvant comporter un seul cycle ou plusieurs cycles le cas échéant fusionnés,

et $R^2$ représente un groupement $-CH(R^3)-R^4$, où $R^3$ est tel que défini ci-dessus et $R^4$ représente un radical hydrocarboné, comportant de préférence de 1 à 18, préférentiellement de 1 à 12 et notamment de 1 à 6, atomes de carbone, linéaire, ramifié et/ou cyclique, saturé ou insaturé, aromatique ou non, éventuellement substitué, pouvant comporter un seul cycle ou plusieurs cycles, le cas échéant fusionnés, et comportant un ou plusieurs hétéroatomes choisis chacun parmi l'oxygène, l'azote, le soufre et le silicium et/ou un ou plusieurs groupements comportant au moins un hétéroatome choisis chacun parmi les groupements carbonyle, sulfoxyde, sulfonyle et silane ; $R^4$ ne représentant pas un radical phényle lorsque $R^1$ et $R^3$ représentent chacun un atome d'hydrogène.

En particulier, dans la formule (I'), lorsque $R^1$ représente un atome d'hydrogène ou un radical hydrocarboné linéaire, ramifié et/ou cyclique, saturé ou insaturé, aromatique ou non, éventuellement substitué, pouvant comporter un ou plusieurs hétéroatomes et/ou un ou plusieurs groupements comportant au moins un hétéroatome et pouvant comporter un seul cycle ou plusieurs cycles le cas échéant fusionné, $R^2$ peut représenter un groupement $-(CHR^7)-SO_2-Ar^1$ où $Ar^1$ représente un radical aryle ou hétéroaryle, le cas échéant substitué, comportant de préférence de 5 à 18 atomes, et $R^7$ représente un atome d'hydrogène ou un radical hydrocarboné, comportant de préférence de 1 à 18, préférentiellement de 1 à 12 et notamment de 1 à 6, atomes de carbone, linéaire, ramifié et/ou cyclique, saturé ou insaturé, aromatique ou non, éventuellement substitué, pouvant comporter un ou plusieurs hétéroatomes et/ou un ou plusieurs groupements comportant au moins un hétéroatome et pouvant comporter un seul cycle ou plusieurs cycles, le cas échéant fusionnés.

Le composé selon l'invention peut par exemple répondre à la formule générale (I'd) :

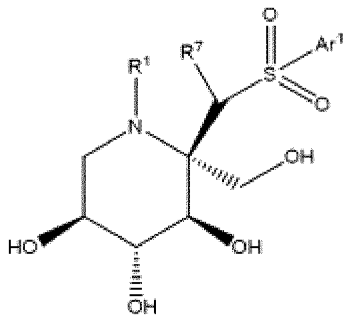

(I'd)

dans laquelle $R^1$, $R^7$ et $Ar^1$ sont tels que définis ci-avant.

Autrement, dans la formule (I'), lorsque $R^1$ représente un atome d'hydrogène ou un radical hydrocarboné linéaire, ramifié et/ou cyclique, saturé ou insaturé, aromatique ou non, éventuellement substitué, pouvant comporter un ou plusieurs hétéroatomes et/ou un ou plusieurs groupements comportant au moins un hétéroatome et pouvant comporter un seul cycle ou plusieurs cycles le cas échéant fusionné, $R^2$ peut représenter un groupement triazole, le cas échéant substitué.

Le composé selon l'invention peut notamment répondre à la formule (I'e) :

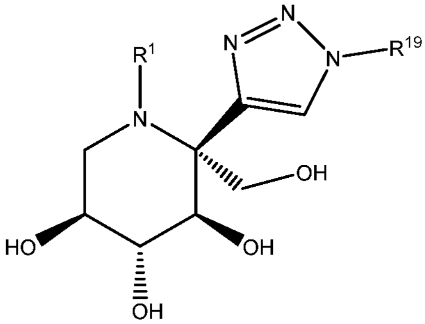

(I'e)

dans laquelle $R^1$ est tel que défini ci-avant et $R^{19}$ représente un atome d'hydrogène ou un radical alkyle, alkylaryle, trialkylsilyle ou aryle en C1-C18, ledit radical étant éventuellement interrompu et/ou substitué par un ou plusieurs hétéroatomes et/ou un ou plusieurs groupements comportant au moins un hétéroatome.

Dans des variantes de l'invention, dans la formule générale (I'), $R^1$ représentant un atome d'hydrogène ou un radical hydrocarboné linéaire, ramifié et/ou cyclique, saturé ou insaturé, aromatique ou non, éventuellement substitué, pouvant comporter un ou plusieurs hétéroatomes et/ou un ou plusieurs groupements comportant au moins un hétéroatome et pouvant comporter un seul cycle ou plusieurs cycles le cas échéant fusionnés, $R^2$ représente un groupement $-(CH_2)_2-R^8$, où $R_8$ représente :

- un atome d'hydrogène ;
- un groupement alkyle ou cycloalkyle en C1-C12, éventuellement substitué, pouvant comporter un seul cycle ou plusieurs cycles fusionnés, tel qu'un radical adamantyle ;
- un groupement $-(CH_2)_a-OH$ où a est en nombre entier compris entre 0 et 18, préférentiellement entre 0 et 12 et notamment entre 0 et 6 ; optionnellement, a peut être différent de 1 lorsque $R^1$ représente un atome d'hydrogène ;
- un groupement $-(CH_2)_b-Ar^2$ où $Ar^2$ représente un radical aryle ou hétéroaryle éventuellement substitué, pouvant comporter un ou plusieurs hétéroatomes et/ou un ou plusieurs groupements comportant au moins un hétéroatome et pouvant comporter un seul cycle ou plusieurs cycles, le cas échéant fusionnés et b est en nombre entier compris entre 0 et 18, préférentiellement entre 0 et 12 et notamment entre 0 et 6 ; optionnellement, b peut être différent de 1 lorsque $R^1$ représente un atome d'hydrogène et $Ar^2$ représente un radical phényle ;
- un groupement $-(CH_2)_c-Si(R^9)_3$ où $R^9$ représente un radical hydroxyle, un radical alkyle en C1-C4, un radical alcoxyle en C1-C4 ou un radical phényle et c est un nombre entier compris entre 0 et 18, préférentiellement entre 0 et 12 et notamment entre 0 et 6 ;
- ou un groupement $-(CH_2)_d-Z-R^{10}$ où Z est un hétéroatome choisi parmi l'oxygène, l'azote et le soufre, $R^{10}$ représente un radical alkyle, cycloalkyle, alkylaryle, aryle ou acyle, en C1-C18, de préférence en C1-C12 et notamment en C1-C6, ledit radical étant éventuellement interrompu et/ou substitué par un ou plusieurs hétéroatomes et/ou un ou plusieurs groupements comportant au moins un hétéroatome, tel qu'un groupement sulfonyle, et d est en nombre entier compris entre 0 et 18, préférentiellement entre 0 et 12 et notamment entre 0 et 6.

Dans des modes de réalisation particulièrement préférés de l'invention, $R^1$ représente un groupe alkyle linéaire, ramifié et/ou cyclique en C1-C18, de préférence en C1-C6, éventuellement interrompu et/ou substitué par un ou plusieurs hétéroatomes et/ou un ou plusieurs groupements comportant au moins un hétéroatome.

Le composé selon l'invention peut par exemple répondre à la formule générale (I'a) :

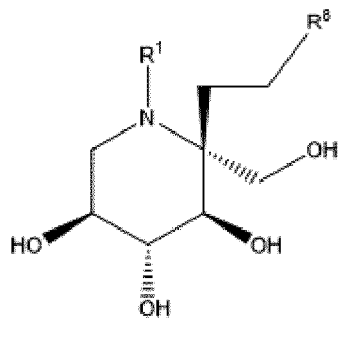

(I'a)

dans laquelle :

$R^1$ représente un atome d'hydrogène ou un groupe alkyle linéaire, ramifié et/ou cyclique en C1-C18, de préférence en C1-C12, notamment en C1-C6, par exemple en C1-C3 et plus particulièrement en C1-C2, ledit groupe alkyle étant éventuellement interrompu et/ou substitué par un ou plusieurs hétéroatomes et/ou un ou plusieurs groupements comportant au moins un hétéroatome, $R^8$ représente :

- un atome d'hydrogène ;
- un groupement hydroxyle ;
- un radical méthyle, éthyle, propyle, butyle, pentyle, hexyle, cycloalkyle, adamantyle, alkylcycloalkyle, alkylaryle ou aryle, ledit radical étant éventuellement interrompu et/ou substitué par un ou plusieurs hétéroatomes et/ou un ou plusieurs groupements comportant au moins un hétéroatome ;
- un groupement $-Si(R^{12})_3$ où $R^{12}$ représente un radical hydroxyle, un radical alkyle en C1-C4, un radical alcoxyle en C1-C4 ou un radical phényle ;
- un groupement $-(CH_2)_f-Y-R^{13}$ où f est un nombre entier compris entre 0 et 6, Y est un hétéroatome choisi parmi

l'oxygène, l'azote et le soufre et $R^{13}$ représente un radical alkyle en C1-C18, de préférence en C1-C12 et notamment en C1-C6, ou un radical aryle ou hétéroaryle, de préférence en C1-C18, de préférence en C1-C12 et notamment en C1-C6 ;

- un groupement $-(CH_2)_g-CO-R^{13}$ où g est un nombre entier compris entre 0 et 6 et $R^{13}$ est tel que défini ci-avant ;
- ou un groupement $-(CH_2)_h-SO_e-R^{13}$ où h est un nombre entier compris entre 0 et 6 et e est égal à 1 ou 2, et $R^{13}$ est tel que défini ci-avant ;

$R^8$ ne représentant pas un atome d'hydrogène lorsque $R^1$ représente un radical propyle.

Le composé selon l'invention peut répondre à la formule générale (I"a) :

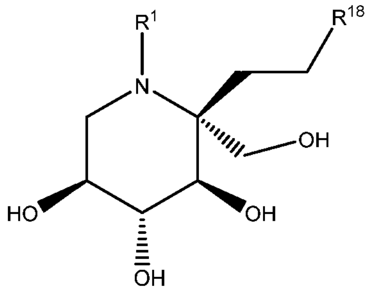

(I"a)

dans laquelle:

$R^1$ représente un atome d'hydrogène ou un groupe alkyle linéaire, ramifié et/ou cyclique en C1-C18, éventuellement interrompu et/ou substitué par un ou
plusieurs hétéroatomes et/ou un ou plusieurs groupements comportant au moins un hétéroatome,
$R^{18}$ représente un radical hydrocarboné linéaire, ramifié et/ou cyclique, saturé ou insaturé, aromatique ou non, éventuellement substitué, comportant de 4 à 18 atomes de carbone, de préférence de 4 à 12 atomes de carbone, et préférentiellement de 5 à 12 atomes de carbone, pouvant comporter un ou plusieurs hétéroatomes et/ou un ou plusieurs groupements comportant au moins un hétéroatome et pouvant comporter un seul cycle ou plusieurs cycles le cas échéant fusionnés.
$R^{18}$ peut notamment représenter un groupement butyle, pentyle, hexyle, cyclohexyle, phényle, benzyle, adamantyle,

Des composés particuliers selon l'invention sont les composés de formules (IIa), (IIb), (IIb1), (IIb2), (IIb3), (IIb4), (IIb5), (IIb6), (IIb7), (IIc), (IIc1), (IId), (IIo), (IIp), (IIp1), (IIq), (IIr), (IIs), (IIt), (IIu), (IIv), (IIw), (IIw1), (IIx), (IIx1) et (IIy) décrits ci-avant.

Dans des modes de réalisation alternatifs de l'invention, le composé répond à la formule générale (I'b) :

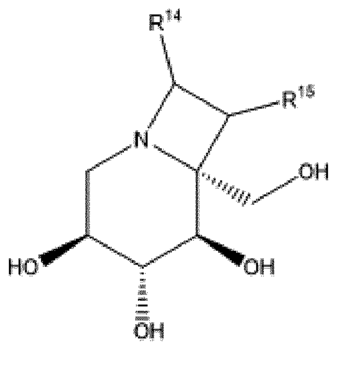

(I'b)

dans laquelle

$R^{14}$ représente un atome d'hydrogène, un radical carbonyle ou un radical alkyle, alcényle, alcynyle, alkylaryle, tel qu'un radical benzyle ou aryle en C1-C18, de préférence en C1-C12 et notamment en C1-C6, ledit radical étant éventuellement interrompu et/ou substitué par un ou plusieurs hétéroatomes et/ou un ou plusieurs groupements comportant au moins un hétéroatome ; un exemple d'un tel radical répondant à la formule $-CH_2-TMS$, où TMS

représente un reste triméthylsilyle ;

et $R^{15}$ représente un atome d'hydrogène, un radical hydroxyle, un radical amino ou un radical alkyle, alcényle ou aryle en C1-C18, de préférence en C1-C12 et notamment en C1-C6, ledit radical étant éventuellement interrompu et/ou substitué par un ou plusieurs hétéroatomes et/ou un ou plusieurs groupements comportant au moins un hétéroatome.

Des composés particuliers répondant à cette définition sont les composés de formules (IIh), (IIz1), (IIz2), (IIz3), (IIz4) et (IIz5) décrits ci-avant.

Dans d'autres variantes de l'invention, le composé répond à la formule générale (I'c) :

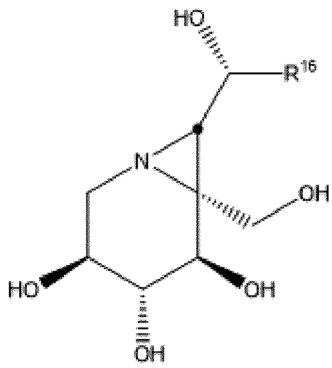

(I'c)

dans laquelle $R^{16}$ représente un atome d'hydrogène ou un radical alkyle en C1-C18, de préférence en C1-C12 et préférentiellement en C1-C6, ledit radical étant éventuellement interrompu et/ou substitué par un ou plusieurs hétéroatomes et/ou un ou plusieurs groupements comportant au moins un hétéroatome.

En particulier, $R^{16}$ peut représenter un groupement $-CH_2-OH$, un radical méthyle ou un radical éthyle.

Des composés particuliers répondant à cette définition sont les composés de formules (IIj) et (IIk) décrits ci-avant.

L'ensemble des composés selon l'invention, répondant à la formule générale (I'), peuvent avantageusement être utilisés en tant que médicaments conformément à la présente invention, en particulier pour le traitement de la maladie de Pompe. Les composés de formule générale (I) utilisés selon l'invention, en particulier les composés répondant à la formule générale (I') tels que définis ci-dessus, peuvent être synthétisés selon toute méthode classique en elle-même pour l'homme du métier. Il entre notamment dans les compétences de l'homme du métier de déterminer, pour chaque composé particulier visé, quels produits de départ utiliser et quelle voie de synthèse appliquer.

La présente invention concerne en outre de nouvelles voies de synthèse, qui ont été développées par les inventeurs pour préparer des composés de formule générale (I), et notamment de formule générale (I').

Certains des procédés de préparation selon l'invention permettent d'obtenir des composés de formule générale (I') dans lesquels $R^1$ et $R^2$ sont indépendants l'un de l'autre, en particulier répondant à la formule générale (I'a) ou à la formule générale (I"a) ci-avant.

Un exemple d'un tel procédé de préparation particulier selon l'invention comprend des étapes successives de :

a/ réaction d'un composé de formule générale (III) ou (IV) :

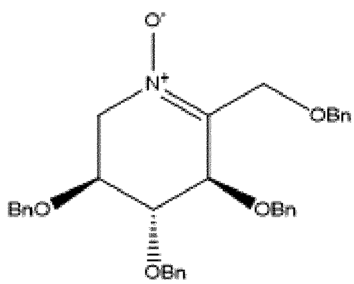

(III)

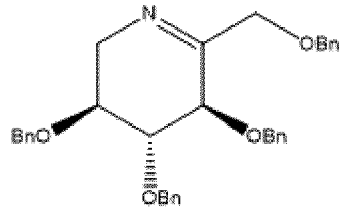

(IV)

dans lesquelles Bn représente un reste benzyle, avec un composé de formule générale (V) :

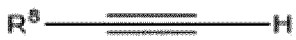

(V)

dans laquelle $R^8$ est tel que défini ci-avant en référence au composé de formule générale (I') selon l'invention, $R^8$ ne représentant toutefois ni un atome d'hydrogène, ni un groupement hydroxyle -OH, ni un groupement amino $-NH_2$, en présence d'un composé organométallique,
b/ le cas échéant, réduction de la fonction hydroxylamine en amine,
c/ le cas échéant, alkylation de l'atome d'azote du cycle pipéridine,
d/ et hydrogénolyse du produit obtenu à l'issue de l'étape a/, le cas échéant de l'étape b/ ou de l'étape c/, notamment pour réaliser le clivage des restes benzyle pour former des groupements hydroxyles, et le cas échéant la transformation de la fonction hydroxylamine en amine et l'hydrogénation de la triple liaison.

Le composé organométallique mis en oeuvre pour l'étape a/ peut être un composé organozincique, organolithien, organomagnésien, organoalane, organocuivreux, etc., ou l'un quelconque de leurs mélanges. Préférentiellement, dans l'étape a/ la réaction est réalisée en présence de dialkylzinc, notamment de diéthylzinc $Et_2Zn$, ou de butyllithium.
Plus généralement, le composé organométallique peut répondre à la formule générale (VII) :

$R^{17}$-M (VII)

dans laquelle

$R^{17}$ représente un radical alkyle, alcényle, alcynyle, alkylaryle ou aryle en C1-C18, ledit radical étant éventuellement interrompu et/ou substitué par un ou plusieurs hétéroatomes et/ou un ou plusieurs groupements comportant au moins un hétéroatome,
et M représente un élément métallique tel que le lithium, le zinc, le cuivre, l'aluminium ou le magnésium, ou M représente Mg-$X^2$, où $X^2$ représente un atome d'halogène.

L'étape b/ de réduction de la fonction hydroxylamine en amine et l'étape c/ de N-alkylation peuvent être réalisées selon toute méthode classique en elle-même pour l'homme du métier.
L'étape d/ d'hydrogénolyse peut également être réalisée selon toute méthode classique en elle-même pour l'homme du métier, notamment par hydrogénation catalytique.
De manière tout à fait surprenante et avantageuse, un tel procédé permet de préparer des composés de formule générale (I'), en particulier de formule générale (I'a) ou de formule générale (I"a), de manière totalement stéréosélective.
Des exemples de tels procédés peuvent être représentés par les voies de synthèse 1 montrées sur la figure 1, pour l'exemple particulier d'un composé répondant à la formule générale (I'a).
Des composés de formule générale (I'a) ou (I"a) peuvent autrement, par exemple, être préparés par un procédé comprenant des étapes successives de :

a/ réaction de cycloaddition d'un composé de formule générale (III) avec le triméthylsilylacétylène,
b/ réaction du cycloadduit obtenu avec un fluorure tel que le fluorure de tétrabutylammonium, par exemple selon un protocole similaire à celui décrit dans la publication de Ahn, et al., 1994, J. Org. Chem. 59: 6282-6286,
c/ réduction par un hydrure métallique tel que l'hydrure de bore et de sodium ($NaBH_4$), l'hydrure de lithium et d'aluminium ($LiAlH_4$) ou l'hydrure de diisobutylaluminium (DIBAL-H), ou par un borane tel qu'un complexe borane-sulfure de diméthyle ($BH_3$•$Me_2S$), un complexe borane-tétrahydrofurane ($BH_3$•THF), un complexe borane -pyridine ($BH_3$•pyridine), le diborane $B_2H_6$, l'hydrure de lithium et d'aluminium ($LiAlH_4$) étant particulièrement préféré,
d/ le cas échéant, une étape de *O*-alkylation, *O*-acylation ou de *O*-sulfonylation, cette étape pouvant être réalisée

selon toute méthode classique en elle-même pour l'homme du métier,

e/ le cas échéant, une étape de *N*-alkylation, pouvant être réalisée selon toute méthode classique en elle-même pour l'homme du métier,

f/ et hydrogénolyse du produit obtenu à l'issue de l'étape c/, le cas échéant de l'étape d/ ou de l'étape e/, notamment pour réaliser le clivage des restes benzyle pour former des groupements hydroxyles.

Ce procédé de synthèse est particulièrement avantageux en ce que :

- l'étape a/ est simple à réaliser et totalement régio- et diastéréosélective en faveur d'un produit de configuration D-gluco ;
- le cycloadduit formé dans l'étape a/ est aisément et efficacement transformé en β-lactame, lui-même aisément et efficacement réduit en pipéridine-alcool (le β-lactame obtenu peut autrement être transformé en cycle à 4 chaînons saturé, par réduction par un silane ou par alkylation réductrice en présence de catalyseurs métalliques).

Un exemple d'un tel procédé peut être représenté par la voie de synthèse 2 montrée sur la figure 2 pour l'exemple particulier d'un composé répondant à la formule générale (I'a).

Des composés de formule (I'd) ci-avant peuvent être préparés par un procédé comprenant la réaction d'un composé de formule (III) ou (IV) ci-avant avec un composé de formule générale (VI) :

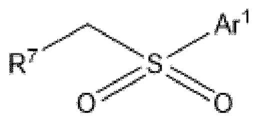
(VI)

en présence d'une base lithiée telle que le diisopropylamidure de lithium (LDA) ou le bis(triméthylsilyl)amidure de lithium (LiHMDS), de préférence en présence de LiHMDS à basse température, de préférence à -78 °C ; puis, après le cas échéant une étape de réduction de la fonction hydroxylamine en amine, et le cas échéant une étape d'alkylation de l'atome d'azote du cycle pipéridine, hydrogénolyse du produit obtenu.

Un exemple d'un tel procédé peut être représenté par la voie de synthèse 3 montrée sur la figure 3, pour l'exemple particulier d'un composé répondant à la formule générale (I'd).

Des composés de formule générale (I), et notamment de formule générale (I'), peuvent autrement, par exemple, être préparés, à partir de composés de formule (III) ou (IV), selon un procédé comprenant les étapes successives de :

- a/ réaction avec un composé de formule générale (VII), le cas échéant en présence d'un acide de Lewis :

$R^{17}$-M (VII)

dans laquelle

$R^{17}$ représente un radical alkyle, alcényle, alcynyle, alkylaryle ou aryle en C1-C18, ledit radical étant éventuellement interrompu et/ou substitué par un ou plusieurs hétéroatomes et/ou un ou plusieurs groupements comportant au moins un hétéroatome,

et M représente un élément métallique tel que le lithium, le zinc, le cuivre, l'aluminium ou le magnésium, ou M représente Mg-$X^2$, où $X^2$ représente un atome d'halogène ;

- b/ puis, le cas échéant, réduction de la fonction hydroxylamine en amine, selon toute méthode classique en elle-même pour l'homme du métier, notamment par traitement par de la poudre de Zinc en milieu acide,
- c/ puis, le cas échéant, alkylation de l'atome d'azote du cycle pipéridine, selon toute méthode classique en elle-même pour l'homme du métier,
- d/ puis, le cas échéant, une étape de métathèse cyclisante d'oléfines catalysée par un complexe à base de ruthénium, préférentiellement le catalyseur de Grubbs de 2$^{ème}$ génération,
- e/ et enfin, hydrogénolyse du produit obtenu à l'issue de l'étape a/, le cas échéant à l'issue de l'étape b/, de l'étape c/, ou de l'étape d/, selon toute méthode classique en elle-même pour l'homme du métier, en particulier en présence d'hydrogène et d'une quantité catalytique de palladium sur charbon, en milieu acide.

La voie de synthèse mise en oeuvre pour obtenir des composés répondant à la formule générale (I) relève de la voie de synthèse générale 4 montrée sur la figure 4.

D'autres procédés de préparation selon l'invention permettent d'obtenir des composés de formule générale (I') dans lesquels $R^1$ et $R^2$ forment ensemble, avec les atomes du cycle pipéridine auquel chacun est rattaché, un hétérocycle à 4 chainons fusionné avec le cycle pipéridine.

Des exemples de tels procédés comprennent des étapes successives de :

- a/ réaction de cycloaddition d'un composé de formule générale (III) avec le triméthylsilylacétylène, suivie d'une réaction avec un fluorure tel que le fluorure de tétrabutylammonium, par exemple selon un protocole similaire à celui préalablement décrit dans la publication de Ahn, et al., 1994, J. Org. Chem. 59: 6282-6286 ; puis
- soit b/ réduction du carbonyle β-lactamique par un silane tel que le phénylsilane $PhSiH_3$, en présence de catalyseur à base de rhodium tel que $[Rh(COD)_2]BF_4$ et de 1,3-bis (diphénylphosphino)propane, selon un protocole similaire à celui préalablement décrit dans la publication de Bornschein et al., 2015, Eur. J. Org. Chem, 1915-1919 ;

  soit b'/ alkylation réductrice du carbonyle β-lactamique par réaction avec le tétraméthyldisiloxane en présence de catalyseur de Vaska à température ambiante, puis addition d'un composé de formule (VII) tel que défini ci-dessus, à -78 °C, selon un protocole similaire à celui décrit dans la publication de Xie et Dixon, 2017, Chem. Sci. 8: 7492-7497 ;
  et enfin

- c/ hydrogénolyse du produit obtenu à l'issue de l'étape b/ ou b'/, notamment pour réaliser le clivage des restes benzyle pour former des groupements hydroxyles.

L'étape c/ d'hydrogénolyse peut être réalisée selon toute méthode classique en elle-même pour l'homme du métier, notamment par hydrogénation catalytique. Les étapes b/ et b'/ sont particulièrement avantageuses, et inédites, en ce qu'elles permettent de réaliser la réduction ou l'alkylation réductrice d'un β-lactame dont l'atome d'azote est inclus dans un cycle pipéridine, pour former un hétérocycle de type conidine.

Des exemples de tels procédés peuvent être représentés par les voies de synthèse 5 montrées sur la figure 5, pour l'exemple particulier d'un composé répondant à la formule générale (I'b).

Un autre exemple d'un tel procédé comprend des étapes successives de : a/ réaction de Kinugasa, telle que décrite dans les revues de Stecko et al., 2014, Tetrahedron 70: 7817-7844 ou de Khangarot & Kaliappan, 2013, Eur. J. Org. Chem. 7664-7677, d'un composé de formule générale (III) avec un composé de formule générale (VIII) :

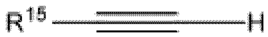
(VIII)

dans laquelle $R^{15}$ représente un radical alkyle, alcényle ou aryle en C1-C18, éventuellement interrompu et/ou substitué par un ou plusieurs hétéroatomes et/ou un ou plusieurs groupements comportant au moins un hétéroatome, en présence de sel de Cu(I) et d'une amine ; puis

b/ alkylation réductrice du carbonyle β-lactamique par réaction avec le tétraméthyldisiloxane en présence de catalyseur de Vaska à température ambiante, puis addition d'un composé de formule (VII) tel que défini ci-dessus, à -78 °C, selon un protocole similaire à celui décrit dans la publication de Xie et Dixon, 2017, Chem. Sci. 8: 7492-7497 ; et enfin

c/ hydrogénolyse du produit obtenu à l'issue de l'étape b/, notamment pour réaliser le clivage des restes benzyle pour former des groupements hydroxyles. L'étape c/ d'hydrogénolyse peut être réalisée selon toute méthode classique en elle-même pour l'homme du métier, notamment par hydrogénation catalytique. L'étape b/ est particulièrement avantageuse, et inédite, en ce qu'elle permet de réaliser l'alkylation réductrice d'un β-lactame dont l'atome d'azote est inclus dans un cycle pipéridine, pour former un hétérocycle de type conidine.

Un exemple d'un tel procédé peut être représenté par la voie de synthèse 6 montrée sur la figure 6, pour l'exemple particulier d'un composé répondant à la formule générale (I'b).

D'autres procédés de préparation selon l'invention permettent d'obtenir des composés de formule générale (I') dans lesquels $R^1$ et $R^2$ forment ensemble, avec les atomes du cycle pipéridine auquel chacun est rattaché, un hétérocycle à 3 chaînons fusionné avec le cycle pipéridine.

Un exemple de tel procédé comprend des étapes successives de :

a/ réaction de cycloaddition d'un composé de formule générale (III) ou d'un composé de formule générale (III') :

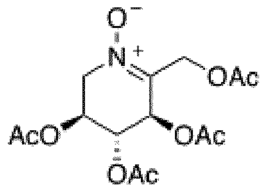

(III')

dans laquelle Ac représente un reste acétyle,

avec un composé de formule générale (V) tel que décrit ci-dessus, dans laquelle $R^8$ est tel que défini ci-avant en référence au composé de formule générale (I') selon l'invention, $R^8$ ne représentant toutefois ni un atome d'hydrogène, ni un groupement hydroxyle -OH, ni un radical amino $-NH_2$,

- b/ réarrangement thermique de Baldwin, selon un protocole similaire à celui décrit dans la publication de Tangara et al., 2017, Org. Lett. 19: 4842-4845, permettant de former une acylaziridine, cette étape étant préférentiellement réalisée à 110 °C sous irradiation par des rayonnements micro-ondes,
- c/ réduction du carbonyle de l'acylaziridine ainsi obtenue par réaction avec un hydrure métallique, préférentiellement $LiAlH_4$ ou $NaBH_4$,
- d/ et débenzylation, selon toute méthode classique en elle-même pour l'homme du métier, notamment par réduction de Birch en présence de métaux dissous dans l'ammoniac liquide à basse température, de préférence à -78 °C, le métal employé étant de préférence le lithium.

Un exemple d'un tel procédé peut être représenté par la voie de synthèse 7 montrée sur la figure 7, pour l'exemple particulier des composés répondant à le formule générale (I'c).

Un exemple de procédé pour préparer des composés de formule générale (I'e) ci-dessus peut être représenté par la voie de synthèse 8 montrée sur la figure 8, avec $R^{20}$ étant identique à $R^{19}$ , $R^{20}$ ne représentant toutefois pas un atome d'hydrogène.

Les caractéristiques et avantages de l'invention apparaîtront plus clairement à la lumière des exemples de mise en oeuvre ci-après, fournis à simple titre illustratif et nullement limitatifs de l'invention, avec l'appui des figures 1 à 8, dans lesquelles :

La figure 1 montre un premier exemple de voies de synthèse générales (voies de synthèse 1) pour la préparation de composés de formule (I'a) selon l'invention.

La figure 2 montre un deuxième exemple de voie de synthèse générale (voie de synthèse 2) pour la préparation de composés de formule (I'a) selon l'invention.

La figure 3 montre un exemple de voie de synthèse générale (voie de synthèse 3) pour la préparation de composés de formule (I'd) selon l'invention.

La figure 4 montre un troisième exemple de voies de synthèse générales (voies de synthèse 4) pour la préparation de composés de formule (I'a) et, de façon plus générale, de composés de formule générale (I) utilisés selon l'invention.

La figure 5 montre de premiers exemples de voies de synthèse générales (voies de synthèse 5) pour la préparation de composés de formule (I'b) selon l'invention.

La figure 6 montre un deuxième exemple de voies de synthèse générales (voies de synthèse 6) pour la préparation de composés de formule (I'b) selon l'invention.

La figure 7 montre un exemple de voies de synthèse générales (voies de synthèse 7) pour la préparation de composés de formule (I'c) selon l'invention.

La figure 8 montre un exemple de voie de synthèse générale (voie de synthèse 8) pour la préparation de composés de formule (I'e) selon l'invention.

**A/ Synthèse et caractérisation de composés utilisés conformément à l'invention**

Toutes les réactions ont été conduites sous atmosphère inerte dans de la verrerie préalablement séchée, sous agitation par un barreau magnétique, le montage étant placé au-dessus d'un agitateur magnétique, sauf dans le cas des réactions sous irradiation par des micro-ondes. Ces dernières ont été réalisées dans des tubes scellés équipés d'un barreau magnétique placés dans un réacteur à micro-ondes, à une température régulée par une sonde infra-rouge interne. Le toluène, l'éther et le dichlorométhane utilisés comme solvants de réactions ont été préalablement filtrés sur un système de purification Inert PureSolv®. L'acétonitrile et le dichloroéthane ont été distillés sur $CaH_2$. Le THF a été distillé sur sodium en présence de benzophénone. Les réactifs commerciaux, le méthanol et l'éthanol ont été utilisés sans purification. Les réactions ont été suivies par chromatographie sur couche mince (CCM) au moyen de plaques de silice sur aluminium (Merck, Kieselgel 60 F254), avec révélation sous rayons ultraviolets puis par une solution à 3% de

permanganate de potassium dans une solution à 10% d'hydroxyde de potassium (w/v). Les purifications par chromatographie sur colonne ont été réalisées au moyen de silice Macherey-Nagel® Silica Gel 60 (70-230 mesh). Les pouvoirs rotatoires ont été mesurés sur un polarimètre PerkinElmer 341. Les spectres infrarouges ont été obtenus avec un spectromètre Nicolet "Magna 550" comportant un module ATR (attenuated total reflexion) sur lequel les produits ont été déposés purs, à l'état solide ou liquide. Les données sont exprimées en $cm^{-1}$. Les spectres RMN $^1H$ et RMN $^{13}C$ {DEPT-Q} ont été obtenus avec des spectromètres Avance 500 ($^1H$ : 500 MHz, $^{13}C$ : 125 MHz) ou Avance 400 ($^1H$ : 400 MHz, $^{13}C$ : 100 MHz). Les déplacements chimiques pour les spectres $^1H$ sont exprimés par rapport à ceux des solvants résiduels contenus dans $CDCl_3$ ($\delta$ 7,26 ppm) ou $CD_3OD$ ($\delta$ 3,31 ppm). Les déplacements chimiques pour les spectres $^{13}C$ sont exprimés par rapport à ceux des solvants $CDCl_3$ ($\delta$ 77,16 ppm) ou $CD_3OD$ ($\delta$ 49 ,00 ppm). Les spectres RMN $^1H$ sont reportés comme suit : déplacement chimique (ppm), multiplicité (br : broad ; s : singulet ; d : doublet ; dd : doublet de doublets ; t : triplet ; *ps*t : pseudo triplet ; m : multiplet), constantes de couplage (Hz) et intégration. Les spectres de masse haute résolution (HRMS) ont été enregistrés sur un spectromètre de masse ESI/LTQ Orbitrap XL® Thermo Scientific ou Waters G2-S Q-TOF.

A. 1/ Voies de synthèse 1

Les voies de synthèse mises en oeuvre pour obtenir des composés répondant à la formule générale (I'a) ci-dessous relèvent des voies de synthèse générales montrées sur la figure 1.

1) Protocole Général A - alcynylation de la nitrone de formule (III) conduisant à des hydroxylamines propargyliques de formule A-1

A une solution d'alcyne de formule (V) (4 équiv.) dans du toluène anhydre à 0 °C sous atmosphère d'argon, est ajoutée goutte à goutte une solution 1M de diéthylzinc dans l'hexane (1,5 équiv.). Le mélange résultant est agité 30 min à 0 °C. Une solution de cétonitrone de formule (III) (1 équiv.) dans du toluène anhydre est alors ajoutée goutte à goutte à 0 °C, puis le mélange réactionnel est agité jusqu'à complétion de la réaction (suivie par CCM). Une solution aqueuse saturée de NaHCOs est ajoutée puis le mélange résultant est dilué avec du diéthyléther. La phase organique est séparée et la phase aqueuse est extraite deux fois avec du diéthyléther. Les phases organiques sont lavées avec de la saumure, séchées sur $MgSO_4$ et évaporées sous pression réduite. Les hydroxylamines de formule A-1 ainsi formées sont purifiées par chromatographie sur gel de silice, avant d'être réduites et débenzylées selon le protocole général B ci-dessous.

2) Protocole Général A' - alcynylation de la nitrone de formule (III) conduisant à des hydroxylamines propargyliques de formule A-1

A une solution d'alcyne de formule (V) (2,5 équiv.) dans du THF anhydre à - 10 °C sous atmosphère d'argon, est ajoutée goutte à goutte une solution à 1,4 M de n-butyllithium dans l'hexane (2,5 équiv.). La solution résultante est agitée 15 min à -10 °C puis est refroidie à -78 °C. Une solution de cétonitrone de formule (III) (1 équiv.) dans du THF anhydre est alors ajoutée goutte à goutte à -78 °C, puis le mélange réactionnel est agité jusqu'à complétion de la réaction (suivie par CCM). Une solution aqueuse saturée de $NH_4Cl$ est ajoutée et le mélange est laissé remonter à température ambiante, puis extrait trois fois par de l'acétate d'éthyle. Les phases organiques sont rassemblées et lavées avec de la saumure, séchées sur $MgSO_4$ et concentrées sous pression réduite. Les hydroxylamines de formule A-1 ainsi isolées sont purifiées par chromatographie sur gel de silice, avant d'être réduites et débenzylées selon le protocole général B ci-dessous.

3) Protocole Général B

A une solution d'iminosucre O-benzylé (1 équiv.) dans de l'éthanol absolu, sont ajoutés 1,5 équiv. d'acide chlorhydrique HCl (solution 2M dans l'éther anhydre) et 0,2 équiv. de Pd/C 10%. La suspension obtenue est agitée à température ambiante pendant 17 h sous pression d'hydrogène (5 bar) puis filtrée sur Célite®. La Célite® est rincée avec du méthanol et le filtrat est concentré sous pression réduite pour donner un chlorhydrate d'iminosucre. Ce sel est purifié sur résine échangeuse d'ions Dowex® 50W-X2 préalablement activée par HCl, et élué avec du $NH_4OH$ aqueux, pour obtenir après évaporation des solvants le composé neutre correspondant de formule (I'a).

Composé de formule (IIc)

Le composé de formule (IIc) ci-avant ((2*R*,3*R*,4*R*,5*S*)-2-(hydroxyméthyl)-2-phénéthylpipéridine-3,4,5-triol : 36,7 mg ; 73% pour 2 étapes) a été préparé selon les procédures générales A et B, à partir de la nitrone de formule (III)

(110,0 mg, 0,205 mmol) et de phénylacétylène (Va : $R^8$ = Ph, 0,034 mL ; 0,307 mmol).

On obtient un solide jaune, de caractéristiques :

$[\alpha]^{20}_D$ +4,7 (*c* 0,98, $CH_3OH$);
IR ν 3291, 2918, 2864, 1632, 1494, 1455, 1363, 1270, 1198, 1094, 1076, 1040, 1004 $cm^{-1}$;
RMN $^1H$ (500 MHz, $CD_3OD$) δ 7.31-7.08 (m, 5H), 3.73 (d, *J*= 10.4 Hz, 1H), 3.55 (d, *J* = 10.5 Hz, 1H), 3.55-3.43 (m, 2H), 3.45-3.34 (m, 1H), 3.00-2.90 (m, 1H), 2.68-2.51 (m, 3H), 1.91-1.72 (m, 2H) ppm;
RMN $^{13}C$ (125 MHz, $CD_3OD$) δ 144.4 ($^{Ar}Cq$), 129.4 ($^{Ar}CH$), 129.3 ($^{Ar}CH$), 126.7 ($^{Ar}CH$), 76.5 (CH), 75.2 (CH), 73.8 (CH), 65.6 ($CH_2$), 60.5 (Cq), 45.9 ($CH_2$), 30.7 ($CH_2$), 29.9 ($CH_2$) ppm;
HRMS ($ESI^+$) Calc. $C_{14}H_{22}NO_4$ $[M + H]^+$: *m*/*z* = 268,15433 ; Trouv. *m*/*z* = 268,15372.

Composé (IIx1)

Le composé de formule (IIx1) ci-avant ((2*R*,3*R*,4*R*,5*S*)-2-(hydroxymethyl)-2-(2-(trimethylsilyl)ethyl)piperidine-3,4,5-triol : 7,1 mg ; 29 % pour 2 étapes) a été préparé selon les procédures générales A et B, à partir de la nitrone de formule (III) (102,1 mg, 0,189 mmol) et de triméthylsilylacétylène (Vb : $R^8$ = TMS, 0,105 mL ; 0,759 mmol).

On obtient une mousse blanche, de caractéristiques :

$[\alpha]^{20}_D$ +4,51 (*c* 0,71, MeOH);

RMN $^1H$ (400 MHz, $CD_3OD$) δ 3.57 (d, *J* = 10.9 Hz, 1H), 3.52 (d, *J* = 9.3 Hz, 1H), 3.44 (t, *J* = 4.0 Hz, 1H), 3.39 (d, *J* = 10.0 Hz, 1H), 3.39-3.33 (m, 1H), 2.85 (dd, *J* = 12.8, 5.5 Hz, 1H), 2.42 (dd, *J* = 12.8, 10.8 Hz, 1H), 1.62-1.51 (m, 2H), 0.56-0.44 (m, 1H), 0.39-0.29 (m, 1H), 0.02 (s, 9H) ppm;
RMN $^{13}C$ (100 MHz, $CD_3OD$) δ 76.6 (CH), 75.5 (CH), 73.8 (CH), 65.6 ($CH_2$), 60.5 (Cq), 45.8 ($CH_2$), 21.0 ($CH_2$), 8.60 ($CH_2$), -1.86 ($CH_3$) ppm;
HRMS ($ESI^+$) Calc. $C_{11}H_{26}NO_4Si$ $[M + H]^+$: *m*/*z* = 264.16256; Trouv. *m*/*z* = 264.16282.

Composé (IIr)

Le composé de formule (IIr) ci-avant ((2*R*,3*R*,4*R*,5*S*)-2-(2-((3*R*,5*R*,7*R*)-adamantan-1-yl)ethyl)-2-(hydroxymethyl)piperidine-3,4,5-triol : 15,0 mg; 27 % pour 2 étapes) a été préparé selon les procédures générales A' et B, à partir de la nitrone de formule (III) (100 mg, 0,185 mmol) et d'adamantylacétylène (Vh : $R^8$ = adamantyle, 74,5 mg, 0,465 mmol).

On obtient une laque jaunâtre, de caractéristiques :

$[\alpha]^{20}_D$ +7,46 (*c* 0,63 , MeOH);

RMN $^1H$ (500 MHz, $CD_3OD$) δ 3.79 (d, *J* = 10.9 Hz, 1H), 3.64-3.48 (m, 4H), 3.11 (br d, *J*= 11.3 Hz, 1H), 2.78-2.66 (m, 1H), 2.00-1.91 (m, 3H), 1.82-1.63 (m, 8H), 1.58-1.49 (m, 6H), 1.19-1.03 (m, 2H) ppm;
RMN $^{13}C$ NMR (100 MHz, $CD_3OD$) δ 74.9 (CH), 72.5 (CH), 70.8 (CH), 63.7 (Cq), 62.5 ($CH_2$), 44.2 ($CH_2$), 43.3 ($CH_2$), 38.2 ($CH_2$), 37.0 ($CH_2$), 30.1 (CH), 20.7 ($CH_2$) ppm;
HRMS ($ESI^+$) Calc. $C_{18}H_{31}NO_4$ $[M + H]^+$: *m*/*z* = 326.23258; Trouv. *m*/*z* = 326.23225.

Composé (IIb7)

Le composé de formule (IIb7) ci-avant ((2*R*,3*R*,4*R*,5*S*)-2-(2-cyclohexylethyl)-2-(hydroxymethyl)piperidine-3,4,5-triol : 57,3 mg ; 59 % pour 2 étapes) a été préparé selon les procédures générales A et B, à partir de la nitrone de formule (III) (99,8 mg ; 0,185 mmol) et de 1-ethynylcyclohexène (Vd : $R^8$ = cyclohexène ; 87 μL ; 0,742 mmol).

On obtient un solide blanc, de caractéristiques :

$[\alpha]^{20}_D$ +3,63 (*c* 1,43, MeOH);

RMN $^1H$ (400 MHz, $CD_3OD$) δ 3.60 (d, *J* = 10.6 Hz, 1H, $^7CH_2$), 3.52-3.43 (m, 2H, $^3CH$, $^4CH$), 3.43-3.33 (m, 2H), 2.87 (dd, *J* = 12.8, 5.0 Hz), 2.49 (ps t, *J* = 11.7 Hz, 1H), 1.83-1.62 (m, 5H), 1.60-1.47 (m, 2H), 1.34-1.06 (m, 6H), 1.01-0.86 (m, 2H) ppm;

RMN $^{13}C$ (100 MHz, $CD_3OD$) δ 76.5 (CH), 75.1 (CH), 73.8 (CH), 65.5 ($CH_2$), 60.3 (Cq), 45.8 ($CH_2$), 39.9 (CH), 34.6 ($CH_2$), 34.5 ($CH_2$), 30.6 ($CH_2$), 27.8 ($CH_2$), 27.5 ($CH_2$), 24.8 ($CH_2$) ppm;
HRMS (ESI+) Calc. $C_{14}H_{20}NO_4$ $[M + H]^+$: *m*/*z* = 274,20128; Trouv. *m*/*z* = 274,20139.

Composé (IIb1)

Le composé de formule (IIb1) ci-avant ((2*R*,3*R*,4*R*,5*S*)-2-(hydroxymethyl)-2-pentylpiperidine-3,4,5-triol : 32,8 mg ; 67 % pour 2 étapes) a été préparé selon les procédures générales A et B, à partir de la nitrone de formule (III) (96,8 mg ; 0,180 mmol) et de 1-pentyne (Ve : $R^8 = C_3H_7$ ; 71 μL ; 0,720 mmol).

On obtient un solide jaune, de caractéristiques :

$[\alpha]^{20}_D$ -1,29 (*c* 1,01, MeOH);

RMN $^1H$ (400 MHz, $CD_3OD$) δ 3.61 (d, *J* = 10.6 Hz, 1H), 3.52-3.33 (m, 4H), 2.87 (dd, *J* = 12.8, 5.3 Hz, 1H), 2.49 (ps t, *J* = 11.8 Hz, 1H), 1.62-1.43 (m, 2H), 1.43-1.17 (m, 6H), 0.92 (t, *J* = 6.7 Hz, 3H) ppm;
RMN $^{13}C$ (100 MHz, $CD_3OD$) δ 76.5 (CH), 75.2 (CH), 73.9 (CH), 65.7 ($CH_2$), 60.2 (Cq), 45.9 ($CH_2$), 33.9 ($CH_2$), 27.7 ($CH_2$), 23.6 ($CH_2$), 22.9 ($CH_2$), 14.4 ($CH_3$) ppm; HRMS (ESI+) Calc. $C_{11}H_{24}NO_4$ $[M + H]^+$ : *m*/*z* = 234,16998; Trouv. *m*/*z* = 234,17004.

Composé (IIb3)

Le composé de formule (IIb3) ci-avant ((2*R*,3*R*,4*R*,5*S*)-2-heptyl-2-(hydroxymethyl)piperidine-3,4,5-triol : 33,2 mg ; 69 % pour 2 étapes) a été préparé selon les procédures générales A et B, à partir de la nitrone de formule (III) (100,7 mg ; 0,187 mmol) et de 1-heptyne (Vf : $R^8 = C_5H_{11}$ ; 98 μL ; 0,749 mmol).

On obtient un solide jaune, de caractéristiques :

$[\alpha]^{20}_D$ +1,04 (*c* 0,96, MeOH);

RMN $^1H$ (400 MHz, $CD_3OD$) δ 3.61 (d, *J* = 10.6 Hz, 1H), 3.51-3.44 (m, 2H), 3.41 (d, *J* = 10.8 Hz), 3.39-3.33 (m, 1H), 2.87 (dd, *J* = 13.0, 5.4 Hz, 1H), 2.49 (ps t, *J* = 11.4 Hz, 1H), 1.58-1.47 (m, 2H), 1.43-1.18 (m, 10H), 0.90 (t, *J* = 6.2 Hz, 3H) ppm;
RMN $^{13}C$ (100 MHz, $CD_3OD$) δ 76.5 (CH), 75.1 (CH), 73.8 (CH), 65.6 ($CH_2$), 60.3 (Cq), 45.9 ($CH_2$), 33.0 ($CH_2$), 31.7 ($CH_2$), 30.4 ($CH_2$), 23.7 ($CH_2$), 23.2 ($CH_2$), 27.8 ($CH_2$), 14.4 ($CH_3$) ppm;
HRMS (ESI+) Calc. $C_{13}H_{28}NO_4$ $[M + H]^+$ : *m*/*z* = 261,20128; Trouv. *m*/*z* = 261,20131.

Composé (!!c1)

Le compose de formule (IIc1) ci-avant ((2*R*,3*R*,4*R*,5*S*)-2-(hydroxymethyl)-2-(3-phenylpropyl)piperidine-3,4,5-triol : 20,2 mg ; 45 % pour 2 étapes) a été préparé selon les procédures générales A et B, à partir de la nitrone de formule (III) (96,7 mg ; 0,179 mmol) et du 3-phényl-1-propyne (Vg : $R^8 = CH_2Ph$ ; 89 μL ; 0,719 mmol).

On obtient une laque jaune, de caractéristiques :

$[\alpha]^{20}_D$ +1,68 (*c* 1,37, MeOH);

RMN $^1H$ (400 MHz, $CD_3OD$) δ 7.10-7.10 (m, 5H), 3.60 (d, *J* = 10.6 Hz, 1H), 3.49-3.37 (m, 3H), 3.37-3.32 (m, 1H), 2.84 (dd, *J* = 13.1, 5.4 Hz, 1H), 2.69-2.53 (m, 2H), 2.42 (dd, *J* = 13.0, 10.8 Hz, 1H), 1.75-1.49 (m, 4H) ppm;
RMN $^{13}C$ (100 MHz, $CD_3OD$) δ 143.6 ($^{Ar}$Cq), 129.4 ($^{Ar}$CH), 129.3 ($^{Ar}$CH), 126.7 ($^{Ar}$CH), 76.5 (CH), 75.1 (CH), 73.8 (CH), 65.6 ($CH_2$), 60.3 (Cq), 45.8 ($CH_2$), 37.6 ($CH_2$), 27.5 ($CH_2$), 25.3 ($CH_2$) ppm;
HRMS (ESI+) Calc. $C_{15}H_{24}NO_4$ $[M + H]^+$ : *m*/*z* = 282,16998; Trouv. *m*/*z* = 282,16968.

A.2/ Voie de synthèse 2

La voie de synthèse mise en oeuvre pour obtenir des composés répondant à la formule générale (l'a) ci-dessous relève de la voie de synthèse générale 2 montrée sur la figure 2.

Composé de formule (IId)

Le composé de formule (IId) ci-avant (2*R*,3*R*,4*R*,5*S*)-2-(2-hydroxyéthyl)-2-(hydroxyméthyl)pipéridine-3,4,5-triol est préparé de la façon suivante.

Un mélange de nitrone (III) (566 mg, 1,05 mmol) et d'alcyne triméthylsilylacétylène (Vb : $R^8$ = $SiMe_3$, 2,2 mL, 15,75 mmol) a été agité à température ambiante pendant 21 h, puis l'excès d'alcyne a été évaporé sous pression réduite. L'isoxazoline B-2 brute ainsi obtenue (667 mg, 1,05 mmol) a été dissoute dans du THF anhydre (20 mL) et une solution de TBAF (1 M in THF, 1,05 mL, 1,05 mmol) a été ajoutée à 0 °C. La solution a été agitée à 0 °C durant 45 min, puis du $CH_2Cl_2$ et de l'eau ont été ajoutés. La phase aqueuse a été extraite trois fois avec du $CH_2Cl_2$. Les phases organiques ont été lavées avec de la saumure, séchées sur $MgSO_4$, puis les solvants ont été évaporés sous pression réduite. La purification du résidu obtenu par chromatographie a fourni le β-lactame bicyclique C-2 (440,6 mg, 74% sur 2 étapes). A une solution de ce β-lactame C-2 (71,3 mg, 0,13 mmol) dans de l'éther (1,5 mL) $LiAlH_4$ (9,6 mg, 0,25 mmol) a été ajouté à 0 °C. Le mélange a été agité à température ambiante durant 2 h puis dilué avec du $CH_2Cl_2$, du $NH_4Cl$ aqueux et quelques gouttes de NaOH aqueux jusqu'à pH basique. La phase aqueuse a été extraite (trois fois) avec du $CH_2Cl_2$. Les phases organiques ont été lavées avec de la saumure, séchées sur $MgSO_4$, et les solvants ont été évaporés sous pression réduite pour donner la pipéridine D-2 (huile translucide, 61,1 mg, 85%) après purification par chromatographie. Ce composé (26,3 mg, 0,463 mmol) a été débenzylé selon la procédure générale B pour donner la pipéridine (IId) (9,3 mg, 97%).

On obtient une laque jaune pâle, de caractéristiques :

$[\alpha]^{20}_D$ +8,2 (*c* 0,61, $CH_3OH$);

IR ν 3287, 2920, 1644, 1431, 1081 $cm^{-1}$;
RMN $^1H$ (500 MHz, $CD_3OD$) $\delta$ 1.86 (t, *J* = 6.5 Hz, 2H), 2.65 (dd, *J* = 11.2, 12.6 Hz, 1H), 2.92 (dd, *J* = 5.4, 13.1 Hz, 1H), 3.33-3.43 (m, 2H), 3.48 (d, *J* = 9.1 Hz, 1H), 3.58 (d, *J* = 10.8 Hz, 1H), 3.64-3.76 (m, 3H) ppm;
RMN $^{13}C$ (125 MHz, $CD_3OD$) $\delta$ 30.5 ($CH_2$), 45.8 ($CH_2$), 58.8 ($CH_2$), 60.9 ($C_q$), 65.7 ($CH_2$), 73.5 (CH), 74.6 (CH), 76.3 (CH) ppm;
HRMS ($ESI^+$) Calc. $C_8H_{18}NO_5$ $[M + H]^+$: *m*/*z* = 208,1185; Trouv. *m*/*z* = 208,1184. A.3/ Voie de synthèse 3

La voie de synthèse mise en oeuvre pour obtenir des composés répondant à la formule générale (I'd) ci-dessous relève de la voie de synthèse générale 3 montrée sur la figure 3.

Composé de formule (IIw)

Le composé de formule (IIw) ci-avant (2*S*,3*S*,4*R*,5*S*)-3,4,5-tris(benzyloxy)-2-((benzyloxy)méthyl)-2-(pyridin-2-ylsulfonyl)pipéridin-1-ol est préparé de la façon suivante. Une solution de nitrone de formule (III) (196 mg ; 0,366 mmol) et de 2-pyridyl-méthylsulfone (VIa) (74,6 mg ; 0,475 mmol) dans du THF anhydre (10 mL) sous atmosphère d'argon a été refroidie à -78 °C. Du LiHMDS 1 M dans le THF (548 μL ; 0,548 mmol) a été ajouté goutte à goutte puis le mélange réactionnel a été agité à cette même température jusqu'à complétion de la réaction. Après ajout d'eau et d'acétate d'éthyle, la phase aqueuse a été extraite (trois fois) avec de l'acétate d'éthyle. Les phases organiques rassemblées ont été lavées avec de la saumure, séchées sur $MgSO_4$ puis concentrées sous pression réduite. La purification du résidu obtenu par chromatographie a fourni le composé de formule A-3a ($R^7$ = H) 115 mg ; 55%). Ce composé a été débenzylé selon une variante de la procédure générale B pour donner la pipéridine (IIw).

A.4/ Voies de synthèse 4

Les voies de synthèses mises en oeuvre pour obtenir des composés répondant à la formule générale (I), notamment des composés répondant à la formule générale (I'a), ci-dessous relèvent d'une voie de synthèse générale 4 montrée sur la figure 4.

Composé de formule (IIa)

Le composé de formule (IIa) ci-avant ((2*R*,3*R*,4*R*,5*S*)-2-éthyl-2-(hydroxyméthyl)pipéridine-3,4,5-triol) a été préparé selon le protocole général B décrit dans la voie de synthèse 1, à partir de la pipéridine D-4b ($R^{17}$ = vinyl, 74,0 mg, 0,137 mmol), obtenue comme décrit dans la publication de Boisson et al., 2015, Org. Lett. 17: 3662-3665, et a été isolé avec un rendement de 95% (24,8 mg).

On obtient une laque jaune pâle, de caractéristiques :

$[\alpha]^{20}_{D}$ +1,6 (*c* 1,22, $CH_3OH$);

IR ν 3286, 2939, 1643, 1445, 1096 $cm^{-1}$;
RMN $^1H$ (500 MHz, $CD_3OD$) $\delta$ 0.86 (t, *J* = 7.6 Hz, 3H), 1.55-1.68 (m, 2H), 2.51 (dd, *J* = 10.8, 12.8 Hz, 1H), 2.89 (dd, *J* = 5.4, 13.0 Hz, 1H), 3.34-3.40 (m, 1H), 3.42 (d, *J* = 10.7 Hz, 1H), 3.44-3.51 (m, 2H), 3.62 (d, *J* = 10.7 Hz, 1H) ppm; RMN $^{13}C$ (125 MHz, $CD_3OD$) δ 7.0 ($CH_3$), 20.1 ($CH_2$), 45.7 ($CH_2$), 60.5 ($C_q$), 65.0 ($CH_2$), 73.6 (CH), 75.1 (CH), 76.4 (CH) ppm;
HRMS ($ESI^+$) Calc. $C_8H_{17}NO_4$ $[M + H]^+$: *m*/*z* = 192,1236 ; Trouv. *m*/*z* = 192,1237.

Composé de formule (IIb)

Le composé de formule (IIb) ci-avant ((2*R*,3*R*,4*R*,5*S*)-2-(hydroxyméthyl)-2-propylpipéridine-3,4,5-triol) a été préparé selon le protocole général B décrit dans la voie de synthèse 1, à partir de la pipéridine D-4a ($R^{17}$ = allyl, 39,7 mg, 0,071 mmol), obtenue comme décrit dans la publication de Vieira Da Cruz et al., 2017, J. Org. Chem. 82: 9866-9872, et a été isolé avec un rendement de 79% (11,4 mg).
On obtient une laque incolore, de caractéristiques :

$[\alpha]^{20}_{D}$ +5,4 (*c* 0,55, $CH_3OH$);

IR ν 3291, 2932, 1625, 1454, 1090 $cm^{-1}$;
RMN $^1H$ (500 MHz, $CD_3OD$) $\delta$ 0.94 (t, *J* = 7.2 Hz, 3H), 1.22-1.42 (m, 2H), 1.49-1.57 (m, 2H), 2.53 (dd, *J* = 10.8, 13.0 Hz, 1H), 2.89 (dd, *J* = 5.5, 13.0 Hz, 1H), 3.34-3.41 (m, 1H), 3.42 (d, *J* = 10.7 Hz, 1H), 3.45-3.51 (m, 2H), 3.64 (d, *J* = 10.7 Hz, 1H) ppm;
RMN $^{13}C$ (125 MHz, $CD_3OD$) $\delta$ 15.3 ($CH_3$), 16.6 ($CH_2$), 30.4 ($CH_2$), 45.7 ($CH_2$), 60.7 ($C_q$), 65.3 ($CH_2$), 73.5 (CH), 74.9 (CH), 76.3 (CH) ppm;
HRMS ($ESI^+$) Calc. $C_9H_{20}NO_4$ $[M + H]^+$ : *m*/*z* = 206,1392 ; Trouv. *m*/*z* = 206,1396

Composé de formule (IIe)

Le composé de formule (IIe) ci-avant (2*R*,3*R*,4*R*,5*S*)-2-éthyl-2-(hydroxyméthyl)-1-propylpipéridine-3,4,5-triol est préparé de la façon suivante.
La pipéridine E-4b ($R^1$ = allyl, $R^{17}$ = vinyl ; 62 mg, 0,105 mmol), obtenue comme décrit dans la publication de Boisson et al., 2015, Org. Lett. 17: 3662-3665, a été dissoute dans de l'acétonitrile anhydre (0,5 mL), traitée par du chlorure de 2-nitrobenzenesulfonyle (186 mg, 0,84 mmol) à 0 °C, puis par de l'hydrazine monohydrate (0,08 mL, 1,68 mmol), additionnée goutte à goutte à 0 °C. Ce mélange réactionnel a été maintenu sous agitation à température ambiante pendant 28 h, puis de l'eau a été ajoutée et la phase aqueuse a été extraite (3 fois) avec du dichlorométhane. Les phases organiques rassemblées ont été lavées par de la saumure, séchées sur $MgSO_4$ et concentrées sous pression réduite. Après purification par chromatographie, un mélange des produits d'hydrogénation du seul groupe allyle et d'hydrogénation des groupes allyle et vinyle a été isolé (58 mg, 94%, mélange 3:7). Ce mélange a été dissout dans le méthanol (1,5 mL), puis traité par le réactif de Pearlman (20% $Pd(OH)_2/C$, 16,8 mg, 0,119 mmol) et du HCl (solution 2M dans l'éther, 0,2 mL, 0,4 mmol) sous atmosphère d'hydrogène (1 atm) et sous agitation vigoureuse durant 40 h. Le mélange a été filtré sur Célite®, la Célite® a été rincée plusieurs fois par du méthanol, puis le filtrat a été concentré sous pression réduite. Le résidu a été solubilisé dans l'eau et purifié sur résine échangeuse de cations (DOWEX 50W-X8, forme $H^+$ ; élution par une solution aqueuse de $NH_4OH$ 1M) pour fournir le produit (IIe) (11,9 mg, 49%).
On obtient un solide beige, de caractéristiques :

$[\alpha]^{20}_{D}$ -40,7 (c 0,60, $CH_3OH$);

IR ν 3363, 2962, 2932, 2874, 2826, 1653, 1464, 1379, 1097, 1048, 1016 $cm^{-1}$ RMN $^1H$ (500 MHz, $CD_3OD$) δ□0.90 (t, J = 7.5 Hz, 3H), 0.96 (t, J = 7.5 Hz, 3H), 1.39-1.50 (m, 1H), 1.51-1.60 (m, 1H), 1.61-1.75 (m, 2H), 2.26-2.34 (m, 1H), 2.42-2.51 (m, 1H), 2.76-2.85 (m, 1H), 2.93-3.00 (m, 1H), 3.40-3.52 (m, 3H), 3,65 (d, J = 11.0 Hz, 1H), 3.79 (d, J = 11.0 Hz, 1H) ppm;
RMN $^{13}C$ (125 MHz, $CD_3OD$) δ 9.6 ($CH_3$), 11.9 ($CH_3$), 19.2 ($CH_2$), 23.2 ($CH_2$), 52.0 ($CH_2$), 52.2 ($CH_2$), 62.5 ($CH_2$), 71.5 (CH), 74.9 (CH), 76.4 (CH) ppm;

HRMS ($ESI^+$) Calc. $C_{11}H_{24}NO_4$ $[M + H]^+$: m/z = 234,1700 ; Trouv. m/z = 234,1696.

Composé de formule (IIf)

Le composé de formule (IIf) ci-avant (1*R*,2*R*,3*S*,9a*R*)-9a-(hydroxyméthyl)octahydro-1H-quinolizine-1,2,3-triol est préparé de la façon suivante.

La pipéridine E-4a ($R^1 = R^{17}$ = allyl ; 35 mg, 0,06 mmol), obtenue comme décrit dans la publication de Vieira Da Cruz et al., 2017, J. Org. Chem. 82: 9866-9872, a été mise en solution dans du $CH_2Cl_2$ anhydre (3 mL). Cette solution a été soigneusement dégazée, puis le catalyseur de Grubbs II (1,3 mg, 2,5 mol%) a été additionné. Le mélange a été maintenu sous agitation à température ambiante durant 20 h, puis filtré sur silice. La silice a été rincée par de l'acétate d'éthyle et du méthanol, et les solvants ont été évaporés sous pression réduite. Après purification du résidu ainsi obtenu par chromatographie, le produit de cyclisation par méthathèse d'oléfines F-4a (32 mg, 95%) a été isolé. Ce dernier (34 mg, 0,06 mmol) a été dissout dans le méthanol (1 mL), puis traité par le réactif de Pearlman (20% $Pd(OH)_2/C$, 14 mg, 0,10 mmol) et du HCl (solution 2M dans l'éther, 0,045 mL, 0,09 mmol) sous atmosphère d'hydrogène (5 bars) et sous agitation vigoureuse durant 17 h. Le mélange a été filtré sur Célite®, la Célite® a été rincée plusieurs fois par du méthanol, puis le filtrat a été concentré sous pression réduite. Le résidu a été solubilisé dans l'eau et purifié sur résine échangeuse de cations (DOWEX® 50W-X8, forme $H^+$ ; élution par une solution aqueuse de $NH_4OH$ 1M) pour fournir le produit (IIf) (9 mg, 73%).

On obtient une huile incolore, de caractéristiques :

$[\alpha]^{20}_D$ +16,6 (*c* 0,41, $CH_3OH$);

IR ν 3336, 2942, 2869, 1053 $cm^{-1}$;
RMN $^1H$ (500 MHz, $CD_3OD$) $\delta$ 1.28 (br d, *J* = 11.6 Hz, 1H), 1.37-1.44 (m, 1H), 1.62-1.76 (m, 3H), 1.81-1.94 (m, 1H), 2.66 (dd, *J* = 3.0, 14.0 Hz, 1H), 2.72 (dd, *J* = 4.9, 11.3 Hz, 1H), 3.12 (t, *J* = 11.0 Hz, 1H), 3.20 (*p*std, *J* = 3.0, 14.0 Hz, 1H), 3.38 (pst, *J* = 9.3 Hz, 1H), 3.45-3.52 (m, 2H), 3.68 (d, *J* = 11.1 Hz, 1H), 3.95 (d, *J* = 11.1 Hz, 1H) ppm;
RMN $^{13}C$ (125 MHz, $CD_3OD$) $\delta$ 18.7 ($CH_2$), 19.2 ($CH_2$), 20.7 ($CH_2$), 48.5 ($CH_2$), 53.3 ($CH_2$), 60.7 ($CH_2$), 62.0 ($C_q$), 71.4 (CH), 74.1 (CH), 75.9 (CH) ppm; HRMS ($ESI^+$) Calc. $C_{10}H_{20}NO_4$ $[M + H]^+$: *m/z* = 218,1392 ; Trouv. *m/z* = 218,1396.

Composé de formule (IIg)

Le composé de formule (IIg) ci-avant (6*S*, 7*R*, 8*R*, 8a*R*)-8a-(hydroxyméthyl)-octahydroindolizine-6,7,8-triol est préparé de la façon suivante.

La pipéridine E-4b ($R^1$ = allyl, $R^{17}$ = vinyl ; 74 mg, 0,125 mmol), obtenue comme décrit dans la publication de Boisson et al., 2015, Org. Lett. 17: 3662-3665, a été mise en solution dans du $CH_2Cl_2$ anhydre (6,3 mL). Cette solution a été soigneusement dégazée, puis le catalyseur de Grubbs II (5,3 mg, 0,006 mmol) a été additionné, et le mélange a été chauffé à 40 °C pendant 3 h, sous agitation. Après refroidissement à température ambiante, le mélange réactionnel a été filtré sur silice, la silice a été rincée par de l'éther, et les solvants ont été évaporés sous pression réduite. Après purification du résidu ainsi obtenu par chromatographie, le produit de cyclisation par méthathèse d'oléfines F-4b (65 mg, 93%) a été isolé. Ce dernier (50 mg, 0.089 mmol) a été dissout dans le méthanol (1,4 mL), puis traité par le réactif de Pearlman (20% $Pd(OH)_2/C$, 17,9 mg, 0,025 mmol) et du HCl (solution 2M dans l'éther, 0,066 mL, 0,133 mmol) sous atmosphère d'hydrogène (5 bars) et sous agitation vigoureuse durant 17 h. Le mélange a été filtré sur Célite®, la Célite® a été rincée plusieurs fois par du méthanol, puis le filtrat a été concentré sous pression réduite. Le résidu a été solubilisé dans l'eau et purifié sur résine échangeuse de cations (DOWEX 50W-X8, forme $H^+$ ; élution par une solution aqueuse de $NH_4OH$ 1M) pour fournir le produit (IIg) (16 mg, 89%).

On obtient un solide beige, de caractéristiques :

$[\alpha]^{20}_D$ +27,9 (*c* 0,41, $CH_3OH$);

IR ν 3330, 2924, 1652, 1031 $cm^{-1}$;
RMN $^1H$ (500 MHz, $CD_3OD$) $\delta$ 1.46-1.53 (m, 1H), 1.67-1.76 (m, 1H), 1.88-1.96 (m, 1H), 1.98-2.08 (m, 1H), 2.46 (pst, *J* = 11.0 Hz, 1H), 2.80-2.89 (m, 2H), 3.09-3.18 (m, 1H), 3.33-3.41 (m, 2H), 3.43-3.49 (m, 1H), 3.53 (d, *J* = 11.5 Hz, 1H), 3.76 (d, *J* = 9.5 Hz, 1H) ppm;
RMN $^{13}C$ (125 MHz, $CD_3OD$) $\delta$ 21.0 ($CH_2$), 26.1 ($CH_2$), 53.8 ($CH_2$), 55.9 ($CH_2$), 63.6 ($CH_2$), 71.0 (CH), 73.0 (CH), 73.2 (Cq), 76.5 (CH) ppm;

HRMS ($ESI^+$) Calc. $C_9H_{18}NO_4$ $[M + H]^+$: *m/z* = 204.1236; Trouv. *m/z* = 204.1234.

A.5/ Voies de synthèse 5

La voie de synthèse mise en oeuvre pour obtenir le composé répondant à la formule générale (I'b) ci-dessous relève d'une voie de synthèse générale 5 montrée sur la figure 5.

Composé (!!h)

Le composé de formule (IIh) ci-avant ((3*S*,4*R*,5*R*,6*R*)-6-(hydroxyméthyl)-1-azabicyclo[4.2.0]octane-3,4,5-triol) est préparé de la façon suivante.

A une solution de β-lactame C-5 (correspondant au composé C-2 obtenu en tant qu'intermédiaire dans la voie de synthèse 2, comme décrit dans le protocole de préparation du composé (IId) ci-dessus) (57,1 mg, 0,10 mmol) dans du THF distillé, ont été ajoutés du $[Rh(COD)_2]BF_4$ (2,5 mg, 0,006 mmol), du 1,3-bis(diphénylphosphino)propane (2,7 mg, 0,006 mmol) et du PhSiHs (25 μL, 0,20 mmol). La solution a été agitée à 50 °C durant 4 h. Après refroidissement, le mélange réactionnel a été dilué avec de l'AcOEt et du NaOH aqueux (1 M). La phase aqueuse a été extraite (trois fois) avec de l'AcOEt. Les phases organiques ont été lavées avec de la saumure, séchées sur $MgSO_4$, et les solvants ont été évaporés sous pression réduite pour donner la conidine G-5 (33,2 mg, 60%) après purification par chromatographie. Ce composé (20,0 mg, 0.04 mmol) a été débenzylé selon la procédure générale B pour donner le composé (IIh) (6,3 mg, 93%).

On obtient une huile jaune pâle, de caractéristiques :

$[\alpha]^{20}_D$ -5,17 (*c* 0,58, $CH_3OH$);

IR ν 3234, 2917, 1429, 1030 $cm^{-1}$;
RMN $^1H$ (500 MHz, $CD_3OD$) δ 2.18-2.26 (m, 1H), 2.55-2.63 (m, 1H), 2.89 (dd, *J* = 5.9, 13.3 Hz, 1H), 3.21 (dd, *J* = 5.1, 13.3 Hz, 1H), 3.62 (d, *J* = 11.7 Hz, 1H), 3.69-3.80 (m, 5H), 3.99 (pst, *J* = 6.5 Hz, 1H) ppm;
RMN $^{13}C$ (125 MHz, $CD_3OD$) δ 21.3 ($CH_2$), 51.4 ($CH_2$), 52.9 ($CH_2$), 65.5 ($CH_2$), 71.2 (CH), 72.7 (CH), 73.2 ($C_q$), 76.8 (CH) ppm;
HRMS ($ESI^+$) Calc. $C_8H_{16}NO_4$ $[M+H]^+$ *m/z* = 190,1074 ; Trouv. *m/z* = 190,1074. Protocole Général C - alkylation réductrice du β-lactame C-5 conduisant à des conidines de formule générale (I'b)

A une solution de β-lactame C-5 (1 équiv.) et de catalyseur de Vaska (4% mol) dans du dichlorométhane anhydre sous atmosphère d'argon, est ajouté du tétraméthyldisiloxane (TMDS, 2 équiv.) à température ambiante. Le mélange est agité durant 45 min puis refroidi à -78 °C. Le réactif de grignard (2 équiv.) est ajouté goutte à goutte puis le mélange est agité durant 7-10 min à cette même température avant de remonter à température ambiante. Il est ensuite agité durant 21 h. Une solution aqueuse saturée de $NH_4Cl$ est ajoutée puis le mélange résultant est dilué avec du $CH_2Cl_2$. La phase organique est séparée et la phase aqueuse est extraite deux fois avec du $CH_2Cl_2$. Les phases organiques sont lavées avec de la saumure, séchées sur $MgSO_4$ et évaporées sont pression réduite. Les conidines de formule (I'b) ainsi formées sont purifiées par chromatographie sur gel de silice avant d'être réduites et débenzylées selon le protocole général B ci-avant.

Composé (IIz2)

Le composé de formule (IIz2) ci-avant ((3*S*,4*R*,5*R*,6*R*,8*R*)-8-benzyl-6-(hydroxymethyl)-1-azabicyclo[4.2.0]octane-3,4,5-triol : 20,3 mg ; 65 % pour 2 étapes) a été préparé selon les procédures générales C et B, à partir du β-lactame C-5 (70,1 mg ; 0,124 mmol) et de chlorure de benzylmagnésium 2 M dans le THF ($R^{14}$ = Bn ; 36,3 μL ; 0,248 mmol).

On obtient une laque translucide, de caractéristiques :

RMN $^1H$ (500 MHz, $CD_3OD$) δ 7.39-7.20 (m, 5H), 4.45-4.34 (m, 1H), 3.95 (t, *J* = 5.7 Hz, 1H), 3.80-3.74 (m, 2H), 3.70 (d, *J* = 5.6 Hz, 1H), 3.56 (d, *J* = 12.1 Hz, 1H), 3.21-3.12 (m, 2H), 3.00 (dd, *J* = 13.6, 8.4 Hz, 1H), 2.94 (dd, *J* = 13.1, 6.5 Hz, 1H), 2.60 (dd, *J* = 11.9, 9.0 Hz, 1H), 2.32-2.20 (m, 1H) ppm;
RMN $^{13}C$ (125 MHz, $CD_3OD$) δ 137.1 ($^{Ar}Cq$), 130.1 ($^{Ar}CH$), 129.7 ($^{Ar}CH$), 128.0 ($^{Ar}CH$), 76.5 (CH), 71.5 (CH), 71.1 (CH), 65.7 (CH), 64.1 ($CH_2$), 51.4 ($CH_2$), 41.0 ($CH_2$), 27.3 ($CH_2$) ppm;
HRMS ($ESI^+$) Calc. $C_{15}H_{22}NO_4$ $[M + H]^+$: *m/z* = 280.15433; Trouv. *m/z* = 280.15437.

Composé (IIz3)

Le composé de formule (IIz3) ci-avant ((3*S*,4*R*,5*R*,6*R*,8*R*)-6-(hydroxymethyl)-8-methyl-1-azabicyclo[4.2.0]octane-3,4,5-triol : 8,5 mg ; 46 % pour 2 étapes) a été préparé selon les procédures générales C et B, à partir du β-lactame C-5 (192,6 mg ; 0,342 mmol) et de chlorure de méthylmagnésium 3 M dans le THF ($R^{14}$ = Me ; 228 μL ; 0,683 mmol).

On obtient un solide jaunâtre, de caractéristiques :

$[\alpha]^{20}_D$ -22,9 (*c* 1,07, MeOH);

RMN $^1H$ (500 MHz, $CD_3OD$) δ 3.99 (t, *J* = 6.7 Hz, 1H), 3.85-3.75 (m, 1H), 3.75-3.69 (m, 1H), 3.67 (d, *J* = 7.6 Hz, 1H), 3.54 (d, *J* = 11.2 Hz, 1H), 3.47 (d, *J* = 11.2 Hz, 1H), 3.10 (dd, *J* = 13.9, 5.5 Hz, 1H), 2.71 (dd, *J* = 13.8, 4.0 Hz, 1H), 2.58 (dd, *J* = 11.5, 8.9 Hz, 1H), 1.64 (dd, *J* = 10.9, 8.0 Hz, 1H), 1.21 (d, *J* = 6.1 Hz, 3H) ppm;
RMN $^{13}C$ (100 MHz, $CD_3OD$) δ 77.2 (CH), 74.7 (CH), 71.7 (CH), 68.3 (Cq), 67.4 ($CH_2$), 58.4 (CH), 52.4 ($CH_2$), 29.1 ($CH_2$), 22.0 ($CH_3$) ppm;
HRMS ($ESI^+$) Calc. $C_9H_{18}NO_4$ $[M + H]^+$: *m*/*z* = 204.12303; Trouv. *m*/*z* = 204.12232.

Composé (IIz4)

Le composé de formule (IIz4) ci-avant ((3*S*,4*R*,5*R*,6*R*,8*S*)-8-cyclopentyl-6-(hydroxymethyl)-1-azabicyclo[4.2.0] octane-3,4,5-triol : 14,6 mg ;62 % pour 2 étapes) a été préparé selon les procédures générales C et B, à partir du β-lactame C-5 (70,0 mg ; 0,124 mmol) et de chlorure de cyclopentylmagnésium 2 M dans l'éther ($R^{14}$ = cPent ; 36,4 μL ; 0.248 mmol).

On obtient un solide jaunâtre, de caractéristiques :

RMN $^1H$ (500 MHz, $CD_3OD$) δ 3.98 (t, *J* = 7.0 Hz, 1H), 3.76-3.63 (m, 2H), 3.58-3.36 (m, 3H), 3.10 (dd, *J* = 14.5, 5.0 Hz, 1H), 2.76 ( dd, *J* = 14.5, 4.0 Hz, 1H), 2.50 (t, *J*= 11.8 Hz, 1H), 2.06-1.95 (m, 1H), 1.89-1.76 (m, 1H), 1.74-1.47 (m, 6H), 1.36-1.22 (m, 1H), 1.15-1.00 (m, 1H) ppm;
RMN $^{13}C$ (125 MHz, $CD_3OD$) δ 77.7 (CH), 75.0 (CH), 71.9 (CH), 67.5 ($CH_2$), 67.2 (CH), 66.3 (Cq), 54.2 ($CH_2$), 48.9 (CH), 31.0 ($CH_2$), 29.1 ($CH_2$), 27.0 ($CH_2$), 26.4 ($CH_2$), 25.9 ($CH_2$) ppm;
HRMS ($ESI^+$) Calc. $C_{13}H_{24}NO_4$ $[M + H]^+$: *m*/*z* = 258.16998; Trouv. *m*/*z* = 258.16987.

Composé (IIz5)

Le composé de formule (IIz5) ci-avant ((3*S*,4*R*,5*R*,6*R*,8*R*)-6-(hydroxymethyl)-8-pentyl-1-azabicyclo[4.2.0]octane-3,4,5-triol : 19.1 mg ; 45 % pour 2 étapes) a été préparé selon les procédures générales C et B, à partir du β-lactame C-5 (70,0 mg ; 0,124 mmol) et de chlorure de pentylmagnésium 2 M dans le THF ($R^{14}$ = *n*-Pent ; 33,6 μL ; 0,248 mmol).

On obtient un solide jaune, de caractéristiques :

$[\alpha]^{20}_D$ -31,1 (*c* 0,46, MeOH);

RMN $^1H$ (500 MHz, $CD_3OD$) δ 3.99 (t, *J* = 7.0 Hz, 1H), 3.74-3.64 (m, 2H), 3.64-3.55 (m, 1H), 3.51 (d, *J* = 11.1 Hz, 1H), 3.46 (d, *J* = 11.1 Hz, 1H), 3.09 (dd, *J* = 13.7, 5.4 Hz, 1H), 2.70 (dd, *J* = 13.7, 4.0 Hz, 1H), 2.52 (dd, *J* = 11.4, 8.9 Hz, 1H), 1.68-1.53 (m, 2H), 1.51-1.39 (m, 1H), 1.39-1.21 (m, 6H), 0.90 (t, *J* = 6.7 Hz, 3H) ppm;
RMN $^{13}C$ (100 MHz, $CD_3OD$) δ 77.5 (CH), 75.2 (CH), 71.9 (CH), 67.8 ($CH_2$), 66.4 (Cq), 62.4 (CH), 53.8 ($CH_2$), 38.8 ($CH_2$), 33.0 ($CH_2$), 27.9 ($CH_2$), 26.5 ($CH_2$), 23.7 ($CH_2$), 14.3 ($CH_3$) ppm;
HRMS ($ESI^+$) Calc. $C_{13}H_{26}NO_4$ $[M + H]^+$: *m*/*z* = 260.18563; Trouv. *m*/*z* = 260.18561.

A.6/ Voie de synthèse 7

La voie de synthèse mise en oeuvre pour obtenir les composés répondant à la formule générale (I'c) ci-dessous relève de la voie de synthèse générale 7 montrée sur la figure 7.

Composé (IIk)

Le composé de formule (IIk) ci-dessus ((3*S*,4*R*,5*R*,6*R*,7*S*)-6,7-bis(hydroxyméthyl)-1-azabicyclo[4.1.0]heptane-3,4,5-triol) est obtenu de la façon suivante.

Un mélange de nitrone (III) (P = Bn ; 193 mg, 0,360 mmol) et d'alcyne Vc ($R^8$ = NBnTs ; 308 mg, 1,079 mmol) en solution dans du $CH_2Cl_2$ (0,4 mL) a été agité à température ambiante pendant 3 jours, puis le mélange réactionnel a été concentré sous pression réduite. Le résidu (cycloadduit brut B-7c) a été dissout dans l'éthanol (0,1 M), transféré dans un tube scellé, et chauffé à 110 °C (sonde IR) sous irradiation micro-ondes durant 15 min. Après concentration sous pression réduite, le résidu a été purifié par chromatographie pour donner l'acylaziridine H-7c (P = Bn, $R^8$ = NBnTs ; 238 mg, 80% sur 2 étapes). Une fraction de ce composé (58 mg, 0,070 mmol) a été dissoute dans du THF (1 mL), puis du $LiAlH_4$ (6 mg, 0,157 mmol) a été ajouté à 0 °C. Le mélange a été agité durant 1,5 h à température ambiante. Après ajout d'eau (0.1 mL) et d'une solution aqueuse à 10% de NaOH (0.1 mL), le mélange réactionnel a été agité à température ambiante durant 2 h puis filtré sur Célite®. Le filtrat, concentré sous pression réduite, a été purifié par chromatographie pour isoler l'aziridine-alcool J-7c (P = Bn, $R^{16}$ = H ; 34,2 mg, 86%). Une solution de cet aziridine-alcool J-7c (28 mg, 0,067 mmol) dans du THF (2 mL) a été ajoutée à une solution de Lithium (54 mg, 7,78 mmol) dans l'ammoniac liquide (10 mL) à -78 °C. Le mélange a été agité durant 45 min à -78 °C, puis de l'eau milliQ (0,5 mL) et du MeOH (2 mL) ont été ajoutés. Après retour de la solution à température ambiante, les solvants ont été évaporés sous pression réduite. Le résidu a été dissout dans de l'eau milliQ et neutralisé avec de l'Amberlite® IR-120 (22,8 g) préalablement traitée avec du HCl 1M (15 mL). La résine a été introduite dans une colonne, lavée avec de l'eau, puis le composé (IIk) a été élué avec une solution 1M de $NH_4OH$ aqueux. Le solide obtenu après évaporation des solvants sous pression réduite a été purifié par chromatographie pour donner l'iminosucre (IIk) pur ($R^{16}$ = H ; 7 mg, 60%).

On obtient un solide blanc, de caractéristiques :

$[\alpha]20_D$ = +2,90 (*c* 0,34, $CH_3OH$);

IR ν 3409, 3010, 2926, 1084, 1032 $cm^{-1}$;

RMN $^1H$ (500 MHz, $CD_3OD$) $\delta$ 4.16 (d, *J* = 7.9 Hz, 1H), 3.79 (d, *J* = 11.6 Hz, 1H), 3.60-3.50 (m, 3H), 3.47-3.40 (m, 1H), 3.33-3.25 (m, 2H), 2.55 (dd, *J* = 10.6, 12.4 Hz, 1H), 2.19 (dd, *J* = 6.8, 5.3 Hz, 1H) ppm;

RMN $^{13}C$ (125 MHz, $CD_3OD$) $\delta$ 76.0 (CH), 73.2 (CH), 70.6 (CH), 63.2 ($CH_2$), 62.0 ($CH_2$), 57.0 ($CH_2$), 51.9 ($C_q$), 51.6 (CH) ppm;

HRMS ($ESI^+$) Calc. $C_8H_{16}NO_5$ $[M + H]^+$ *m/z* = 206,1028 ; Trouv. *m/z* = 206,1028.

Composé (IIj)

Le compose de formule (IIj) ci-dessus ((3*S*,4*R*,5*R*,6*R*,7*S*)-7-((S)-1,2-dihydroxyéthyl)-6-(hydroxyméthyl)-1-aza-bicyclo[4.1.0]heptane-3,4,5-triol) est obtenu comme suit.

L'acylaziridine H-7d (P = Bn, $R^8$ = $CH_2OA_C$ ; 33 mg, 0,052 mmol), obtenue comme décrit dans la publication de Tangara et al., 2017, Org. Lett. 19: 4842-4845, a été dissoute dans du THF (1 mL), puis du $LiAlH_4$ (38 mg, 0,165 mmol) a été ajouté à 0 °C et le mélange a été agité 1 h à température ambiante. Après ajout d'eau (0,1 mL) et d'une solution aqueuse à 10% de NaOH (0,1 mL), le mélange réactionnel a été agité à température ambiante durant 2 heures puis filtré sur Célite®. Le filtrat, concentré sous pression réduite, a été purifié par chromatographie pour isoler une huile translucide J-7d ($R^{16}$ = $CH_2OH$ ; 26 mg, 83%). Une solution de cet aziridine-alcool J-7d (40 mg, 0,067 mmol) dans du THF (2 mL) a été ajoutée à une solution de lithium (25 mg, 3,571 mmol) dans l'ammoniac liquide (10 mL) à -78 °C. Le mélange a été agité durant 45 min à - 78 °C puis de l'eau milliQ (0,5 mL) et du MeOH (2 mL) ont été ajoutés. Après retour de la solution à température ambiante, les solvants ont été évaporés sous pression réduite. Le résidu a été dissout dans de l'eau milliQ et neutralisé avec de l'Amberlite® IR-120 (8,5 g) préalablement traitée avec du HCl 1M (5 mL). La résine a été introduite dans une colonne, lavée avec de l'eau, puis le composé (IIj) a été élué avec une solution 1M de $NH_4OH$ aqueux. Le solide obtenu après évaporation des solvants sous pression réduite a été purifié par chromatographie pour donner l'iminosucre (IIj) pur ($R^{16}$ = $CH_2OH$ ; 10,1 mg, 64%).

On obtient un solide blanc, de caractéristiques :

$[\alpha]^{20}_D$ = +5,90 (*c* 0,34, $CH_3OH$);

IR: ν 3249, 2939, 2885, 1748, 1656, 1404, 1031, 995 $cm^{-1}$;

RMN $^1H$ (500 MHz, $CD_3OD$) $\delta$ 4.16 (d, *J* = 8.0 Hz, 1H), 3.91 (d, *J* = 11.6 Hz, 1H), 3.71 (dd, *J* = 3.5, 11.4 Hz, 1H), 3.61 (dd, *J* = 5.8, 11.4 Hz, 1H), 3.54-3.48 (m, 1H), 3.48-3.41 (m, 1H), 3.39 (d, *J* = 11.6 Hz, 1H), 3.30-3.25 (m, 2H), 2.52 (dd, J = 10.2, 12.2 Hz, 1H), 2.10 (d, *J* = 8.7 Hz, 1H) ppm;

RMN $^{13}C$ (125 MHz, $CD_3OD$) $\delta$ 75.9 (CH), 73.1 (CH), 72.4 (CH), 70.7 (CH), 66.1 ($CH_2$), 63.8 ($CH_2$), 56.5 ($CH_2$), 52.0 ($C_q$), 51.2 (CH) ppm;

HRMS (ESI+) Calc. $C_9H_{18}NO_6$ $[M + H]^+$ *m/z* = 236,1129 ; Trouv. *m/z* = 236,1124.

Composé (IIm)

Le composé de formule (IIm) ci-dessus (3*S*,4*R*,5*R*,6*R*,7*S*)-7-((S)-hydroxy(phényl)méthyl)-6-(hydroxyméthyl)-1-azabicyclo[4.1.0]heptane-3,4,5-triol est obtenu comme suit.

Les cycloadduits B-7e (P = Ac, $R^8$ = Ph ; 15,2 mg, 0,030 mmol, mélange 4:1 de diastéréoisomères), obtenus comme décrit dans la publication de Tangara et al., 2018, New J. Chem. 42: 16735-16743, ont été dissous dans du dichloroéthane (solution 0,1 M) dans un tube scellé. La solution a été chauffée à 110 °C (sonde IR) sous irradiation par des micro-ondes durant 45 min (30+15). Après évaporation du solvant sous pression réduite, la purification du résidu par chromatographie a permis d'isoler les acylaziridines H-7e (P = Ac, $R^8$ = Ph ; 12 mg, 79%) sous forme d'un mélange 9:1 de deux diastéréoisomères. Ces acylaziridines (30 mg, 0,067 mmol) ont été solubilisées dans du THF (1,2 mL), la solution a été refroidie à 0 °C puis du $LiAlH_4$ (5,6 mg, 0,147 mmol) a été ajouté. Le mélange réactionnel a été agité à température ambiante durant 1,5 h, puis de l'eau (0,1 mL) et une solution aqueuse de NaOH à 10% (0,1 mL) lui ont été ajoutés. Après agitation à température ambiante pendant 1,5 h, ce mélange a été filtré sur Célite®, le filtrat a été concentré sous pression réduite, et le résidu a été purifié par chromatographie pour fournir l'aziridinyl iminosucre (IIm) ($R^{16}$ = Ph ; 15 mg, 82%) sous forme d'un seul diastéréoisomère.

On obtient une huile incolore, de caractéristiques :

$[\alpha]^{20}_D$ -4,1 (c 1,58, $CH_3OH$);

IR ν 3296, 2931, 1557, 1409, 1053, 697 $cm^{-1}$;

RMN $^1H$ (500 MHz, $CD_3OD$) δ 7.49-7.44 (m, 2H), 7.40-7.35 (m, 2H), 7.31-7.25 (m, 1H), 4.29 (d, J = 8.7 Hz, 1H), 4.18 (d, J = 7.9 Hz, 1H), 4.01 (d, J = 11.6 Hz, 1H) , 3.56 (d, J = 11.6 Hz, 1H), 3.48-3.37 (m, 2H), 3.26 (dd, J = 9.1, 8.0 Hz, 1H), 2.26-2.18 (m, 2H) ppm;

RMN $^{13}C$ (125 MHz, $CD_3OD$) δ 144.6 ($^{Ar}C_q$), 129.5 ($^{Ar}CH$), 128.6 ($^{Ar}CH$), 127.1 ($^{Ar}CH$), 75.8 (CH), 73.8 (CH), 73.1 (CH), 70.7 (CH), 63.6 ($CH_2$), 56.4 ($CH_2$), 56.0 (CH), 52.8 ($C_q$) ppm;

HRMS (ESI+) Calc. $C_{14}H_{20}NO_5$ [M + H]+: m/z = 282,1341 ; Trouv. m/z = 282,1346.

Composé (IIn)

Le compose de formule (IIn) ci-dessus (3*S*,4*R*,5*R*,6*R*,7*S*)-7-((R)-cyclohexyl(hydroxy)methyl)-6-(hydroxymethyl)-1-azabicyclo[4.1.0]heptane-3,4,5-triol est obtenu comme suit.

L'acylaziridine H-7f (P = Bn, $R^8$ = c-Hex ; 77 mg, 0,119 mmol), obtenue comme décrit dans la publication de Tangara et al., 2017, Org. Lett. 19: 4842-4845, a été dissoute dans l'éthanol (1,4 mL), refroidie à 0 °C, puis du $NaBH_4$ (13 mg, 0,342 mmol) a été ajouté. Le mélange a été agité à la même température durant 3,3 h, puis de l'eau, une solution aqueuse de NaOH à 10% et du $CH_2Cl_2$ ont été ajoutés. La phase aqueuse a été extraite (3 fois) par le $CH_2Cl_2$, les phases organiques ont été rassemblées, séchées sur $MgSO_4$ et concentrées sous pression réduite. Le résidu ainsi obtenu a été filtré sur silice pour isoler l'aziridine-alcool J-7f ($R^{16}$ = c-Hex ; 72 mg, 94%) sous forme d'huile. Une solution de cet aziridine-alcool J-7f (42 mg, 0,064 mmol) dans du THF (4 mL) a été ajoutée à une solution de lithium (12 mg, 2,0 mmol) dans l'ammoniac liquide (10 mL) à - 78 °C. Le mélange réactionnel a été agité durant 45 min à -78 °C, puis de l'eau milliQ (0,5 mL) et du MeOH (2 mL) ont été ajoutés. Après retour de la solution à température ambiante, les solvants ont été évaporés sous pression réduite. Le résidu a été dissout dans de l'eau milliQ et neutralisé avec de l'Amberlite® IR-120 (3,5 g) préalablement traitée avec du HCl 1M (2 mL). La résine a été introduite dans une colonne, lavée avec de l'eau, puis le composé (IIn) a été élué avec une solution 1M de $NH_4OH$ aqueux. Le solide obtenu après évaporation des solvants sous pression réduite a été purifié par chromatographie pour donner l'iminosucre (IIn) pur ($R^{16}$ = c-Hex ; 13 mg, 72%).

On obtient un solide blanc, de caractéristiques :

$[\alpha]^{20}_D$ +2,8 (*c* 0,80, $CH_3OH$);

IR ν 3335, 2916, 2846, 1451, 1087, 1054, 1008, 660 $cm^{-1}$;

RMN $^1H$ (500 MHz, $CD_3OD$) $\delta$ 4.17 (d, *J* = 7.8 Hz, 1H), 3.88 (d, *J* = 11.7 Hz, 1H), 3.50-3.44 (m, 2H), 3.42 (d, *J* = 11.7 Hz, 1H), 3.33-3.29 (m, 1H), 2.95 (dd, *J* = 8.6, 6.6 Hz, 1H), 2.51 (dd, *J* = 11.8, 9.0 Hz, 1H), 2.08 (d, *J* = 8.7 Hz, 1H), 1.99-1.66 (m, 5H), 1.53-1.45 (m, 1H), 1.36-1.03 (m, 5H) ppm;

RMN $^{13}C$ (125 MHz, $CD_3OD$) $\delta$ 76.3 (CH), 76.0 (CH), 73.3 (CH), 70.8 (CH), 64.1 ($CH_2$), 56.0 ($CH_2$), 52.9 (CH), 51.1

($C_q$), 45.3 (CH), 30.1 ($CH_2$), 29.7 ($CH_2$), 27.7 ($CH_2$), 27.4 ($CH_2$), 27.2 ($CH_2$) ppm;
HRMS (ESI+) Calc. $C_{14}H_{26}NO_5$ [M + H]+: *m/z* = 288,1811 ; Trouv. *m/z* = 288,1810. A.7/ Voie de synthèse 8

Une voie de synthèse mise en oeuvre pour obtenir un composé répondant à la formule générale (I'e) ci-dessous relève de la voie de synthèse générale 8 montrée sur la figure 8.

Composé (IIw1)

Le composé de formule (IIw1) ci-avant ((2*R*,3*R*,4*R*,5*S*)-2-(hydroxyméthyl)-2-(1*H*-1,2,3-triazol-4-yl)pipéridine-3,4,5-triol est préparé de la façon suivante.

Une solution d'hydroxylamine de formule A-1a (240,0 mg ; 0,377 mmol) et de poudre de Zinc (247,0 mg ; 3,77 mmol) dans un mélange 4:1 EtOH/AcOH (5 mL) a été agitée à 65 °C sous ultrasons jusqu'à complétion de la réaction. Le mélange réactionnel est filtré sur célite puis évaporé sous pression réduite. Le produit brut est redissous dans du $CH_2Cl_2$ puis traité avec du NaOH 1 M aqueux. La phase aqueuse a été extraite (trois fois) avec du $CH_2Cl_2$. Les phases organiques ont été lavées avec de la saumure, séchées sur $MgSO_4$ puis évaporées sous pression réduite. La purification du résidu obtenu par chromatographie a fourni la pipéridine correspondante D-8 (178.0 mg ; 76 %). Un mélange de cette pipéridine D-8 (176,0 mg ; 0,284 mmol), de chloroformiate de benzyle (121 μL ; 0,852 mmol) et de $K_2CO_3$ (157,0 mg ; 1,14 mmol) dans du THF anhydre (1,7 mL) a été agité à température ambiante jusqu'à complétion de la réaction. Du méthanol (2 mL) a été ajouté puis le mélange réactionnel a été agité à température ambiante jusqu'à complétion de la réaction. La purification du résidu obtenu par chromatographie a fourni le composé de formule K-8 (164,0 mg ; 78 % sur 2 étapes). A une solution de ce même composé K-8 (46,8 mg ; 0,062 mmol) et d'azoture de benzyle (247 μL ; 0,124 mmol) dans du DMF (1.5 mL) sous atmosphère inerte ont été ajoutés de l'iodure de cuivre (3,5 mg ; 0,018 mmol) et du DIPEA (108 μL ; 0,618 mmol). Le mélange réactionnel a été agité à température ambiante jusqu'à complétion de la réaction. Le produit brut a été dilué dans de l'acétate d'éthyle et lavé plusieurs fois avec de la saumure. La phase organique a été séchée sur $MgSO_4$ et évaporée sous pression réduite. La purification du résidu obtenu par chromatographie a fourni le composé de formule L-8 (38,2 mg ; 76 %). Ce composé (32,1 mg ; 0,039 mmol) a été débenzylé selon la procédure générale B pour donner la pipéridine (IIw1) (11,0 mg ; 100 %).

On obtient une laque incolore, de caractéristiques :

$[\alpha]^{20}_D$ -17,2 (c1,09, $CH_3OH$);

RMN $^1H$ (500 MHz, $CD_3OD$) δ 7.90 (s, 1H), 3.80 (d, *J* = 10.9 Hz, 1H), 3.69 (d, *J* = 9.7 Hz, 1H), 3.59 (d, *J* = 11.1 Hz, 1H), 3.52-3.45 (m, 1H), 3.27 (t, *J* = 9.3 Hz, 1H), 2.99 (dd, *J* = 12.6, 5.5 Hz, 1H), 2.54 (dd, *J* = 12.3, 11.2 Hz, 1H) ppm;
RMN $^{13}C$ (125 MHz, $CD_3OD$) δ 144.5 (Cq), 131.0 (CH), 76.9 (CH), 74.5 (CH), 73.0 (CH), 68.1 ($CH_2$), 62.5 (Cq), 47.2 ($CH_2$) ppm;
HRMS ($ESI^+$) Calc. $C_8H_{15}N_4O_4$ [M + H]+: *m/z* = 231,10878; Trouv. *m/z* = 231,10861.

**B/ Evaluations biologiques**

Les différents tests d'activité biologique suivants sont réalisés sur les composés (IIa) à (IIn) décrits ci-avant.

B.1/ Inhibition de la rhGAA déterminée par Fluopol-ABPP

L'activité inhibitrice de l'α-glucosidase acide humaine recombinante (rhGAA) des composés est effectuée en utilisant la méthode Fluopol-ABPP (pour l'anglais Fluorescence Polarization Activity Based Protein Profiling) décrite dans la publication de Lahav et al., 2017, J. Am. Chem. Soc. 139: 14192-14197. Cette technique, basée sur la compétition entre un inhibiteur et une sonde fluorescente capable de se lier de façon covalente au site actif d'une enzyme, permet de mesurer l'affinité de cet inhibiteur pour le site actif de l'enzyme rhGAA utilisée pour ces expériences par le laboratoire des Prof Herman S. Overkleeft et Johanes M. F. G. Aerts, Leiden Institute of Chemistry, Leiden University (NL), est l'enzyme commercialisée sous la dénomination Myozyme®. La détermination des concentrations inhibitrices médianes ($IC_{50}$) est effectuée dans le tampon McIlvaine (citrate-phosphate) 150 mM à pH 5,0, en présence de 0,1% de gamma-globuline bovine (p/v) et de 0,5 mg/mL de détergent Chaps (Sigma) dans des plaques à 96 puits (Griener). L'enzyme rhGAA (10 μg/mL) est pré-incubée avec des solutions d'inhibiteur (contenant 2,5% de DMSO ayant servi à préparer les solutions mères des composés) à différentes concentrations [I] dans le tampon, pendant 45 min à 37 °C. La sonde fluorescente tétra-aminométhylrhodamine (TAMRA) en solution (25 nM) dans le tampon est ensuite ajoutée au mélange. Après 4 h, les échantillons sont irradiés avec une lumière polarisée (λ = 530 nm) et la fluorescence émise (λ = 580 nm) est mesurée à l'aide d'un spectrofluorimètre Infinite® M1000Pro (Tecan). Pour chaque concentration en inhibiteur, le

pourcentage d'inhibition de l'enzyme est déterminé par la formule :

$$\%Inhibition = [(F_{mesurée}-F_{Contrôle1})/F_{contrôle2}) X 100$$

où $F_{mesurée}$ correspond à la fluorescence mesurée en présence des iminosucres ; $F_{contrôle1}$ correspond à la fluorescence mesurée en présence d'un inhibiteur puissant de l'α-glucosidase acide humaine servant de contrôle positif (100% d'inhibition), le CF 022 ((1*S*,2*R*,3*S*,4*R*,5*R*,6*R*)-2,3,4-trihydroxy-5-(hydroxyméthyl)-7-(8-azidooctyl)-7-aza-bicyclo[4.1.0] heptane), et $F_{contrôle2}$ représente la fluorescence de la sonde mesurée en absence de l'inhibiteur (0% d'inhibition).

Les valeurs d'$IC_{50}$ sont calculées par une régression non linéaire du %inhibition en fonction de la concentration [I] à l'aide du logiciel GraphPad Prism 6.0. Les résultats sont une moyenne de trois expériences identiques (triplicats).

B.2/ Inhibition de la rhGAA déterminée par la mesure de l'activité résiduelle en présence d'inhibiteur *in vitro*

De façon complémentaire, l'activité inhibitrice de l'α-glucosidase acide humaine recombinante (rhGAA) des composés a été évaluée par l'équipe du Prof Marco Moracci, Département de Biologie, Université de Naples Federico II (IT), en utilisant la méthode décrite dans la publication de Porto et al., 2012, Molecular Therapy 20: 2201-2211. L'enzyme rhGAA commercialisée sous la dénomination Myozyme® utilisée provient de résidus de perfusions de l'enzyme recombinante utilisée pour traiter par enzymothérapie les patients de la maladie de Pompe dans le Département des Sciences Médicales Translationelles, Université de Naples Federico II (IT).

Les composés sont solubilisés à différentes concentrations dans un tampon acétate de sodium 100 mM, pH 4,0, ainsi que le substrat 4-nitrophényl-α-D-glucopyranoside (20 mM). Après 2 min d'équilibration de la température à 37 °C, l'enzyme rhGAA en solution dans le même tampon est ajoutée (volume total : 200 μL). Après 2 min de réaction à 37 °C, une solution 1M de carbonate de sodium (800 μL), pH 11,0, est ajoutée et le mélange est refroidi dans la glace. L'absorbance des solutions est mesurée à 420 nm à température ambiante. L'hydrolyse spontanée du substrat est soustraite en mesurant l'absorbance de contrôles (blancs) sans enzyme. Les résultats donnés sont la moyenne d'au moins deux expériences identiques. Les données sont traitées et analysées à l'aide du logiciel Prism 5.0 (GraphPad).

B.3/ Détermination de la stabilisation thermique de la rhGAA

La stabilisation de la rhGAA en présence des composés est déterminée conformément à la méthode décrite dans la publication de Niesen et al., 2007, Nat. Protoc. 2: 2212-2221. L'enzyme rhGAA (2,5 μg, 0,1 mg/mL) est incubée en l'absence et en présence de chaque composé (à une concentration [I] = 100 μM), de colorant orange SYPRO® (Life Technologies), et de tampon phosphate de sodium (25 mM) et de NaCl (150 mM), pH 7,4 ou de tampon acétate de sodium (25 mmol) et de NaCl (150 mM), pH 4,0. La stabilité thermique de l'enzyme dans ces différentes conditions est évaluée par DSF (fluorimétrie différentielle à balayage) en faisant varier la température de 1 °C/min sur un intervalle de 25 à 95 °C, et en mesurant la fluorescence du colorant orange SYPRO® toutes les minutes à l'aide d'un spectrofluorimètre Real-Time Cycler (Biorad). La fluorescence relative est déterminée en rapportant chaque valeur de fluorescence mesurée à celle de la valeur maximale de fluorescence du colorant orange SYPRO® pour chaque scan. Les résultats sont une moyenne de trois expériences identiques (triplicats).

B.4/ Evaluation de l'inhibition d'autres enzymes d'origine humaine

L'évaluation de la sélectivité des composés a été effectuée dans le laboratoire des Prof Herman S. Overkleeft et Johanes M. F. G. Aerts, Leiden Institute of Chemistry, Leiden University (NL). Les enzymes utilisées pour déterminer la sélectivité d'inhibition des composés vis-à-vis de différentes enzymes humaines sont l'α-glucosidase II du réticulum endoplasmique (GANAB), la β-glucocérébrosidase lysosomale humaine recombinante (GBA1), la β-glucosylceramidase non lysosomale humaine (GBA2) et la β-glucosylceramide synthase (GCS). L'activité inhibitrice des composés sur ces différentes enzymes est déterminée comme décrit dans la publication de Artola et al., 2017, ACS Cent. Sci. 3: 784-793. Sont utilisées l'enzyme GBA1 commercialisée sous la dénomination Cerezyme® et l'enzyme rhGAA commercialisée sous la dénomination Myozyme®. L'enzyme GANAB humaine utilisée pour cette étude est celle de fibroblastes de patients de la maladie de Pompe diagnostiqués comme dépourvus de GAA active et donneurs volontaires. Ces fibroblastes ont été cultivés sur un milieu HAMF12-DMEM (Sigma) supplémenté par 10% (v/v) de FCS (sérum de veau foetal). L'enzyme GBA2 a été surexprimée dans des cellules HEK298T cultivées sur un milieu DMEM enrichi en glucose (Gibco) supplémenté par 10% de NBS (sérum de nouveau-né bovin) et 100 unités/mL de pénicilline/streptomycine (Gibco) et 5% de $CO_2$ à 37 °C. L'activité des composés sur la β-glucosylceramide synthase humaine (GCS) est évaluée *in situ* sur des cellules de type RAW 264.7 cultivées sur un milieu RPMI (Gibco) supplémenté par 10% de FCS, 1 mM de glutamax, 100 unités/mL de penicilline/streptomycine (Gibco) et 5% de $CO_2$ à 37 °C.

Les valeurs d'$IC_{50}$ pour les enzymes GANAB, GBA2 et GCS sont déterminées à partir de lysats cellulaires préparés dans un tampon (20 mM hepes, 2 mM DTT, 0,25 M sucrose, 1 mM $MgCl_2$, 2,5 U/mL benzonase) à pH 7,0, et placés sur de la glace pendant 30 min. Ces lysats cellulaires sont homogénéisés à l'aide d'un broyeur SilentCrusher (Heidolph®), puis soumis à une ultracentrifugation à 32 000 tr/min pendant 30 min à 4 °C. La concentration totale en protéines est déterminée selon la méthode de Bradford (Bradford, 1976, Anal. Biochem. 72: 248-254), à l'aide d'un kit BioRad Quick Start® Bradford (Pierce) et de BSA (Sigma). Les lysats sont ensuite aliquotés et stockés à -80 °C avant utilisation. Les valeurs d'$IC_{50}$ pour l'enzyme GANAB sont déterminées sur des lysats cellulaires de fibroblastes de patients atteints de la maladie de Pompe, en utilisant comme tampon le McIlvaine 150 mM, pH 7,0, 0,1% de d'albumine de sérum bovin (BSA) (p/v), une concentration en substrat (4-méthyl-umbelliferone-α-D-glucopyranoside) de 2,4 mM et un temps d'incubation de 2 h.

Les valeurs d'$IC_{50}$ pour l'enzyme GBA1 sont déterminées en utilisant comme tampon le McIlvaine 150 mM, pH 5,2, 0,2% taurocholate (p/v), 0,1% Triton X-100 (v/v), 0,1% de d'albumine de sérum bovin (BSA) (p/v), une concentration en enzyme de 0,7 nM, une concentration en substrat (4-méthyl-umbelliferone-β-D-glucopyranoside) de 3,0 mM et un temps d'incubation de 30 min.

L'activité résiduelle de la GBA2 en présence des composés est déterminée après une pré-incubation pendant 30 min des homogénats de cellules HEK298T surexprimant GBA2 avec un inhibiteur de la GBA1, le conduritol β-époxyde (Sigma), à une concentration de 1 mM. Les valeurs d'$IC_{50}$ sont déterminées sur ces lysats cellulaires, en utilisant comme tampon le McIlvaine 150 mM, pH 5,8, 0,1% d'albumine de sérum bovin (BSA) (p/v), une concentration en substrat (4-méthyl-umbelliferone-β-D-glucopyranoside) de 3,0 mM et un temps d'incubation de 1 h.

L'activité résiduelle de la GCS en présence des composés est déterminée après une pré-incubation pendant 1 h des homogénats de cellules RAW 264.7 avec un inhibiteur de la GBA1, le conduritol β-époxyde (Sigma), à une concentration de 300 μM. Les valeurs d'$IC_{50}$ pour l'enzyme GCS sont déterminées in situ dans le milieu de culture cellulaire à pH 7,0 en utilisant 1 μM de NBD-ceramide (N-[12-[(7-nitro-2-1,3-benzoxadiazol-4-yl)amino]dodecanoyl]-D-erythro-sphingosine) comme substrat, comme décrit dans la publication de Lahav et al. précitée.

Les résultats obtenus pour l'ensemble de ces tests sont regroupés dans les tableaux 1 et 2 ci-dessous. Dans ces tableaux, à titre comparatif, sont également indiquées les valeurs obtenues pour les composés de l'art antérieur DNJ et NB-DNJ, telles que décrites dans la littérature. Dans ces tableaux : [a] indique Porto et al., 2012, Mol. Ther. 20: 2201-2211 ; [b] indique Bruckmann et al., 2012, ChemMedChem 7: 1943-1953 ; [c] indique Flanagan et al., 2009, Human Mut. 30: 1683-1692 ; [d] indique D'Alonzo et al., 2017, J. Med. Chem. 60: 9462-9469 ; * indique ΔTm mesurée à [composé] = 10 fois la valeur de $K_i$ ; [e] indique Asano et al., 1995, J. Med. Chem. 38: 2349-56 ; [f] indique Wennekes et al., 2010, J. Med. Chem. 53: 689-698.

Tableau 1

| Composé | rhGAA (Fluopol-ABPP) $IC_{50}$ (μM) | rhGAA (activité résiduelle) $IC_{50}$ (μM) | rhGAA ΔTm à pH 4,0 (°C) | rhGAA ΔTm à pH 7,4 (°C) |
|---|---|---|---|---|
| (IIa) | 0,42 | 341 | 8,1 | 12,7 |
| (IIb) | 0,39 | 104<br>(Ki = 13 μM) | 7,8 | 11,5 |
| (IIc) | 0,22 | 37,2<br>($K_i$ = 7,6 μM) | 11,8 | 13,0 |
| (IId) | 0,15 | 182<br>($K_i$ =18,9 μM) | 9,4 | 10,7 |
| (IIf) | 0,87 | 297 | 6,4 | 11,8 |
| (IIg) | 0,46 | 387 | 6,7 | 14,4 |
| (IIh) | 9,7 | - | - | - |
| (IIj) | 15,2 | - | - | - |
| (IIk) | 11,6 | - | - | - |
| (IIm) | 45,0 | - | - | - |
| (IIn) | 24,6 | - | - | - |
| (IIx1) | - | 40,3 | - | 16,5 |

(suite)

| Composé | rhGAA (Fluopol-ABPP) $IC_{50}$ (μM) | rhGAA (activité résiduelle) $IC_{50}$ (μM) | rhGAA ΔTm à pH 4,0 (°C) | rhGAA ΔTm à pH 7,4 (°C) |
|---|---|---|---|---|
| (IIb7) | - | 31,6 | - | 16,8 |
| (IIb3) | - | 29,6 ($K_i$ =1 0,8 μM) | - | 16,9 |
| (IIc1) | - | 32,9 | - | 15,0 |
| | | ($K_i$ =13,0 μM) | | |
| (IIr) | - | 29,5 ($K_i$ =20,0 μM) | - | 14,5 |
| (IIb1) | - | 49,1 ($K_i$ =6,4 μM) | - | 6,2 |
| DNJ | 0,41 | $K_i$= 3,4 μM[a] | 2,7 (pH 4,3)[*b] | 15[c] |
| NB-DNJ | - | $K_i$= 3,1 μM[b] | 6,2 (pH 4,3)[*b] | 12[d] |

Tableau 2

| Composé | GANAB $IC_{50}$ (μM) | GBA1 $IC_{50}$ (μM) | GBA2 $IC_{50}$ (μM) | GCS $IC_{50}$ (μM) |
|---|---|---|---|---|
| (IIa) | > 100 | > 1000 | > 1000 | > 50 |
| (IIb) | > 100 | > 1000 | > 1000 | > 50 |
| (IIc) | > 100 | > 1000 | > 1000 | > 50 |
| (IId) | > 100 | > 1000 | > 1000 | > 50 |
| (IIf) | > 100 | > 1000 | > 1000 | > 50 |
| (IIg) | > 100 | > 1000 | > 1000 | > 50 |
| DNJ | 4,6[e] | 250[f] | 21[f] | > 100[f] |
| NB-DNJ | 15[e] | 400[f] | 0,23[f] | 50[f] |

L'ensemble de ces résultats démontre une sélectivité des composés utilisés conformément à l'invention pour l'α-glucosidase acide humaine, par rapport aux autres enzymes humaines testées. Cette sélectivité est bien supérieure à celle des molécules proposées par l'art antérieur DNJ et NB-DNJ. Les composés utilisés conformément à l'invention présentent en outre un effet important de stabilisation de la rhGAA, comparable à celui de la DNJ et de la NB-DNJ.

En outre, les composés (IIb7), (IIb3), (IIc1), (IIr) et (IIc), répondant à la formule générale (I"a), ainsi que le composé (IIx1), présentent des performances supérieures à celles des autres composés selon l'invention.

## Revendications

**1.** Composé de formule générale (I), ou l'un de ses sels pharmaceutiquement acceptables, pour son utilisation en tant que médicament :

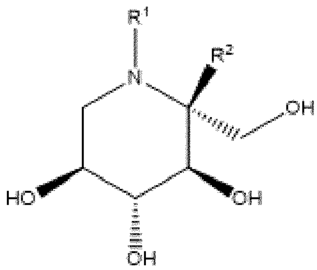

(I)

dans laquelle

- $R^1$ représente un atome d'hydrogène ou un radical hydrocarboné linéaire, ramifié et/ou cyclique, saturé ou insaturé, aromatique ou non, éventuellement substitué, pouvant comporter un ou plusieurs hétéroatomes et/ou un ou plusieurs groupements comportant au moins un hétéroatome et pouvant comporter un seul cycle ou plusieurs cycles le cas échéant fusionnés,
et $R^2$ représente un radical hydrocarboné linéaire, ramifié et/ou cyclique, saturé ou insaturé, aromatique ou non, éventuellement substitué, pouvant comporter un ou plusieurs hétéroatomes et/ou un ou plusieurs groupements comportant au moins un hétéroatome et pouvant comporter un seul cycle ou plusieurs cycles le cas échéant fusionnés, ledit radical hydrocarboné comportant au moins 2 atomes de carbone lorsque $R^1$ représente un atome d'hydrogène,
- ou $R^1$ et $R^2$ forment ensemble, avec les atomes du cycle pipéridine auquel chacun est rattaché, un hétérocycle de 3 à 6 chaînons fusionné avec le cycle pipéridine, éventuellement substitué par un ou plusieurs radicaux, pouvant être identiques ou différents, choisis chacun parmi un groupement hydroxyle, un groupement amino ou un radical carboné linéaire, ramifié et/ou cyclique, saturé ou insaturé, aromatique ou non, éventuellement substitué, pouvant comporter un ou plusieurs hétéroatomes et/ou un ou plusieurs groupements comportant au moins un hétéroatome et pouvant comporter un seul cycle ou plusieurs cycles le cas échéant fusionnés.

**2.** Composé pour son utilisation selon la revendication 1, selon lequel, dans la formule générale (I) :

$R^1$ représente un atome d'hydrogène ou un radical hydrocarboné linéaire, ramifié et/ou cyclique, saturé ou insaturé, aromatique ou non, éventuellement substitué, pouvant comporter un ou plusieurs hétéroatomes et/ou un ou plusieurs groupements comportant au moins un hétéroatome et pouvant comporter un seul cycle ou plusieurs cycles le cas échéant fusionnés,
et $R^2$ représente un groupement $-CH(R^3)-R^4$, où $R^3$ et $R^4$, pouvant être identiques ou différents, représentent chacun un atome d'hydrogène ou un radical hydrocarboné linéaire, ramifié et/ou cyclique, saturé ou insaturé, aromatique ou non, éventuellement substitué, pouvant comporter un ou plusieurs hétéroatomes et/ou un ou plusieurs groupements comportant au moins un hétéroatome et pouvant comporter un seul cycle ou plusieurs cycles le cas échéant fusionnés, $R^3$ et $R^4$ ne représentant pas simultanément un atome d'hydrogène lorsque $R^1$ représente un atome d'hydrogène.

**3.** Composé pour son utilisation selon la revendication 1 ou 2, selon lequel, dans la formule générale (I) :

$R^1$ représente un atome d'hydrogène ou un radical hydrocarboné linéaire, ramifié et/ou cyclique, saturé ou insaturé, aromatique ou non, éventuellement substitué, pouvant comporter un ou plusieurs hétéroatomes et/ou un ou plusieurs groupements comportant au moins un hétéroatome et pouvant comporter un seul cycle ou plusieurs cycles le cas échéant fusionnés,
et $R^2$ représente un groupement $-(CHR^7)-SO_2-Ar^1$ où $Ar^1$ représente un radical aryle ou hétéroaryle, le cas échéant substitué, et $R^7$ représente un atome d'hydrogène ou un radical hydrocarboné linéaire, ramifié et/ou cyclique, saturé ou insaturé, aromatique ou non, éventuellement substitué, pouvant comporter un ou plusieurs hétéroatomes et/ou un ou plusieurs groupements comportant au moins un hétéroatome et pouvant comporter un seul cycle ou plusieurs cycles le cas échéant fusionnés.

**4.** Composé pour son utilisation selon la revendication 1 ou 2, selon lequel, dans la formule générale (I) :

$R^1$ représente un atome d'hydrogène ou un radical hydrocarboné linéaire, ramifié et/ou cyclique, saturé ou insaturé, aromatique ou non, éventuellement substitué, pouvant comporter un ou plusieurs hétéroatomes et/ou

un ou plusieurs groupements comportant au moins un hétéroatome et pouvant comporter un seul cycle ou plusieurs cycles le cas échéant fusionnés,
et $R^2$ représente un groupement triazole, le cas échéant substitué.

**5.** Composé pour son utilisation selon la revendication 1 ou 2, selon lequel, dans la formule (I) :

$R^1$ représente un atome d'hydrogène ou un radical hydrocarboné linéaire, ramifié et/ou cyclique, saturé ou insaturé, aromatique ou non, éventuellement substitué, pouvant comporter un ou plusieurs hétéroatomes et/ou un ou plusieurs groupements comportant au moins un hétéroatome et pouvant comporter un seul cycle ou plusieurs cycles le cas échéant fusionnés,
et $R^2$ représente un groupement $-(CH_2)_2-R^8$, où $R^8$ représente :

- un atome d'hydrogène ;
- un groupement alkyle ou cycloalkyle en C1-C12, éventuellement substitué, pouvant comporter un seul cycle ou plusieurs cycles fusionnés ;
- un groupement $-(CH_2)_a-OH$ où a est en nombre entier compris entre 0 et 18 ;
- un groupement - $(CH_2)_b-Ar^2$ où $Ar^2$ représente un radical aryle ou hétéroaryle éventuellement substitué, pouvant comporter un ou plusieurs hétéroatomes et/ou un ou plusieurs groupements comportant au moins un hétéroatome et pouvant comporter un seul cycle ou plusieurs cycles le cas échéant fusionnés et b est en nombre entier compris entre 0 et 18 ;
- un groupement $-(CH_2)_c-Si(R^9)_3$ où $R^9$ représente un radical hydroxyle, un radical alkyle en C1-C4, un radical alcoxyle en C1-C4 ou un radical phényle et c est un nombre entier compris entre 0 et 18 ;
- ou un groupement $-(CH_2)_d-Z-R^{10}$ où Z est un hétéroatome choisi parmi l'oxygène, l'azote et le soufre, $R^{10}$ représente un atome d'hydrogène ou un radical alkyle, cycloalkyle, alkylaryle, aryle ou acyle en C1-C18, ledit radical étant éventuellement interrompu et/ou substitué par un ou plusieurs hétéroatomes et/ou un ou plusieurs groupements comportant au moins un hétéroatome, et d est en nombre entier compris entre 0 et 18.

**6.** Composé pour son utilisation selon l'une quelconque des revendications 1 à 5, selon lequel, dans la formule générale (I), $R^1$ représente un groupe alkyle linéaire, ramifié et/ou cyclique en C1-C18, de préférence en C1-C6, éventuellement interrompu et/ou substitué par un ou plusieurs hétéroatomes et/ou un ou plusieurs groupements comportant au moins un hétéroatome.

**7.** Composé pour son utilisation selon la revendication 1 ou 2, de formule générale (I'a) :

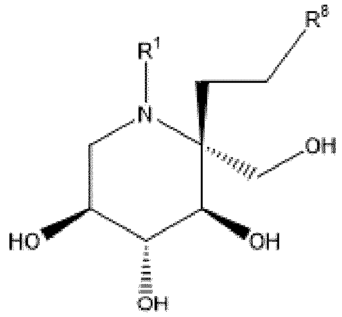

(I'a)

dans laquelle:

$R^1$ représente un atome d'hydrogène ou un groupe alkyle linéaire, ramifié et/ou cyclique en C1-C18, éventuellement interrompu et/ou substitué par un ou plusieurs hétéroatomes et/ou un ou plusieurs groupements comportant au moins un hétéroatome,
$R^8$ représente : un atome d'hydrogène ; un radical méthyle, éthyle, propyle, butyle, pentyle, hexyle, cycloalkyle, adamantyle, alkylcycloalkyle, alkylaryle ou aryle, ledit radical étant éventuellement interrompu et/ou substitué par un ou plusieurs hétéroatomes et/ou un ou plusieurs groupements comportant au moins un hétéroatome ; un groupement hydroxyle ; un groupement $-Si(R^{12})_3$ où $R^{12}$ représente un radical hydroxyle, un radical alkyle en C1-C4, un radical alcoxyle en C1-C4 ou un radical phényle ; un groupement $-(CH_2)_f-Y-R^{13}$ où f est un nombre entier compris entre 0 et 6, Y est un hétéroatome choisi parmi l'oxygène, l'azote et le soufre et $R^{13}$ représente

un radical alkyle en C1-C18 ou un radical aryle ou hétéroaryle en C1-C18 ; un groupement $-(CH_2)_g-CO-R^{13}$ où g est un nombre entier compris entre 0 et 6 ; ou un groupement $-(CH_2)_h-SO_e-R^{13}$ où h est un nombre entier compris entre 0 et 6 et e est égal à 1 ou 2,
$R^8$ ne représentant pas un atome d'hydrogène lorsque $R^1$ représente un radical propyle.

**8.** Composé pour son utilisation selon la revendication 1 ou 2, de formule générale (I"a) :

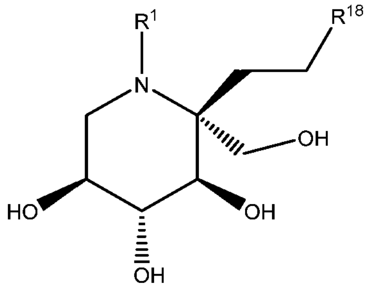

(I"a)

dans laquelle:

$R^1$ représente un atome d'hydrogène ou un groupe alkyle linéaire, ramifié et/ou cyclique en C1-C18, éventuellement interrompu et/ou substitué par un ou plusieurs hétéroatomes et/ou un ou plusieurs groupements comportant au moins un hétéroatome,
$R^{18}$ représente un radical hydrocarboné linéaire, ramifié et/ou cyclique, saturé ou insaturé, aromatique ou non, éventuellement substitué, comportant de 4 à 18 atomes de carbone, pouvant comporter un ou plusieurs hétéroatomes et/ou un ou plusieurs groupements comportant au moins un hétéroatome et pouvant comporter un seul cycle ou plusieurs cycles le cas échéant fusionnés.

**9.** Composé pour son utilisation selon l'une quelconque des revendications 1 à 8, pour le traitement de la maladie de Pompe.

**10.** Composé pour son utilisation selon la revendication 9, pour la stabilisation de l'$\alpha$-glucosidase acide humaine.

**11.** Composé pour son utilisation selon l'une quelconque des revendications 1 à 10, ledit composé étant contenu dans une composition de forme convenant à une administration par voie orale.

**12.** Composition pharmaceutique contenant un composé tel que défini dans l'une quelconque des revendications 1 à 8 dans un véhicule pharmaceutiquement acceptable.

**13.** Composition pharmaceutique selon la revendication 12, sous une forme convenant à une administration par voie orale.

**14.** Composé de formule générale (I') :

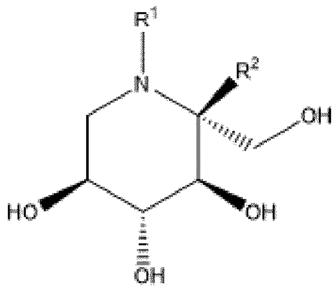

(I')

dans laquelle

- $R^1$ représente un atome d'hydrogène ou un radical hydrocarboné linéaire, ramifié et/ou cyclique, saturé ou insaturé, aromatique ou non, éventuellement substitué, pouvant comporter un ou plusieurs hétéroatomes et/ou un ou plusieurs groupements comportant au moins un hétéroatome et pouvant comporter un seul cycle ou plusieurs cycles le cas échéant fusionnés,

et $R^2$ représente un groupement -CH($R^3$)-$R^4$, où $R^3$ et $R^4$, pouvant être identiques ou différents, représentent chacun un atome d'hydrogène ou un radical hydrocarboné linéaire, ramifié et/ou cyclique, saturé ou insaturé, aromatique ou non, éventuellement substitué, pouvant comporter un ou plusieurs hétéroatomes et/ou un ou plusieurs groupements comportant au moins un hétéroatome et pouvant comporter un seul cycle ou plusieurs cycles le cas échéant fusionnés, $R^3$ et $R^4$ étant tels que, lorsque $R^1$ et $R^3$ représentent chacun un atome d'hydrogène, $R^4$ ne représente pas un atome d'hydrogène ou un radical phényle,
et $R^1$ et $R^2$ étant tels qu'ils ne représentent pas simultanément, respectivement, un radical propyle et un radical éthyle,

- ou $R^1$ et $R^2$ forment ensemble, avec les atomes du cycle pipéridine auquel chacun est rattaché, un hétérocycle à 4 chaînons fusionné avec le cycle pipéridine, éventuellement substitué par un ou plusieurs radicaux, pouvant être identiques ou différents, choisis chacun parmi un groupement hydroxyle, un groupement amino ou un radical carboné linéaire, ramifié et/ou cyclique, saturé ou insaturé, aromatique ou non, éventuellement substitué, pouvant comporter un ou plusieurs hétéroatomes et/ou un ou plusieurs groupements comportant au moins un hétéroatome et pouvant comporter un seul cycle ou plusieurs cycles le cas échéant fusionnés,
- ou $R^1$ et $R^2$ forment ensemble, avec les atomes du cycle pipéridine auquel chacun est rattaché, un hétérocycle à 3 chaînons fusionné avec le cycle pipéridine, éventuellement substitué par un groupement -X-$R^5$, dans lequel :

• X représente un radical -C(=O)- ou -CH($OR^6$)- où $R^6$ représente un radical hydrocarboné linéaire, ramifié et/ou cyclique, saturé ou insaturé, aromatique ou non, éventuellement substitué, pouvant comporter un ou plusieurs hétéroatomes et/ou un ou plusieurs groupements comportant au moins un hétéroatome et pouvant comporter un seul cycle ou plusieurs cycles le cas échéant fusionnés, et $R^5$ représente un atome d'hydrogène, un groupement amino ou un radical hydrocarboné linéaire, ramifié et/ou cyclique, saturé ou insaturé, aromatique ou non, éventuellement substitué, pouvant comporter un ou plusieurs hétéroatomes et/ou un ou plusieurs groupements comportant au moins un hétéroatome et pouvant comporter un seul cycle ou plusieurs cycles le cas échéant fusionnés,
• ou X représente un radical -CH(OH)- et $R^5$ représente un atome d'hydrogène ou un radical hydrocarboné linéaire, ramifié et/ou cyclique, saturé ou insaturé, aromatique ou non, éventuellement substitué, pouvant comporter un ou plusieurs hétéroatomes et/ou un ou plusieurs groupements comportant au moins un hétéroatome et pouvant comporter un seul cycle ou plusieurs cycles le cas échéant fusionnés, $R^5$ ne représentant pas un radical n-propyle, un radical cyclohexyle ou un radical phényle,

ou l'un de ses sels pharmaceutiquement acceptable.

**15.** Composé selon la revendication 14, selon lequel, dans la formule (I') :

$R^1$ représente un atome d'hydrogène ou un radical hydrocarboné linéaire, ramifié et/ou cyclique, saturé ou insaturé, aromatique ou non, éventuellement substitué, pouvant comporter un ou plusieurs hétéroatomes et/ou un ou plusieurs groupements comportant au moins un hétéroatome et pouvant comporter un seul cycle ou plusieurs cycles le cas échéant fusionnés,
et $R^2$ représente un groupement -($CHR^7$)-$SO_2$-$Ar^1$ où $Ar^1$ représente un radical aryle ou hétéroaryle, le cas échéant substitué, et $R^7$ représente un atome d'hydrogène ou un radical hydrocarboné linéaire, ramifié et/ou cyclique, saturé ou insaturé, aromatique ou non, éventuellement substitué, pouvant comporter un ou plusieurs hétéroatomes et/ou un ou plusieurs groupements comportant au moins un hétéroatome et pouvant comporter un seul cycle ou plusieurs cycles le cas échéant fusionnés.

**16.** Composé selon la revendication 14, selon lequel, dans la formule (I') :

$R^1$ représente un atome d'hydrogène ou un radical hydrocarboné linéaire, ramifié et/ou cyclique, saturé ou insaturé, aromatique ou non, éventuellement substitué, pouvant comporter un ou plusieurs hétéroatomes et/ou un ou plusieurs groupements comportant au moins un hétéroatome et pouvant comporter un seul cycle ou plusieurs cycles le cas échéant fusionnés,
et $R^2$ représente un groupement triazole, le cas échéant substitué.

**17.** Composé selon la revendication 14, selon lequel, dans la formule (I') :

$R^1$ représente un atome d'hydrogène ou un radical hydrocarboné linéaire, ramifié et/ou cyclique, saturé ou insaturé, aromatique ou non, éventuellement substitué, pouvant comporter un ou plusieurs hétéroatomes et/ou un ou plusieurs groupements comportant au moins un hétéroatome et pouvant comporter un seul cycle ou plusieurs cycles le cas échéant fusionnés,
et $R^2$ représente un groupement $-(CH_2)_2-R^8$, où $R^8$ représente :

- un atome d'hydrogène ;
- un groupement alkyle ou cycloalkyle en C1-C12, éventuellement substitué, pouvant comporter un seul cycle ou plusieurs cycles fusionnés ;
- un groupement $-(CH_2)_a-OH$ où a est en nombre entier compris entre 0 et 18 ;
- un groupement $-(CH_2)_b-Ar^2$ où $Ar^2$ représente un radical aryle ou hétéroaryle éventuellement substitué, pouvant comporter un ou plusieurs hétéroatomes et/ou un ou plusieurs groupements comportant au moins un hétéroatome et pouvant comporter un seul cycle ou plusieurs cycles le cas échéant fusionnés et

b est en nombre entier compris entre 0 et 18 ;

- un groupement $-(CH_2)_c-Si(R^9)_3$ où $R^9$ représente un radical hydroxyle, un radical alkyle en C1-C4, un radical alcoxyle en C1-C4 ou un radical phényle et c est un nombre entier compris entre 0 et 18 ;
- ou un groupement $-(CH_2)_d-Z-R^{10}$ où Z est un hétéroatome choisi parmi l'oxygène, l'azote et le soufre, $R^{10}$ représente un radical alkyle, cycloalkyle, alkylaryle, aryle ou acyle en C1-C18, ledit radical étant éventuellement interrompu et/ou substitué par un ou plusieurs hétéroatomes et/ou un ou plusieurs groupements comportant au moins un hétéroatome, et d est en nombre entier compris entre 0 et 18.

**18.** Composé selon la revendication 14, de formule générale (I'a) :

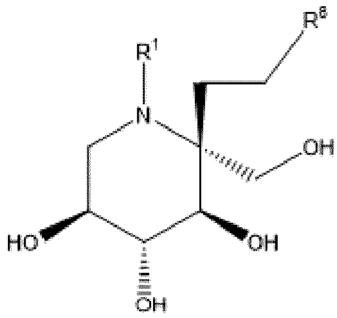

(I'a)

dans laquelle

$R^1$ représente un atome d'hydrogène ou un groupe alkyle linéaire, ramifié et/ou cyclique en C1-C18, éventuellement interrompu et/ou substitué par un ou plusieurs hétéroatomes et/ou un ou plusieurs groupements comportant au moins un hétéroatome,
$R^8$ représente : un atome d'hydrogène ; un groupement hydroxyle ; un radical méthyle, éthyle, propyle, butyle, pentyle, hexyle, cyclohexyle, adamantyle, alkylcycloalkyle, alkylaryle ou aryle, ledit radical étant éventuellement interrompu et/ou substitué par un ou plusieurs hétéroatomes et/ou un ou plusieurs groupements comportant au moins un hétéroatome ; un groupement $-Si(R^{12})_3$ où $R^{12}$ représente un radical hydroxyle, un radical alkyle en C1-C4, un radical alcoxyle en C1-C4 ou un radical phényle ; un groupement $-(CH_2)_f-Y-R^{13}$ où f est un nombre entier compris entre 0 et 6, Y est un hétéroatome choisi parmi l'oxygène, l'azote et le soufre et $R^{13}$ représente un radical alkyle en C1-C18 ou un radical aryle ou hétéroaryle en C1-C18 ; un groupement $-(CH_2)_g-CO-R^{13}$ où g est un nombre entier compris entre 0 et 6 ; ou un groupement $-(CH_2)_h-SO_e-R^{13}$ où h est un nombre entier compris entre 0 et 6 et e est égal à 1 ou 2,
$R^8$ ne représentant pas un atome d'hydrogène lorsque $R^1$ représente un radical propyle.

**19.** Composé selon la revendication 14, de formule générale (I"a) :

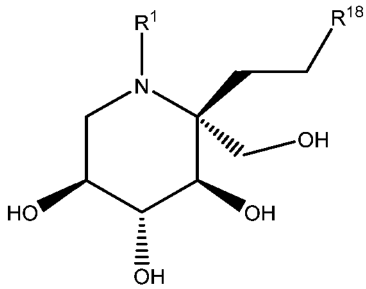

(I"a)

dans laquelle:

$R^1$ représente un atome d'hydrogène ou un groupe alkyle linéaire, ramifié et/ou cyclique en C1-C18, éventuellement interrompu et/ou substitué par un ou plusieurs hétéroatomes et/ou un ou plusieurs groupements comportant au moins un hétéroatome,
$R^{18}$ représente un radical hydrocarboné linéaire, ramifié et/ou cyclique, saturé ou insaturé, aromatique ou non, éventuellement substitué, comportant de 4 à 18 atomes de carbone, pouvant comporter un ou plusieurs hétéroatomes et/ou un ou plusieurs groupements comportant au moins un hétéroatome et pouvant comporter un seul cycle ou plusieurs cycles le cas échéant fusionnés.

**20.** Procédé de préparation d'un composé selon l'une des revendications 17 à 19, comprenant des étapes successives de :

a/ réaction d'un composé de formule générale (III) ou (IV) de formules :

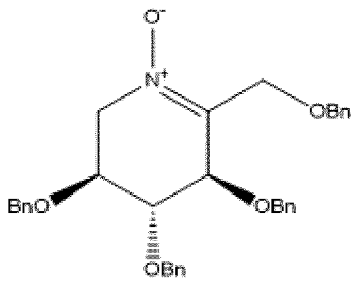

(III)

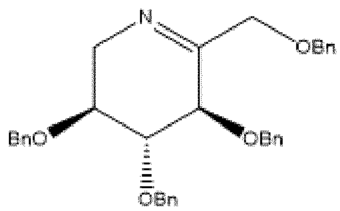

'(IV)

dans lesquelles Bn représente un reste benzyle,
avec un composé de formule générale (V) :

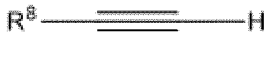

(V)

dans laquelle $R^8$ est tel que défini dans l'une des revendications 19 ou 20, $R^8$ ne représentant toutefois ni un atome d'hydrogène, ni un groupement hydroxyle, ni un groupement amino,
en présence d'un composé organométallique,
b/ le cas échéant, réduction de la fonction hydroxylamine en amine,
c/ le cas échéant, alkylation de l'atome d'azote du cycle pipéridine,

d/ et hydrogénolyse du produit obtenu à l'issue de l'étape a/, le cas échéant de l'étape b/ ou de l'étape c/.

**Patentansprüche**

1. Verbindung der allgemeinen Formel (I) oder eines der pharmazeutisch akzeptablen Salze davon zur Verwendung als Medikament:

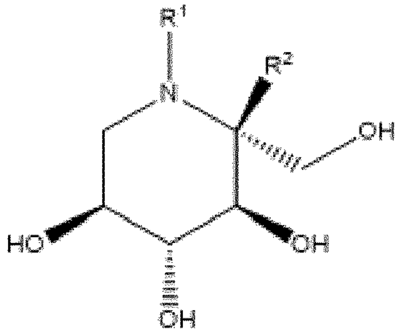

(I)

wobei

- $R^1$ für ein Wasserstoffatom oder einen geradkettigen, verzweigten und/oder cyclischen Kohlenwasserstoffrest steht, gesättigt oder ungesättigt, aromatisch oder nicht, gegebenenfalls substituiert, der ein oder mehrere Heteroatome und/oder eine oder mehrere Gruppen, die mindestens ein Heteroatom umfassen, und einen einzelnen Ring oder mehrere Ringe, die gegebenenfalls miteinander kondensiert sind, umfassen kann,
und $R^2$ für einen geradkettigen, verzweigten und/oder cyclischen Kohlenwasserstoffrest steht, gesättigt oder ungesättigt, aromatisch oder nicht, gegebenenfalls substituiert, der ein oder mehrere Heteroatome und/oder eine oder mehrere Gruppen, die mindestens ein Heteroatom umfassen, und einen einzelnen Ring oder mehrere Ringe, die gegebenenfalls miteinander kondensiert sind, umfassen kann, wobei der Kohlenwasserstoffrest mindestens 2 Kohlenstoffatome umfasst, wenn $R^1$ für ein Wasserstoffatom steht,
- oder $R^1$ und $R^2$ zusammen mit den Atomen des Piperidinrings, an den sie jeweils gebunden sind, einen 3- bis 6-gliedrigen Heterocyclus bilden, der mit dem Piperidinring kondensiert und gegebenenfalls durch einen oder mehrere Reste substituiert ist, die gleich oder verschieden sein können, jeweils ausgewählt aus einer Hydroxylgruppe, einer Aminogruppe oder einem geradkettigen, verzweigten und/oder cyclischen Kohlenwasserstoffrest, gesättigt oder ungesättigt, aromatisch oder nicht, gegebenenfalls substituiert, der ein oder mehrere Heteroatome und/oder eine oder mehrere Gruppen, die mindestens ein Heteroatom umfassen, und einen einzelnen Ring oder mehrere Ringe, die gegebenenfalls miteinander kondensiert sind, umfassen kann.

2. Verbindung zur Verwendung nach Anspruch 1, wobei in der allgemeinen Formel (I):

$R^1$ für ein Wasserstoffatom oder einen geradkettigen, verzweigten und/oder cyclischen Kohlenwasserstoffrest steht, gesättigt oder ungesättigt, aromatisch oder nicht, gegebenenfalls substituiert, der ein oder mehrere Heteroatome und/oder eine oder mehrere Gruppen, die mindestens ein Heteroatom umfassen, und einen einzelnen Ring oder mehrere Ringe, die gegebenenfalls miteinander kondensiert sind, umfassen kann,
und $R^2$ für eine Gruppe $-CH(R^3)-R^4$ steht, wobei $R^3$ und $R^4$ gleich oder verschieden sein können und jeweils für ein Wasserstoffatom oder einen geradkettigen, verzweigten und/oder cyclischen Kohlenwasserstoffrest steht, gesättigt oder ungesättigt, aromatisch oder nicht, gegebenenfalls substituiert, der ein oder mehrere Heteroatome und/oder eine oder mehrere Gruppen, die mindestens ein Heteroatom umfassen, und einen einzelnen Ring oder mehrere Ringe, die gegebenenfalls miteinander kondensiert sind, umfassen kann, wobei $R^3$ und $R^4$ nicht gleichzeitig für ein Wasserstoffatom stehen, wenn $R^1$ für ein Wasserstoffatom steht.

3. Verbindung zur Verwendung nach Anspruch 1 oder 2, wobei in der allgemeinen Formel (I):

$R^1$ für ein Wasserstoffatom oder einen geradkettigen, verzweigten und/oder cyclischen Kohlenwasserstoffrest steht, gesättigt oder ungesättigt, aromatisch oder nicht, gegebenenfalls substituiert, der ein oder mehrere Heteroatome und/oder eine oder mehrere Gruppen, die mindestens ein Heteroatom umfassen, und einen einzelnen Ring oder mehrere Ringe, die gegebenenfalls miteinander kondensiert sind, umfassen kann, und einen einzelnen

Ring oder mehrere Ringe, die gegebenenfalls miteinander kondensiert sind, umfassen kann, und $R^2$ für eine Gruppe $-(CHR^7)-SO_2-Ar^1$ steht, wobei $Ar^1$ für einen gegebenenfalls substituierten Aryl- oder Heteroarylrest steht und $R^7$ für ein Wasserstoffatom oder einen geradkettigen, verzweigten und/oder cyclischen Kohlenwasserstoffrest steht, gesättigt oder ungesättigt, aromatisch oder nicht, gegebenenfalls substituiert, der ein oder mehrere Heteroatome und/oder eine oder mehrere Gruppen, die mindestens ein Heteroatom umfassen, und einen einzelnen Ring oder mehrere Ringe, die gegebenenfalls miteinander kondensiert sind, umfassen kann.

**4.** Verbindung zur Verwendung nach Anspruch 1 oder 2, wobei in der allgemeinen Formel (I):

$R^1$ für ein Wasserstoffatom oder einen geradkettigen, verzweigten und/oder cyclischen Kohlenwasserstoffrest steht, gesättigt oder ungesättigt, aromatisch oder nicht, gegebenenfalls substituiert, der ein oder mehrere Heteroatome und/oder eine oder mehrere Gruppen, die mindestens ein Heteroatom umfassen, und einen einzelnen Ring oder mehrere Ringe, die gegebenenfalls miteinander kondensiert sind, umfassen kann, und $R^2$ für eine gegebenenfalls substituierte Triazolgruppe steht.

**5.** Verbindung zur Verwendung nach Anspruch 1 oder 2, wobei in der Formel (I):

$R^1$ für ein Wasserstoffatom oder einen geradkettigen, verzweigten und/oder cyclischen Kohlenwasserstoffrest steht, gesättigt oder ungesättigt, aromatisch oder nicht, gegebenenfalls substituiert, der ein oder mehrere Heteroatome und/oder eine oder mehrere Gruppen, die mindestens ein Heteroatom umfassen, und einen einzelnen Ring oder mehrere Ringe, die gegebenenfalls miteinander kondensiert sind, umfassen kann, und $R^2$ für eine Gruppe $-(CH_2)_2-R^8$ steht, wobei $R^8$ für Folgendes steht:

- ein Wasserstoffatom;
- eine gegebenenfalls substituierte C1-C12-Alkyl- oder - Cycloalkylgruppe, die einen einzelnen Ring oder mehrere Ringe, die gegebenenfalls miteinander kondensiert sind, umfassen kann;
- eine $-(CH_2)_a-OH$-Gruppe, wobei a eine ganze Zahl zwischen 0 und 18 ist;
- eine $-(CH_2)_b-Ar^2$-Gruppe, wobei $Ar^2$ für einen gegebenenfalls substituierten Aryl- oder Heteroarylrest steht, der ein oder mehrere Heteroatome und/oder eine oder mehrere Gruppen, die mindestens ein Heteroatom umfassen, und einen einzelnen Ring oder eine mehrere Ringe, die gegebenenfalls miteinander kondensiert sind, umfassen kann, und b eine ganze Zahl zwischen 0 und 18 ist;
- eine $-(CH_2)_c-Si(R^9)_3$-Gruppe, wobei $R^9$ für einen Hydroxylrest, einen C1-C4-Alkylrest, einen C1-C4-Alkoxylrest oder einen Phenylrest steht und c eine ganze Zahl zwischen 0 und 18 ist;
- oder eine $-(CH_2)_d-Z-R^{10}$-Gruppe, wobei Z ein Heteroatom ist, ausgewählt aus Sauerstoff, Stickstoff und Schwefel, $R^{10}$ für ein Wasserstoffatom oder einen C1-C18-Alkyl-, - Cycloalkyl-, -Alkylaryl-, -Aryl- oder -Acylrest steht, wobei der Rest gegebenenfalls durch ein oder mehrere Heteroatome und/oder eine oder mehrere Gruppen, die mindestens ein Heteroatom umfassen, unterbrochen und/oder substituiert ist, und d eine ganze Zahl zwischen 0 und 18 ist.

**6.** Verbindung zur Verwendung nach einem der Ansprüche 1 bis 5, wobei in der allgemeinen Formel (I) $R^1$ für eine geradkettige, verzweigte und/oder cyclische C1-C18-Alkylgruppe, vorzugsweise C1-C6-Alkylgruppe, steht, die gegebenenfalls durch ein oder mehrere Heteroatome und/oder eine oder mehrere Gruppen, die mindestens ein Heteroatom umfassen, unterbrochen und/oder substituiert ist.

**7.** Verbindung zur Verwendung nach Anspruch 1 oder 2 der allgemeinen Formel (I'a):

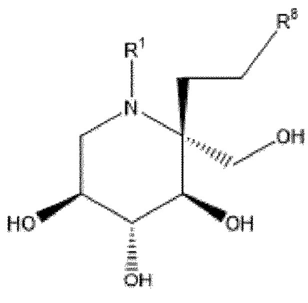

(I'a)

wobei:

$R^1$ für ein Wasserstoffatom oder eine geradkettige, verzweigte und/oder cyclische C1-C18-Alkylgruppe steht, die gegebenenfalls durch ein oder mehrere Heteroatome und/oder eine oder mehrere Gruppen, die mindestens ein Heteroatom umfassen, unterbrochen und/oder substituiert ist,
$R^8$ für Folgendes steht: ein Wasserstoffatom; einen Methyl-, Ethyl-, Propyl-, Butyl-, Pentyl-, Hexyl-, Cycloalkyl-, Adamantyl-, Alkylcycloalkyl-, Alkylaryl- oder Arylrest, wobei der Rest gegebenenfalls durch ein oder mehrere Heteroatome und/oder eine oder mehrere Gruppen, die mindestens ein Heteroatom umfassen, unterbrochen und/oder substituiert ist; eine Hydroxylgruppe; eine -$Si(R^{12})_3$-Gruppe, wobei $R^{12}$ für einen Hydroxylrest, einen C1-C4-Alkylrest, einen C1-C4-Alkoxylrest oder einen Phenylrest steht; eine -$(CH_2)_f$-Y-$R^{13}$-Gruppe, wobei f eine ganze Zahl zwischen 0 und 6 ist, Y ein Heteroatom ist, ausgewählt aus Sauerstoff, Stickstoff und Schwefel, und $R^{13}$ für einen C1-C18-Alkylrest oder einen C1-C18-Aryl- oder -Heteroarylrest steht; eine -$(CH_2)_g$-CO-$R^{13}$-Gruppe, wobei g eine ganze Zahl zwischen 0 und 6 ist; oder eine -$(CH_2)_h$-$SO_e$-$R^{13}$-Gruppe, wobei h eine ganze Zahl zwischen 0 und 6 ist und e gleich 1 oder 2 ist,
$R^8$ nicht für ein Wasserstoffatom steht, wenn $R^1$ für einen Propylrest steht.

8. Verbindung zur Verwendung nach Anspruch 1 oder 2 der allgemeinen Formel (I"a):

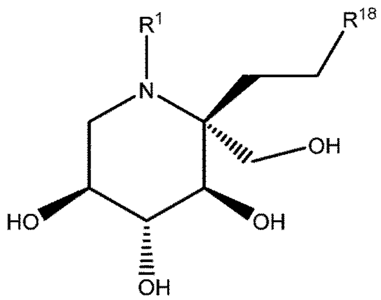

(I"a)

wobei:

$R^1$ für ein Wasserstoffatom oder eine geradkettige, verzweigte und/oder cyclische C1-C18-Alkylgruppe steht, die gegebenenfalls durch ein oder mehrere Heteroatome und/oder eine oder mehrere Gruppen, die mindestens ein Heteroatom umfassen, unterbrochen und/oder substituiert ist,
$R^{18}$ für einen geradkettigen, verzweigten und/oder cyclischen Kohlenwasserstoffrest steht, gesättigt oder ungesättigt, aromatisch oder nicht, gegebenenfalls substituiert, mit 4 bis 18 Kohlenstoffatomen, der ein oder mehrere Heteroatome und/oder eine oder mehrere Gruppen, die mindestens ein Heteroatom umfassen, und einen einzelnen Ring oder mehrere Ringe, die gegebenenfalls miteinander kondensiert sind, umfassen kann.

9. Verbindung zur Verwendung nach einem der Ansprüche 1 bis 8 zur Behandlung von Morbus Pompe.

10. Verbindung zur Verwendung nach Anspruch 9 zur Stabilisierung menschlicher saurer $\alpha$-Glucosidase.

11. Verbindung zur Verwendung nach einem der Ansprüche 1 bis 10, wobei die Verbindung in einer Zusammensetzung in einer Form enthalten ist, die für die orale Verabreichung geeignet ist.

12. Pharmazeutische Zusammensetzung, die eine wie in einem der Ansprüche 1 bis 8 definierte Verbindung in einem pharmazeutisch akzeptablen Träger enthält.

13. Pharmazeutische Zusammensetzung nach Anspruch 12 in einer Form, die für die orale Verabreichung geeignet ist.

14. Verbindung der allgemeinen Formel (I'):

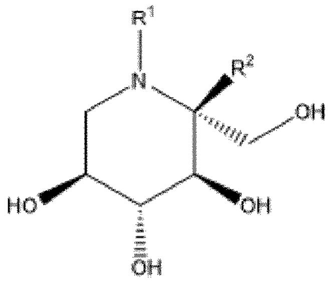

(I')

wobei

- $R^1$ für ein Wasserstoffatom oder einen geradkettigen, verzweigten und/oder cyclischen Kohlenwasserstoffrest steht, gesättigt oder ungesättigt, aromatisch oder nicht, gegebenenfalls substituiert, der ein oder mehrere Heteroatome und/oder eine oder mehrere Gruppen, die mindestens ein Heteroatom umfassen, und einen einzelnen Ring oder mehrere Ringe, die gegebenenfalls miteinander kondensiert sind, umfassen kann,

und $R^2$ für eine Gruppe -CH($R^3$)-$R^4$ steht, wobei $R^3$ und $R^4$ gleich oder verschieden sein können und jeweils für ein Wasserstoffatom oder einen geradkettigen, verzweigten und/oder cyclischen Kohlenwasserstoffrest steht, gesättigt oder ungesättigt, aromatisch oder nicht, gegebenenfalls substituiert, der ein oder mehrere Heteroatome und/oder eine oder mehrere Gruppen, die mindestens ein Heteroatom umfassen, und einen einzelnen Ring oder mehrere Ringe, die gegebenenfalls miteinander kondensiert sind, umfassen kann, wobei $R^3$ und $R^4$ derart sind, dass, wenn $R^1$ und $R^3$ jeweils für ein Wasserstoffatom stehen, $R^4$ nicht für ein Wasserstoffatom oder einen Phenylrest steht,
und $R^1$ und $R^2$ derart sind, dass sie nicht gleichzeitig für einen Propylrest bzw. einen Etyhlrest stehen,

- oder $R^1$ und $R^2$ zusammen mit den Atomen des Piperidinrings, an den sie jeweils gebunden sind, einen 4-gliedrigen Heterocyclus bilden, der mit dem Piperidinring kondensiert und gegebenenfalls durch einen oder mehrere Reste substituiert ist, die gleich oder verschieden sein können, jeweils ausgewählt aus einer Hydroxylgruppe, einer Aminogruppe oder einem geradkettigen, verzweigten und/oder cyclischen Kohlenwasserstoffrest, gesättigt oder ungesättigt, aromatisch oder nicht, gegebenenfalls substituiert, der ein oder mehrere Heteroatome und/oder eine oder mehrere Gruppen, die mindestens ein Heteroatom umfassen, und der einen einzelnen Ring oder mehrere Ringe, die gegebenenfalls miteinander kondensiert sind, umfassen kann,
- oder $R^1$ und $R^2$ zusammen mit den Atomen des Piperidinrings, an den sie jeweils gebunden sind, einen 3-gliedrigen Heterocyclus bilden, der mit dem Piperidinring kondensiert und gegebenenfalls durch eine -X-$R^5$-Gruppe substituiert ist, in der:

  - X für einen -C(=O)- oder -CH($OR^6$)-Rest steht, wobei $R^6$ für einen geradkettigen, verzweigten und/oder cyclischen Kohlenwasserstoffrest steht, gesättigt oder ungesättigt, aromatisch oder nicht, gegebenenfalls substituiert, der ein oder mehrere Heteroatome und/oder eine oder mehrere Gruppen, die mindestens ein Heteroatom umfassen, und einen einzelnen Ring oder mehrere Ringe, die gegebenenfalls miteinander kondensiert sind, umfassen kann, und $R^5$ für ein Wasserstoffatom, eine Aminogruppe oder einen geradkettigen, verzweigten und/oder cyclischen Kohlenwasserstoffrest steht, gesättigt oder ungesättigt, aromatisch oder nicht, gegebenenfalls substituiert, der ein oder mehrere Heteroatome und/oder eine oder mehrere Gruppen, die mindestens ein Heteroatom umfassen, und einen einzelnen Ring oder mehrere Ringe, die gegebenenfalls miteinander kondensiert sind, umfassen kann,
  - oder X für einen -CH(OH)-Rest steht und $R^5$ für ein Wasserstoffatom oder einen geradkettigen, verzweigten und/oder cyclischen Kohlenwasserstoffrest steht, gesättigt oder ungesättigt, aromatisch oder nicht, gegebenenfalls substituiert, der ein oder mehrere Heteroatome und/oder eine oder mehrere Gruppen, die mindestens ein Heteroatom umfassen, und einen einzelnen Ring oder mehrere Ringe, die gegebenenfalls miteinander kondensiert sind, umfassen kann, wobei $R^5$ nicht für einen n-Propylrest, einen Cyclohexylrest oder einen Phenylrest steht,

  oder eines der pharmazeutisch akzeptablen Salze davon.

**15.** Verbindung nach Anspruch 14, wobei in der Formel (I'):

$R^1$ für ein Wasserstoffatom oder einen geradkettigen, verzweigten und/oder cyclischen Kohlenwasserstoffrest steht, gesättigt oder ungesättigt, aromatisch oder nicht, gegebenenfalls substituiert, der ein oder mehrere Heteroatome und/oder eine oder mehrere Gruppen, die mindestens ein Heteroatom umfassen, und einen einzelnen Ring oder mehrere Ringe, die gegebenenfalls miteinander kondensiert sind, umfassen kann,
und $R^2$ für eine Gruppe $-(CHR^7)-SO_2-Ar^1$ steht, wobei $Ar^1$ für einen gegebenenfalls substituierten Aryl- oder Heteroarylrest steht und $R^7$ für ein Wasserstoffatom oder einen geradkettigen, verzweigten und/oder cyclischen Kohlenwasserstoffrest steht, gesättigt oder ungesättigt, aromatisch oder nicht, gegebenenfalls substituiert, der ein oder mehrere Heteroatome und/oder eine oder mehrere Gruppen, die mindestens ein Heteroatom umfassen, und einen einzelnen Ring oder mehrere Ringe, die gegebenenfalls miteinander kondensiert sind, umfassen kann.

**16.** Verbindung nach Anspruch 14, wobei in der allgemeinen Formel (I'):

$R^1$ für ein Wasserstoffatom oder einen geradkettigen, verzweigten und/oder cyclischen Kohlenwasserstoffrest steht, gesättigt oder ungesättigt, aromatisch oder nicht, gegebenenfalls substituiert, der ein oder mehrere Heteroatome und/oder eine oder mehrere Gruppen, die mindestens ein Heteroatom umfassen, und einen einzelnen Ring oder mehrere Ringe, die gegebenenfalls miteinander kondensiert sind, umfassen kann,
und $R^2$ für eine gegebenenfalls substituierte Triazolgruppe steht.

**17.** Verbindung nach Anspruch 14, wobei in der Formel (I'):

$R^1$ für ein Wasserstoffatom oder einen geradkettigen, verzweigten und/oder cyclischen Kohlenwasserstoffrest steht, gesättigt oder ungesättigt, aromatisch oder nicht, gegebenenfalls substituiert, der ein oder mehrere Heteroatome und/oder eine oder mehrere Gruppen, die mindestens ein Heteroatom umfassen, und einen einzelnen Ring oder mehrere Ringe, die gegebenenfalls miteinander kondensiert sind, umfassen kann,
und $R^2$ für eine Gruppe $-(CH_2)_2-R^8$ steht, wobei $R^8$ für Folgendes steht:

- ein Wasserstoffatom;
- eine gegebenenfalls substituierte C1-C12-Alkyl- oder - Cycloalkylgruppe, die einen einzelnen Ring oder mehrere Ringe, die miteinander kondensiert sind, umfassen kann;
- eine $-(CH_2)_a-OH$-Gruppe, wobei a eine ganze Zahl zwischen 0 und 18 ist;
- eine $-(CH_2)_b-Ar^2$-Gruppe, wobei $Ar^2$ für einen gegebenenfalls substituierten Aryl- oder Heteroarylrest steht, der ein oder mehrere Heteroatome und/oder eine oder mehrere Gruppen, die mindestens ein Heteroatom umfassen, und einen einzelnen Ring oder eine mehrere Ringe, die gegebenenfalls miteinander kondensiert sind, umfassen kann, und b eine ganze Zahl zwischen 0 und 18 ist;
- eine $-(CH_2)_c-Si(R^9)_3$-Gruppe, wobei $R^9$ für einen Hydroxylrest, einen einen C1-C4-Alkoxylrest oder einen Phenylrest steht und c eine ganze Zahl zwischen 0 und 18 ist;
- oder eine $-(CH_2)_d-Z-R^{10}$-Gruppe, wobei Z ein Heteroatom ist, ausgewählt aus Sauerstoff, Stickstoff und Schwefel, $R^{10}$ für ein Wasserstoffatom oder einen C1-C18-Alkyl-, - Cycloalkyl-, -Alkylaryl-, -Aryl- oder -Acylrest steht, wobei der Rest gegebenenfalls durch ein oder mehrere Heteroatome und/oder eine oder mehrere Gruppen, die mindestens ein Heteroatom umfassen, unterbrochen und/oder substituiert ist, und d eine ganze Zahl zwischen 0 und 18 ist.

**18.** Verbindung nach Anspruch 14 der allgemeinen Formel (I'a):

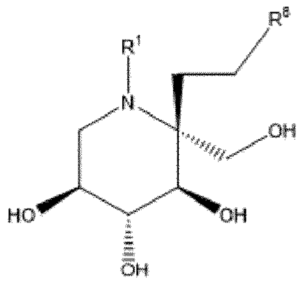

(I'a)

wobei

$R^1$ für ein Wasserstoffatom oder eine geradkettige, verzweigte und/oder cyclische C1-C18-Alkylgruppe steht, die gegebenenfalls durch ein oder mehrere Heteroatome und/oder eine oder mehrere Gruppen, die mindestens ein Heteroatom umfassen, unterbrochen und/oder substituiert ist,

$R^8$ für Folgendes steht: ein Wasserstoffatom; einen Methyl-, Ethyl-, Propyl-, Butyl-, Pentyl-, Hexyl-, Cyclohexyl-, Adamantyl-, Alkylcycloalkyl-, Alkylaryl- oder Arylrest, wobei der Rest gegebenenfalls durch ein oder mehrere Heteroatome und/oder eine oder mehrere Gruppen, die mindestens ein Heteroatom umfassen, unterbrochen und/oder substituiert ist; eine $-Si(R^{12})_3$-Gruppe, wobei $R^{12}$ für einen Hydroxylrest, einen einen C1-C4-Alkoxylrest oder einen Phenylrest steht; eine $-(CH_2)_f$-Y-$R^{13}$-Gruppe, wobei f eine ganze Zahl zwischen 0 und 6 ist, Y ein Heteroatom ist, ausgewählt aus Sauerstoff, Stickstoff und Schwefel, und $R^{13}$ für einen C1-C18-Alkylrest oder einen C1-C18-Aryl- oder -Heteroarylrest steht; eine $-(CH_2)_g$-CO-$R^{13}$-Gruppe, wobei g eine ganze Zahl zwischen 0 und 6 ist; oder eine $-(CH_2)_h-SO_e-R^{13}$-Gruppe, wobei h eine ganze Zahl zwischen 0 und 6 ist und e gleich 1 oder 2 ist, $R^8$ nicht für ein Wasserstoffatom steht, wenn $R^1$ für einen Propylrest steht.

**19.** Verbindung nach Anspruch 14 der allgemeinen Formel (I"a):

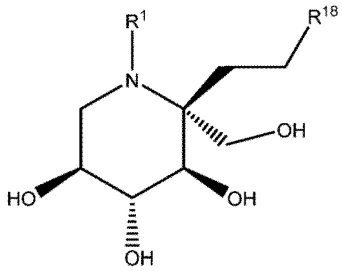

(I"a)

wobei:

$R^1$ für ein Wasserstoffatom oder eine geradkettige, verzweigte und/oder cyclische C1-C18-Alkylgruppe steht, die gegebenenfalls durch ein oder mehrere Heteroatome und/oder eine oder mehrere Gruppen, die mindestens ein Heteroatom umfassen, unterbrochen und/oder substituiert ist,

$R^{18}$ für einen geradkettigen, verzweigten und/oder cyclischen Kohlenwasserstoffrest steht, gesättigt oder ungesättigt, aromatisch oder nicht, gegebenenfalls substituiert, mit 4 bis 18 Kohlenstoffatomen, der ein oder mehrere Heteroatome und/oder eine oder mehrere Gruppen, die mindestens ein Heteroatom umfassen, und einen einzelnen Ring oder mehrere Ringe, die gegebenenfalls miteinander kondensiert sind, umfassen kann.

**20.** Verfahren zum Herstellen einer Verbindung nach einem der Ansprüche 17 bis 19, umfassend die aufeinanderfolgenden Schritte:

a/ Umsetzen einer Verbindung der allgemeinen Formel (III) oder (IV) der Formeln:

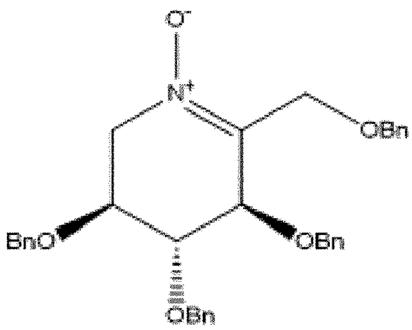

(III)

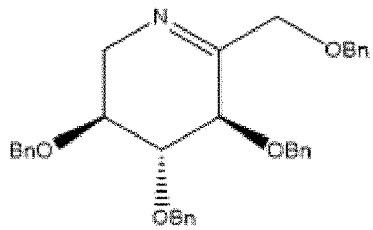

(IV)

wobei Bn für einen Benzylrest steht,
mit einer Verbindung der allgemeinen Formel (V):

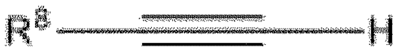

(V)

wobei $R^8$ derart wie in einem der Ansprüche 19 oder 20 definiert ist, $R^8$ jedoch weder für ein Wasserstoffatom noch für eine Hydroxylgruppe oder eine Aminogruppe steht,
in Gegenwart einer metallorganischen Verbindung,
b/ gegebenenfalls Reduzieren der Hydroxylaminfunktion zu einem Amin,
c/ gegebenenfalls Alkylieren des Stickstoffatoms des Piperidinrings,
d/ und Hydrogenolysieren des am Ende von Schritt a/, gegebenenfalls von Schritt b/ oder von Schritt c/, erhaltenen Produkts.

## Claims

**1.** Compound of general formula (I), or one of the pharmaceutically acceptable salts thereof, for use as a drug:

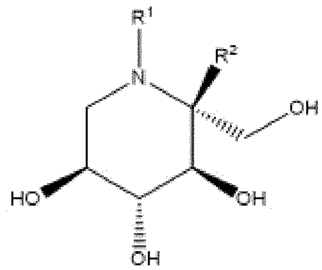

(I)

wherein

- $R^1$ represents a hydrogen atom or a linear, branched and/or cyclic hydrocarbon radical, saturated or unsaturated, aromatic or not, optionally substituted, optionally comprising one or more heteroatoms and/or one or more groups including at least one heteroatom and optionally comprising a single ring or a plurality of rings optionally fused,
and $R^2$ represents a linear, branched and/or cyclic hydrocarbon radical, saturated or unsaturated, aromatic or not, optionally substituted, optionally comprising one or more heteroatoms and/or one or more groups including at least one heteroatom and optionally comprising a single ring or a plurality of rings optionally fused, said hydrocarbon radical comprising at least 2 carbon atoms when $R^1$ represents a hydrogen atom,
- or $R^1$ and $R^2$ form together, with the atoms of the piperidine ring to which each is attached, a 3- to 6-membered heterocycle fused with the piperidine ring, optionally substituted by one or several radicals, which may be identical or different, each selected from a hydroxyl group, an amino group or a linear, branched and/or cyclic carbon radical, saturated or unsaturated, aromatic or not, optionally substituted, optionally comprising one or more heteroatoms and/or one or more groups including at least one heteroatom and optionally comprising a single ring or a plurality of rings optionally fused.

**2.** Compound for use according to claim 1, wherein, in the general formula (I):

$R^1$ represents a hydrogen atom or a linear, branched and/or cyclic hydrocarbon radical, saturated or unsaturated, aromatic or not, optionally substituted, optionally comprising one or more heteroatoms and/or one or more groups including at least one heteroatom and optionally comprising a single ring or a plurality of rings optionally fused,
and $R^2$ represents a $-CH(R^3)-R^4$ group, wherein $R^3$ and $R^4$, identical or different, each represent a hydrogen atom or a linear, branched and/or cyclic hydrocarbon radical, saturated or unsaturated, aromatic or not, optionally substituted, optionally comprising one or more heteroatoms and/or one or more groups including at least one heteroatom and optionally comprising a single ring or a plurality of rings optionally fused, $R^3$ and $R^4$ not simultaneously representing a hydrogen atom when $R^1$ represents a hydrogen atom.

**3.** Compound for use according to claim 1 or 2, wherein, in the general formula (I):

$R^1$ represents a hydrogen atom or a linear, branched and/or cyclic hydrocarbon radical, saturated or unsaturated, aromatic or not, optionally substituted, optionally comprising one or more heteroatoms and/or one or more groups including at least one heteroatom and optionally comprising a single ring or a plurality of rings optionally fused,
and $R^2$ represents a $-(CHR^7)-SO_2-Ar^1$ group wherein $Ar^1$ represents an aryl or heteroaryl radical, optionally substituted, and $R^7$ represents a hydrogen atom or a linear, branched and/or cyclic hydrocarbon radical, saturated or unsaturated, aromatic or not, optionally substituted, optionally comprising one or more heteroatoms and/or one or more groups including at least one heteroatom and optionally comprising a single ring or a plurality of rings optionally fused.

**4.** Compound for use according to claim 1 or 2, wherein, in the general formula (I):

$R^1$ represents a hydrogen atom or a linear, branched and/or cyclic hydrocarbon radical, saturated or unsaturated, aromatic or not, optionally substituted, optionally comprising one or more heteroatoms and/or one or more groups including at least one heteroatom and optionally comprising a single ring or a plurality of rings optionally fused,
and $R^2$ represents a triazole group, optionally substituted.

**5.** Compound for use according to claim 1 or 2, wherein, in the formula (I):

$R^1$ represents a hydrogen atom or a linear, branched and/or cyclic hydrocarbon radical, saturated or unsaturated, aromatic or not, optionally substituted, optionally comprising one or more heteroatoms and/or one or more groups including at least one heteroatom and optionally comprising a single ring or a plurality of rings optionally fused,
and $R^2$ represents a $-(CH_2)_2-R^8$ group, wherein $R^8$ represents:

- a hydrogen atom;
- a C1-C12 alkyl or cycloalkyl group, optionally substituted, optionally comprising a single ring or a plurality of fused rings;
- a $-(CH_2)_a-OH$ group wherein a is an integer between 0 and 18;
- a $-(CH_2)_b-Ar^2$ group wherein $Ar^2$ represents an aryl or heteroaryl radical, optionally substituted, optionally comprising one or more heteroatoms and/or one or more groups including at least one heteroatom and optionally comprising a single ring or a plurality of rings optionally fused and b is an integer between 0 and 18;
- a $-(CH_2)_c-Si(R^9)_3$ group wherein $R^9$ represents a hydroxyl radical, a C1-C4 alkyl radical, a C1-C4 alkoxyl radical or a phenyl radical and c is an integer between 0 and 18;
- or a $-(CH_2)_d-Z-R^{10}$ group wherein Z is a heteroatom selected from oxygen, nitrogen and sulfur, $R^{10}$ represents a hydrogen atom or a C1-C18 alkyl, cycloalkyl, alkylaryl, aryl or acyl radical, said radical optionally being interrupted and/or substituted by one or more heteroatoms and/or one or more groups including at least one heteroatom, and d is an integer between 0 and 18.

**6.** Compound for use according to any one of claims 1 to 5, wherein, in the general formula (I), $R^1$ represents a C1-C18, preferably C1-C6, linear, branched and/or cyclic alkyl group, optionally interrupted and/or substituted by one or more heteroatoms and one or more groups including at least one heteroatom.

**7.** Compound for use according to claim 1 or 2, of general formula (I'a):

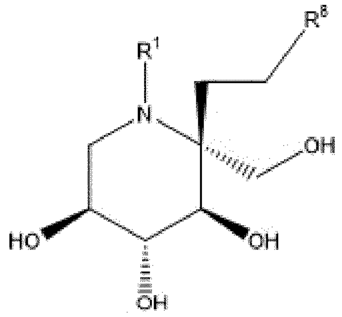

(I'a)

wherein:

$R^1$ represents a hydrogen atom or C1-C18 linear, branched and/or cyclic alkyl group, optionally interrupted and/or substituted by one or more heteroatoms and/or one or more groups including at least one heteroatom,
$R^8$ represents: a hydrogen atom; a methyl, ethyl, propyl, butyl, pentyl, hexyl, cycloalkyl, adamantyl, alkylcycloalkyl, alkylaryl or aryl radical, said radical optionally being interrupted and/or substituted by one or more heteroatoms and/or one or more groups including at least one heteroatom; a hydroxyl group; a $-Si(R^{12})_3$ group wherein $R^{12}$ represents a hydroxyl radical, a C1-C4 alkyl radical, a C1-C4 alkoxyl radical or a phenyl radical; a $-(CH_2)_f-Y-R^{13}$ group wherein f is an integer between 0 and 6, Y is a heteroatom selected from oxygen, nitrogen and sulfur and $R^{13}$ represents a C1-C18 alkyl radical or a C1-C18 aryl or heteroaryl radical; a $-(CH_2)_g-CO-R^{13}$ group wherein g is an integer between 0 and 6; or a $-(CH_2)_h-SO_e-R^{13}$ group wherein h is an integer between 0 and 6 and e is equal to 1 or 2,
$R^8$ not representing a hydrogen atom when $R^1$ represents a propyl radical.

**8.** Compound for use according to claim 1 or 2, of general formula (I"a):

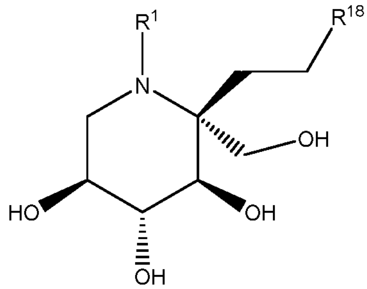

(I''a)

wherein:

$R^1$ represents a hydrogen atom or a C1-C18 linear, branched and/or cyclic alkyl group, optionally interrupted and/or substituted by one or more heteroatoms and/or one or more groups including at least one heteroatom,
$R^{18}$ represents a linear, branched and/or cyclic hydrocarbon radical, saturated or unsaturated, aromatic or not, optionally substituted, comprising 4 to 18 carbon atoms, optionally comprising one or more heteroatoms and/or one or more groups including at least one heteroatom and optionally comprising a single ring or a plurality of rings optionally fused.

**9.** Compound for use according to any one of claims 1 to 8, for treating Pompe disease.

**10.** Compound for use according to claim 9, for stabilizing human acid α-glucosidase.

**11.** Compound for use according to any one of claims 1 to 10, said compound being contained in a composition having a form suitable for oral administration.

**12.** Pharmaceutical composition containing a compound as defined in any one of claims 1 to 8 in a pharmaceutically acceptable vehicle.

**13.** Pharmaceutical composition according to claim 12, in a form suitable for oral administration.

**14.** Compound of general formula (I'):

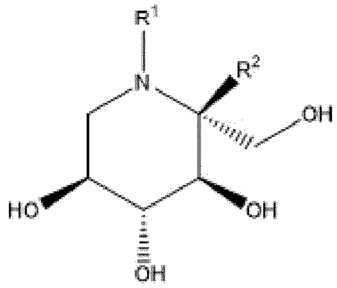

(I')

wherein

- $R^1$ represents a hydrogen atom or a linear, branched and/or cyclic hydrocarbon radical, saturated or unsaturated, aromatic or not, optionally substituted, optionally comprising one or more heteroatoms and/or one or more groups including at least one heteroatom and optionally comprising a single ring or a plurality of rings optionally fused,

  and $R^2$ represents a -CH($R^3$)-$R^4$ group, wherein $R^3$ and $R^4$, identical or different, each represent a hydrogen atom or a linear, branched and/or cyclic hydrocarbon radical, saturated or unsaturated, aromatic or not, optionally substituted, optionally comprising one or more heteroatoms and/or one or more groups including at least one heteroatom and optionally comprising a single ring or a plurality of rings optionally fused, $R^3$ and $R^4$ being such that, when $R^1$ and $R^3$ each represent a hydrogen atom, $R^4$ does not represent a hydrogen atom or a phenyl radical,
  and $R^1$ and $R^2$ being such that they do not simultaneously represent, respectively, a propyl radical and an ethyl radical,

- or $R^1$ and $R^2$ form together, with the atoms of the piperidine ring to which each is attached, a 4-membered heterocycle fused with the piperidine ring, optionally substituted by one or more radicals, which may be identical or different, each selected from a hydroxyl group, an amino group or a linear, branched and/or cyclic carbon radical, saturated or unsaturated, aromatic or not, optionally substituted, optionally comprising one or more heteroatoms and/or one or more groups including at least one heteroatom and optionally comprising a single ring or a plurality of rings optionally fused,
- or $R^1$ and $R^2$ form together, with the atoms of the piperidine ring to which each is attached, a 3-membered heterocycle fused with the piperidine ring, optionally substituted by a -X-$R^5$ group, wherein:

  • X represents a -C(=O)- or -CH($OR^6$)- radical wherein $R^6$ represents a linear, branched and/or cyclic hydrocarbon radical, saturated or unsaturated, aromatic or not, optionally substituted, optionally comprising one or more heteroatoms and/or one or more groups including at least one heteroatom and optionally comprising a single ring or a plurality of rings optionally fused, and $R^5$ represents a hydrogen atom, an amino group or a linear, branched and/or cyclic hydrocarbon radical, saturated or unsaturated, aromatic or not, optionally substituted, optionally comprising one or more heteroatoms and/or one or more groups including at least one heteroatom and optionally comprising a single ring or a plurality of rings optionally fused;
  • or X represents a -CH(OH)- radical and $R^5$ represents a hydrogen atom or a linear, branched and/or cyclic hydrocarbon radical, saturated or unsaturated, aromatic or not, optionally substituted, optionally comprising one or more heteroatoms and/or one or more groups including at least one heteroatom and optionally comprising a single ring or a plurality of rings optionally fused, $R^5$ not representing an n-propyl radical, a cyclohexyl radical or a phenyl radical,

or one of the pharmaceutically acceptable salts thereof.

**15.** Compound according to claim 14, wherein, in the formula (I'):

$R^1$ represents a hydrogen atom or a linear, branched and/or cyclic hydrocarbon radical, saturated or unsaturated, aromatic or not, optionally substituted, optionally comprising one or more heteroatoms and/or one or more groups including at least one heteroatom and optionally comprising a single ring or a plurality of rings optionally fused,
and $R^2$ represents a $-(CHR^7)-SO_2-Ar^1$ group wherein $Ar^1$ represents an aryl or heteroaryl radical, optionally substituted, and $R^7$ represents a hydrogen atom or a linear, branched and/or cyclic hydrocarbon radical, saturated or unsaturated, aromatic or not, optionally substituted, optionally comprising one or more heteroatoms and/or one or more groups including at least one heteroatom and optionally comprising a single ring or a plurality of rings optionally fused.

**16.** Compound according to claim 14, wherein, in the formula (I'):

$R^1$ represents a hydrogen atom or a linear, branched and/or cyclic hydrocarbon radical, saturated or unsaturated, aromatic or not, optionally substituted, optionally comprising one or more heteroatoms and/or one or more groups including at least one heteroatom and optionally comprising a single ring or a plurality of rings optionally fused,
and $R^2$ represents a triazole group, optionally substituted.

**17.** Compound according to claim 14, wherein, in the formula (I'):

$R^1$ represents a hydrogen atom or a linear, branched and/or cyclic hydrocarbon radical, saturated or unsaturated, aromatic or not, optionally substituted, optionally comprising one or more heteroatoms and/or one or more groups including at least one heteroatom and optionally comprising a single ring or a plurality of rings optionally fused,
and $R^2$ represents a $-(CH_2)_2-R^8$ group, wherein $R^8$ represents:

- a hydrogen atom;
- a C1-C12 alkyl or cycloalkyl group, optionally substituted, optionally comprising a single ring or a plurality of fused rings;
- a $-(CH_2)_a-OH$ group wherein a is an integer between 0 and 18;
- a $-(CH_2)_b-Ar^2$ group wherein $Ar^2$ represents an aryl or heteroaryl radical, optionally substituted, optionally comprising one or more heteroatoms and/or one or more groups including at least one heteroatom and optionally comprising a single ring or a plurality of rings optionally fused and b is an integer between 0 and 18;
- a $-(CH_2)_c-Si(R^9)_3$ group wherein $R^9$ represents a hydroxyl radical, a C1-C4 alkyl radical, a C1-C4 alkoxyl radical or a phenyl radical and c is an integer between 0 and 18,
- or a $-(CH_2)_d-Z-R^{10}$ group wherein Z is a heteroatom selected from oxygen, nitrogen and sulfur, $R^{10}$ represents a C1-C18 alkyl, cycloalkyl, alkylaryl, aryl or acyl radical, said radical optionally being interrupted and/or substituted by one or more heteroatoms and/or one or more groups including at least one heteroatom, and d is an integer between 0 and 18.

**18.** Compound according to claim 14, of general formula (I'a):

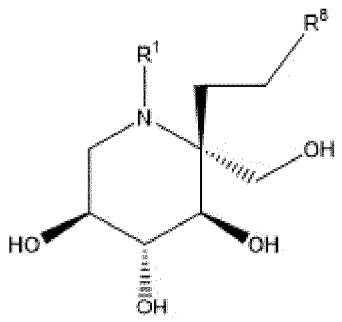

(I'a)

wherein

$R^1$ represents a hydrogen atom or a C1-C18 linear, branched and/or cyclic alkyl group, optionally interrupted and/or substituted by one or more heteroatoms and/or one or more groups including at least one heteroatom, $R^8$ represents: a hydrogen atom; a hydroxyl group; a methyl, ethyl, propyl, butyl, pentyl, hexyl, cyclohexyl, adamantyl, alkylcycloalkyl, alkylaryl or aryl radical, said radical optionally being interrupted and/or substituted by one or more heteroatoms and/or one or more groups including at least one heteroatom; a - $Si(R^{12})_3$ group wherein $R^{12}$ represents a hydroxyl radical, a C1-C4 alkyl radical, a C1-C4 alkoxyl radical or a phenyl radical; a $-(CH_2)_f$-Y-$R^{13}$ group wherein f is an integer between 0 and 6, Y is a heteroatom selected from oxygen, nitrogen and sulfur and $R^{13}$ represents a C1-C18 alkyl radical or a C1-C18 aryl or heteroaryl radical; a $-(CH_2)_g$-CO-$R^{13}$ group wherein g is an integer between 0 and 6; or a - $(CH_2)_h$-$SO_e$-$R^{13}$ group wherein h is an integer between 0 and 6 and e is equal to 1 or 2,

$R^8$ not representing a hydrogen atom when $R^1$ represents a propyl radical.

**19.** Compound according to claim 14, of general formula (I"a):

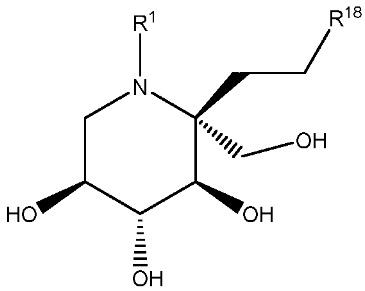

(I"a)

wherein:

$R^1$ represents a hydrogen atom or a C1-C18 linear, branched and/or cyclic alkyl group, optionally interrupted and/or substituted by one or more heteroatoms and/or one or more groups including at least one heteroatom, $R^{18}$ represents a linear, branched and/or cyclic hydrocarbon radical, saturated or unsaturated, aromatic or not, optionally substituted, comprising from 4 to 18 carbon atoms, optionally comprising one or more heteroatoms and/or one or more groups including at least one heteroatom and optionally comprising a single ring or a plurality of rings optionally fused.

**20.** Method for preparing a compound according to one of claims 17 to 19, comprising successive steps of:

a/ reacting a compound of general formula (III) or (IV) of formulae:

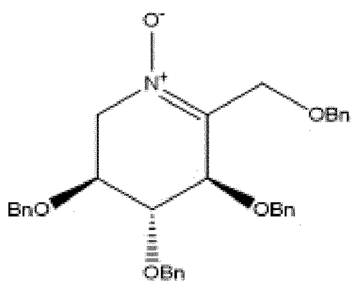

(III)

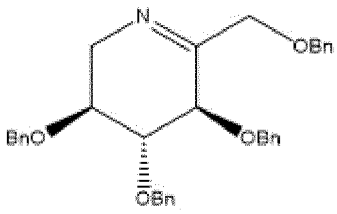

(IV)

wherein Bn represents a benzyl radical,
with a compound of general formula (V):

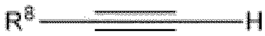

(V)

wherein $R^8$ is as defined in one of claims 19 or 20, $R^8$ however representing neither a hydrogen atom, nor a hydroxyl group, nor an amino group,
in the presence of an organometallic compound,
b/ optionally, reduction of the hydroxylamine function into amine,
c/ optionally, alkylation of the nitrogen atom of the piperidine ring,
d/ and hydrogenolysis of the product obtained at the end of step a/, where applicable of step b/ or of step c/.

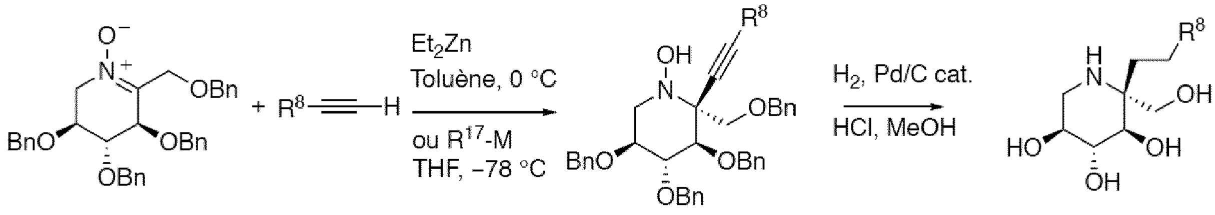


**III** **V** **A-1** **I'a** ($R^1$ = H)
**IIc** ($R^8$ = Ph)

FIG 1

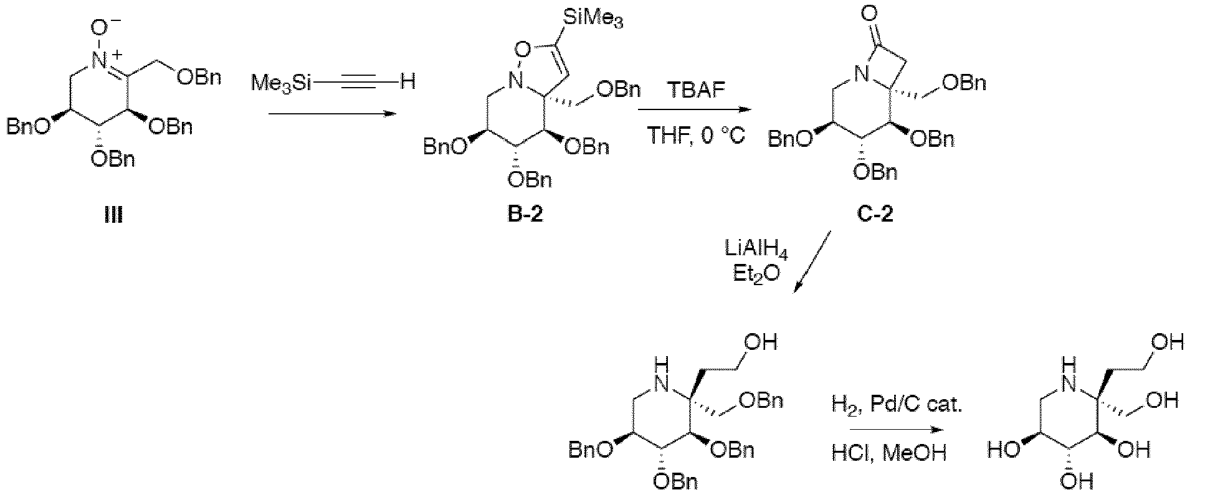


**III** **B-2** **C-2**

**D-2** **IId**
(**I'a** avec $R^1$ = H, $R^8$ = OH)

FIG 2

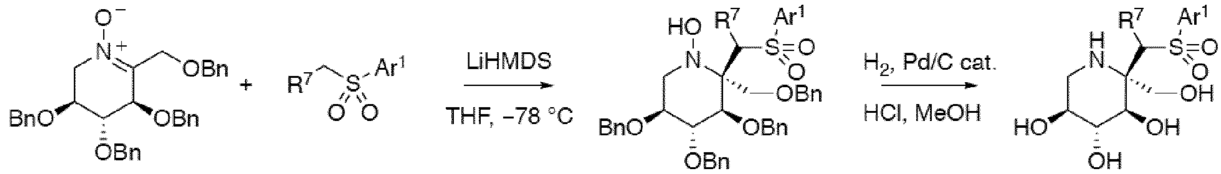


**III** **VI** **A-3** **I'd** ($R^1$ = H )

FIG 3

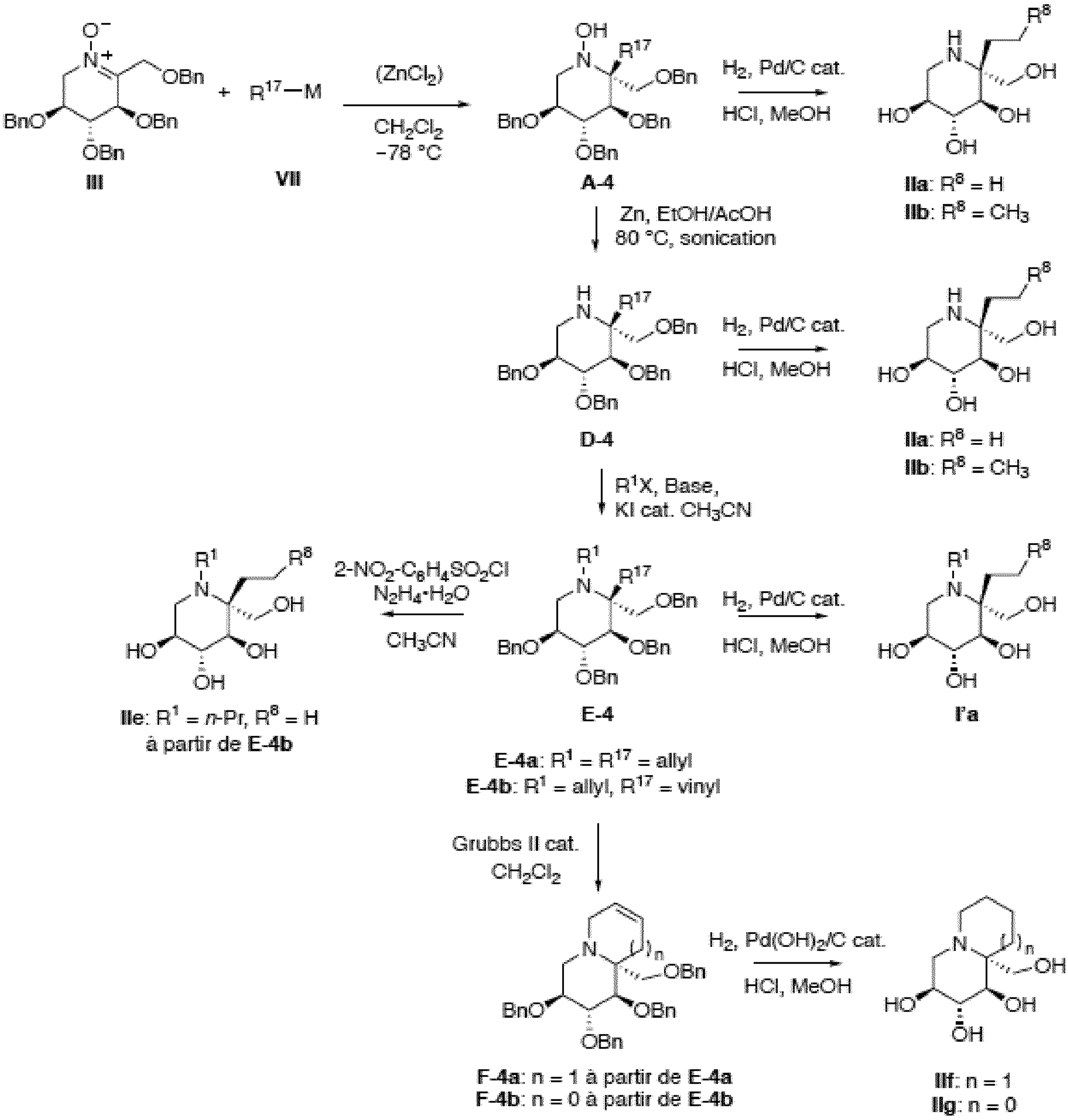

III
VII
(ZnCl2)
CH2Cl2
−78 °C
A-4
H2, Pd/C cat.
HCl, MeOH
IIa: R8 = H
IIb: R8 = CH3
Zn, EtOH/AcOH
80 °C, sonication
D-4
H2, Pd/C cat.
HCl, MeOH
IIa: R8 = H
IIb: R8 = CH3
R1X, Base,
KI cat. CH3CN
2-NO2-C6H4SO2Cl
N2H4·H2O
CH3CN
E-4
H2, Pd/C cat.
HCl, MeOH
IIe: R1 = n-Pr, R8 = H
à partir de E-4b
I'a
E-4a: R1 = R17 = allyl
E-4b: R1 = allyl, R17 = vinyl
Grubbs II cat.
CH2Cl2
H2, Pd(OH)2/C cat.
HCl, MeOH
F-4a: n = 1 à partir de E-4a
F-4b: n = 0 à partir de E-4b
IIf: n = 1
IIg: n = 0


FIG 4

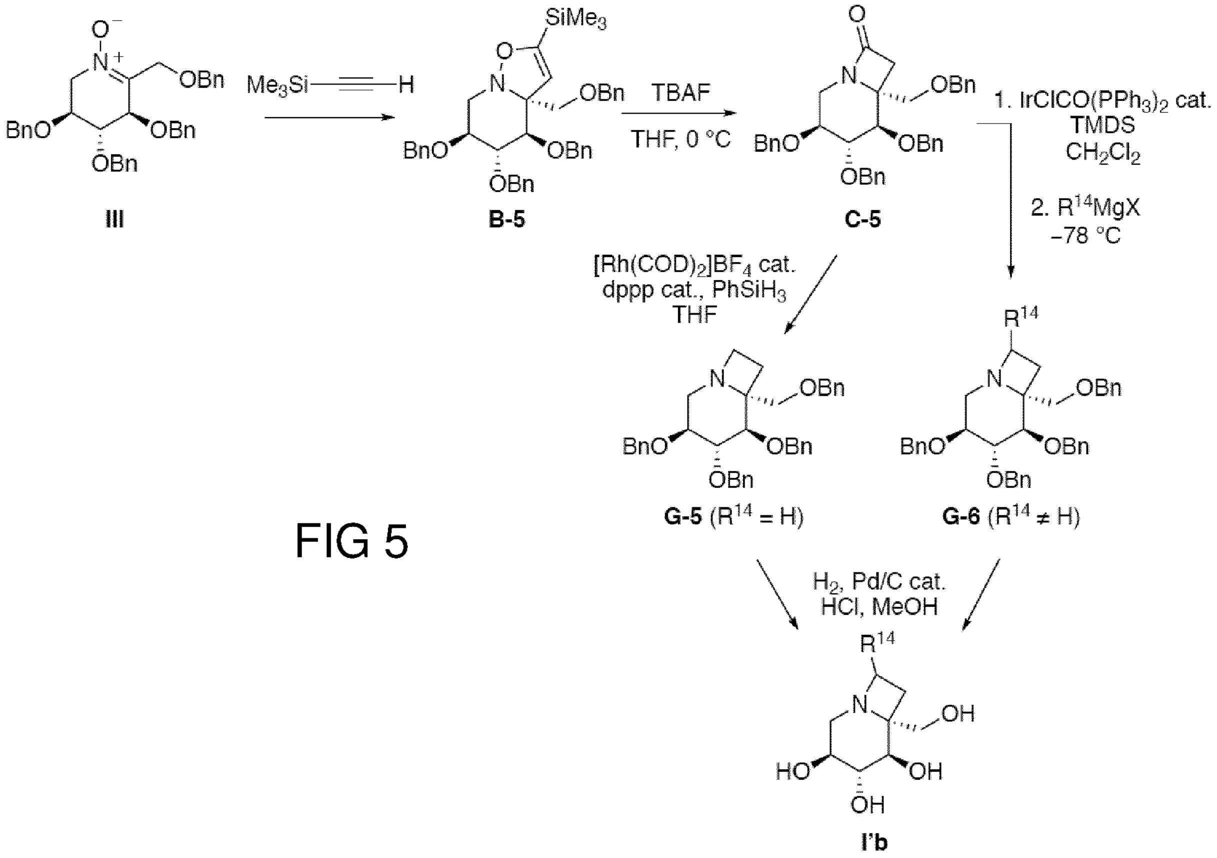
Me3Si—≡—H
III
B-5
TBAF
THF, 0 °C
C-5
1. IrClCO(PPh3)2 cat.
TMDS
CH2Cl2
2. R14MgX
−78 °C
[Rh(COD)2]BF4 cat.
dppp cat., PhSiH3
THF
G-5 (R14 = H)
G-6 (R14 ≠ H)
H2, Pd/C cat.
HCl, MeOH
I'b

FIG 5

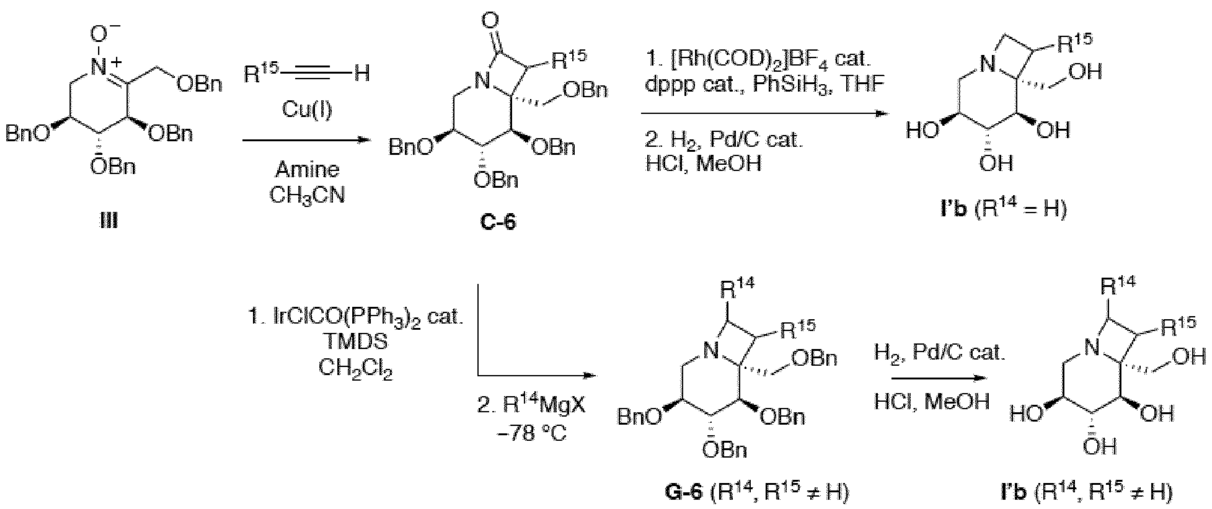
R15—≡—H
Cu(I)
Amine
CH3CN
III
C-6
1. [Rh(COD)2]BF4 cat.
dppp cat., PhSiH3, THF
2. H2, Pd/C cat.
HCl, MeOH
I'b (R14 = H)
1. IrClCO(PPh3)2 cat.
TMDS
CH2Cl2
2. R14MgX
−78 °C
G-6 (R14, R15 ≠ H)
H2, Pd/C cat.
HCl, MeOH
I'b (R14, R15 ≠ H)

FIG 6

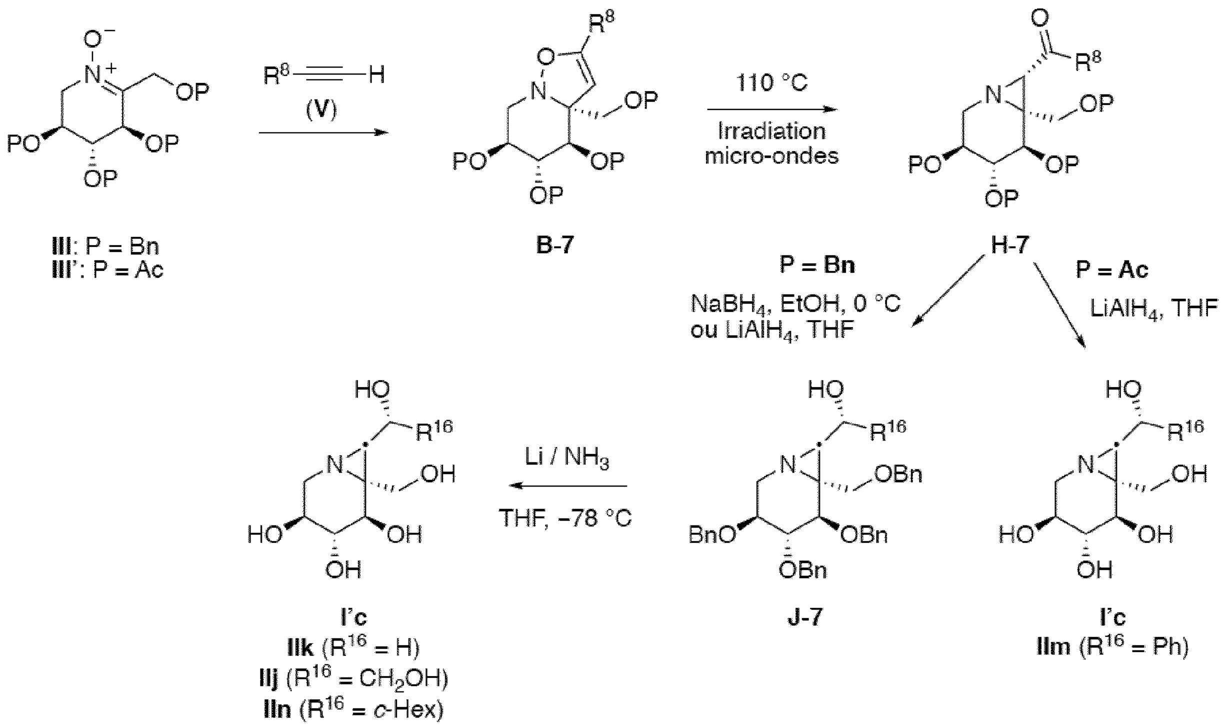


**III**: P = Bn
**III'**: P = Ac
**B-7**
**H-7**
**J-7**
**I'c**
**IIk** ($R^{16}$ = H)
**IIj** ($R^{16}$ = $CH_2OH$)
**IIn** ($R^{16}$ = *c*-Hex)
**I'c**
**IIm** ($R^{16}$ = Ph)

FIG 7

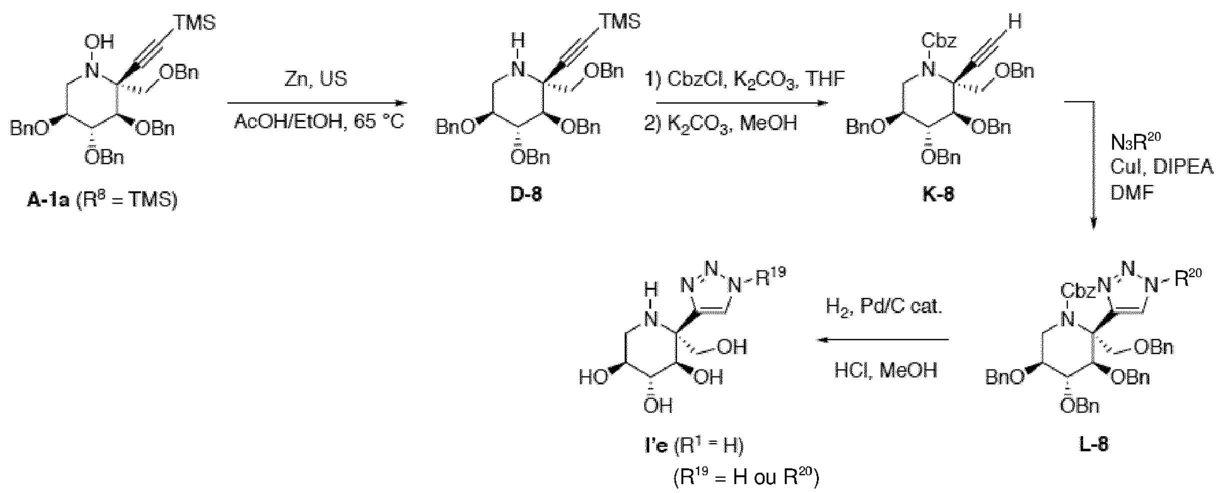


**A-1a** ($R^8$ = TMS)
**D-8**
**K-8**
**L-8**
**I'e** ($R^1$ = H)
($R^{19}$ = H ou $R^{20}$)

FIG 8

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Littérature non-brevet citée dans la description


- **BOYD et al.** *J. Med. Chem.,* 2013, vol. 56, 2705-2725
- **MARKHAM.** *Drugs,* 2016, vol. 76, 1147-1152
- **FLANAGAN et al.** *Hum. Mutat,* 2009, vol. 30, 1683-1692
- **PARENTI et al.** *Mol. Ther.,* 2007, vol. 15 (3), 508-14
- **BOISSON et al.** *Org. Lett.,* 2015, vol. 17 (15), 3662-5
- **TANGARA et al.** *Org. Lett.,* 2017, vol. 19 (18), 4842-5
- **VIEIRA DA CRUZ et al.** *J. Org. Chem.,* 2017, vol. 82 (18), 9866-72
- **AHN et al.** *J. Org. Chem.,* 1994, vol. 59, 6282-6286
- **BORNSCHEIN et al.** *Eur. J. Org. Chem,* 2015, 1915-1919
- **XIE ; DIXON.** *Chem. Sci.,* 2017, vol. 8, 7492-7497
- **STECKO et al.** *Tetrahedron,* 2014, vol. 70, 7817-7844
- **KHANGAROT ; KALIAPPAN.** *Eur. J. Org. Chem.,* 2013, 7664-7677
- **TANGARA et al.** *Org. Lett.,* 2017, vol. 19, 4842-4845
- **BOISSON et al.** *Org. Lett.,* 2015, vol. 17, 3662-3665
- **VIEIRA DA CRUZ et al.** *J. Org. Chem.,* 2017, vol. 82, 9866-9872
- **BOISSON et al.** *Org. Lett,* 2015, vol. 17, 3662-3665
- **TANGARA et al.** *New J. Chem.,* 2018, vol. 42, 16735-16743
- **TANGARA et al.** *Org. Lett,* 2017, vol. 19, 4842-4845
- **LAHAV et al.** *J. Am. Chem. Soc.,* 2017, vol. 139, 14192-14197
- **PORTO et al.** *Molecular Therapy,* 2012, vol. 20, 2201-2211
- **NIESEN et al.** *Nat. Protoc.,* 2007, vol. 2, 2212-2221
- **ARTOLA et al.** *ACS Cent. Sci.,* 2017, vol. 3, 784-793
- **BRADFORD.** *Anal. Biochem.,* 1976, vol. 72, 248-254
- **PORTO et al.** *Mol. Ther.,* 2012, vol. 20, 2201-2211
- **BRUCKMANN et al.** *ChemMedChem,* 2012, vol. 7, 1943-1953
- **FLANAGAN et al.** *Human Mut.,* 2009, vol. 30, 1683-1692
- **D'ALONZO et al.** *J. Med. Chem.,* 2017, vol. 60, 9462-9469
- **ASANO et al.** *J. Med. Chem.,* 1995, vol. 38, 2349-56
- **WENNEKES et al.** *J. Med. Chem.,* 2010, vol. 53, 689-698